# EUROPEAN PATENT SPECIFICATION

(11) **EP 2 670 744 B1**
(45) Date of publication and mention of the grant of the patent: **27.05.2015**
(21) Application number: 12706923.5
(22) Date of filing: 31.01.2012
(51) Int. Cl.: C07D 401/04, C07D 405/04, C07D 207/333, A61K 31/402, A61K 31/4025, A61K 31/435, A61P 23/00, A61P 25/16, A61P 25/18, A61P 25/28

(54) **PYRROLE DERIVATIVES USED AS MODULATORS OF ALPHA7 NACHR**
PYRROLDERIVATE ALS MODULATOREN VON ALPHA7-NACHR
DÉRIVÉS DE PYRAZOLE UTILISÉS COMME MODULATEURS DE NACHR ALPHA-7

(30) Priority: 03.02.2011 IN KO01512011
(43) Date of publication of application: 11.12.2013
(73) Proprietor: Lupin Limited, State of Maharashtra, Mumbai 400 098 (IN)
(72) Inventor: SINHA, Neelima, Pune 411 042 (IN); JANA, Gourhari, Pune 411 042 (IN); KARCHE, Navnath, Popat, Pune 411 042 (IN); ADURKAR, Shridhar, Keshav, Pune 411 042 (IN); HATNAPURE, Girish, Dhanraj, Pune 411 042 (IN); PALLE, Venkata, P., Pune 411 042 (IN); KAMBOJ, Rajender, Kumar, Pune 411 042 (IN)
(74) Representative: O'Neill, Michelle
(86) International application number: PCT/IB2012/050442
(87) International publication number: WO 2012/104782

(56) References cited:
- WO-A1-2008/002974
- DEMIRAYAK SEREF ET AL: "Synthesis and cytotoxic and analgesic activities of some 1,5-diaryl-3-ethoxycarbonylpyrrole derivatives", JOURNAL OF ENZYME INHIBITION AND MEDICINAL CHEMISTRY, TAYLOR, READING, GB, vol. 21, no. 1, 1 February 2006 (2006-02-01), pages 113-118, XP008084523, ISSN: 1475-6366, DOI: 10.1080/14756360500472845

## Description

### Field of the Invention:

The present invention is related to novel compounds of the general formula I, 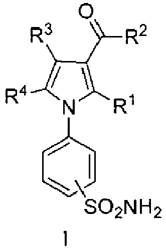 their tautomeric forms, their stereoisomers, their pharmaceutically acceptable salts, combinations with suitable medicament, pharmaceutical compositions containing them, methods of making of the above compounds. Their use as nicotinic acetylcholine receptor α7 subunit (u7 nAChR) modulator is disclosed herein.

### Background of the invention:

Cholinergic neurotransmission, mediated primarily through the neurotransmitter acetylcholine (ACh), is a predominant regulator of the physiological functions of the body via the central and autonomic nervous system. ACh acts on the synapses of the neurons present in of all the autonomic ganglia, neuromuscular junctions and the central nervous system. Two distinct classes of ACh target receptors viz. muscarinic (mAChRs) and the nicotinic (nAChRs) have been identified in brain, forming a significant component of receptors carrying its mnemonic and other vital physiological functions.

Neural nicotinic ACh receptors (NNRs) belong to the class of ligand-gated ion channels (LGIC) comprising of five subunits (α2-α10, β2-β4) arranged in heteropentameric (α4β2) or homopertameric (α7) configuration (Paterson D et al., Prog. Neurobiol., 2000, 61, 75-111). α4β2 and α7 nAChR constitute the predominant subtypes expressed in the mammalian brain. α7 nAChR has attained prominence as a therapeutic target due to its abundant expression in the learning and memory centers of brain, hippocampus and the cerebral cortex (Rubboli F et al., Neurochem. Int., 1994, 25, 69-71). Particularly, α7 nAChR is characterized by a high Ca²⁺ ion permeability, which is responsible for neurotransmitter release and consequent modulation of excitatory and inhibitory neurotransmission (Alkondon M et al., Eur. J. Pharmacol., 2000, 393, 59-67; Dajas-Bailador F et al., Trends Pharmacol. Sci., 2004, 25, 317-324). Furthermore, high Ca²⁺ ion influx also has implications on the long-term potentiation of memory via alterations in gene expression (Bitner RS et al., J. Neurosci., 2007, 27, 10578-10587; McKay BE et al., Biochem. Pharmacol., 2007, 74, 1120-1133).

Several recent studies have confirmed the role of α7 nAChR in neural processes like attention, memory and cognition (Mansvelder HD et al., Psychopharmacology (Berl), 2006, 184, 292-305; Chan WK et al., Neuropharmacology, 2007, 52, 1641-1649; Young JW et al., Eur, Neuropsychopharmacol" 2007, 17, 145-155). Gene polymorphisms associated with the α7 nAChR protein CHRNA7 have been implicated in the genetic transmission of schizophrenia, related neurophysiological sensory gating deficits and resultant cognitive impairment (Freedman R et al., Biol. Psychiatry, 1995, 38, 22-33; Tsuang DW et al., Am. J. Med. Genet., 2001, 105, 662-668). Also, preclinical studies in α 7 nAChR knock-out and anti-sense oligonucleotide treated mice have demonstrated impaired attention and defective cognition underscoring the prominent role of α7 nAChR in cognition (Curzon P et al., Neurosci. Lett., 2006, 410, 15-19; Young JW et al., Neuropsychopharmacology, 2004, 29, 891-900). Additionally, pharmacological blockade of α7 nAChR impairs memory and its activation enhances same in preclinical rodent models implicating α7 nAChR as target for cognitive enhancement (Hashimoto K et al., Biol. Psychiatry, 2008, 63, 92-97).

Pathological brain function in sensory-deficit disorders has been associated with nicotinic cholinergic transmission particularly through α7 receptors (Freedman R et al., Biol. Psychiatry, 1995, 38, 22-33; Tsuang DW et al., Am. J. Med. Genet., 2001, 105, 662-668; Carson R et al., Neuromolecular, 2008, Med 10, 377-384; Leonard S et al., Pharmacol. Biochem. Behav., 2001, 70, 561-570; Freedman R et al., Curr. Psychiatry Rep., 2003, 5, 155-161; Cannon TD et al., Curr. Opin. Psychiatry, 2005, 18, 135-140). A defective pre-attention processing of sensory information is understood to be the basis of cognitive fragmentation in schizophrenia and related neuropsychiatric disorders (Leiser SC et al., Pharmacol. Ther., 2009, 122, 302-311). Genetic linkage studies have traced sharing of the α7 gene locus for several affective, attention, anxiety and psychotic disorders (Leonard S et al., Pharmacol. Biochem. Behav., 2001, 70, 561-570; Suemaru K et al., Nippon. Yakurigaku. Zasshi., 2002, 119, 295-300). Modulation of the nicotinic cholinergic receptors, particularly α7 may provide for efficacy in a range of cognitive states, right from pre-attention to attention and subsequently working, reference and recognition memory. Accordingly, this invention may find application in the treatment and prophylaxis of multitude of disease conditions including, either one or combinations of, schizophrenia, schizophreniform disorder, cognitive deficits in schizophrenia, brief psychotic disorder, delusional disorder, schizoaffective disorder, shared psychotic disorder, paranoid personality disorder, schizoid personality disorder, schizotypal personality disorder, attention deficit disorder, attention deficit hyperactivity disorder (ADHD), depression, maniac depression, major depressive disorder, posttraumatic stress disorder, generalized anxiety disorder, tourette's syndrome, cyclothymic disorder, dysthymic disorder, agoraphobia, panic disorder (with or without agoraphobia), phobias (including social phobia) and bipolar disorders (Thomsen MS et al., Curr. Pharm. Des. 2010, 16, 323-343; Peng ZZ et al., Zhonghua Yi Xue Yi Chuan Xue Za Zhi 2008, 25, 154-158; Young JW et al., Eur. Neuropsychopharmacol. 2007, 17, 145-155; Martin LF et al., Am. J. Med. Genet. B Neuropsychiatr. Genet. 2007, 144B, 611-614; Martin LF et al., Psychopharmacology (Berl), 2004, 174, 54-64; Feher A et al., Dement. Geriatr. Cogn. Disord. 2009, 28, 56-62; Wilens TE et al., Biochem. Pharmacol. 2007, 74, 1212-1223; Verbois SL et al., Neuropharmacology, 2003, 44, 224-233; Sanberg PR et al., Pharmacol. Ther. 1997, 74, 21-25). Cholinergic system, particularly through α7 nAChR seems to have implications in traumatic brain injury-induced **psychosis.** Chronic nicotine treatment has shown to attenuate same. Thus, this invention may also find application in the treatment of deficits in cholinergic α7 nAChR following traumatic brain injury (Bennouna M et al., Encephale, 2007, 33, 616-620; Verbois SL et al., Neuropharmacology, 2003, 44, 224-233).

Perturbations in the cholinergic and glutamatergic homeostasis, has long been implicated as causative factors for host of neurological disease, including dementia(s) (Nizri E et al., Drug News Perspect. 2007, 20, 421-429). Dementia is a severe, progressive, multi-factorial cognitive disorder affecting memory, attention, language and problem solving. Nicotinic ACh receptor, particularly the interaction of α7 receptor to Aβ₁₋₄₂ is implicated as an up-stream pathogenic event in Alzheimer's disease, a major causative factor for dementia (Wang HY et al., J. Neurosci., 2009, 29, 10961-10973). Moreover, gene polymorphisms in CHRNA7 have been implicated in dementia with Lewy bodies (DLB) and Pick's disease (Feher A et al., Dement. Geriatr. Cogn. Disord. 2009, 28, 56-62). Modulation of nicotinic ACh receptors, particularly the α7 subtype could help supplement the downregulated cholinergic receptor expression and transmission as in dementia(s), and also slowing disease progression by reduction of α7-Aβ₁₋₄₂ complexation and internalization in AD and Down's syndrome (Nordberg A et al., Neurotox. Res. 2000, 2, 157-165; Haydar SN et al., Bioorg. Med. Chem., 2009, 17, 5247-5258; Deutsch SI et al., Clin. Neuropharmacol., 2003, 26, 277-283). Appropriately, this invention may find application in the treatment and prophylaxis of multitude of disease conditions including, either one or combinations of, dementia(s) due to Alzheimer's disease, dementia with Lewy bodies, Down's syndrome, head trauma, Stroke, hypoperfusion, Parkinson's disease, Huntington's disease, Prion diseases, progressive supranuclear palsy, radiation therapy, brain tumors, normal-pressure hydrocephalus, subdural hematoma, human immunodeficiency virus (HIV) infection, vitamin deficiency, hypothyroidism, drugs, alcohol, lead, mercury, aluminium, heavy metals, syphilis, Lyme disease, viral encephalitis, fungal infection and cryptococcosis (Zhao X et al., Ann N Y Acad. Sci., 2001, 939, 179-186; Perry E et al., Eur. J. Pharmacol., 2000, 393, 215-222; Harrington CR et al., Dementia, 1994, 5, 215-228; Wang J et al., J. Neurosci. Res., 2010, 88, 807-815).

Disease modification potential of nAChRs particularly the α7 receptor has application for disease-modification of Alzheimer's disease (AD) and Parkinson's disease (PD) by enhancing neuron survival and preventing neurodegeneration (Wang et al. 2009; Nagele RG et al., Neuroscience, 2002, 110, 199-211; Jeyarasasingam G et al., Neuroscience, 2002, 109, 275-285). Additionally, α7 nAChR induced activation of anti-apoptotic (BCL-2) and anti-inflammatory pathways in brain could have neuroprotective effects in neurodegenerative diseases (Marrero MB et al., Brain Res., 2009, 1256, 1-7). Thus, this invention may find application in the prophylaxis and preventive measures immediately after early-stage identification of neurodegenerative disease like Alzheimer's disease and Parkinson's disease.

Dopamine containing neurons of ventral tegmental area (VTA) and laterodorsal tegmental nucleus (LDT) are known to express nicotinic ACh receptors, particularly α4, α3, β2, β3, β4 subunits (Kuzmin A et al., Psychopharmacology (Berl), 2009, 203, 99-108). Nicotinic ACh receptors, α4β2 and α3β4 have been identified with candidate-gene approach to have strong mechanistic link for nicotine addiction (Weiss RB et al., PLoS Genet 2008, 4, e1000125). α7 nAChR has particularly been studied for a putative role in cannabis addiction (Solinas M et al., J. Neurosci., 2007, 27, 5615-5620). Varenicline, a partial agonist at α4β2, has demonstrated better efficacy in reducing the smoking addiction and relapse prevention in comparison to buproprion (Ebbert JO et al., Patient Prefer Adherence, 2010, 4, 355-362). Modulation of nicotinic ACh receptors particularly α4β2, α3β4 and α7 may have implications in the development of therapies for nicotine, cannabis addiction and relapse prevention. Accordingly, this invention may find application in the prophylaxis or therapy of nicotine addiction, cannabis addiction, relapse prevention of nicotine or cannabis addiction. Additionally, this invention may also provide for an alternative therapy for non-responding addiction patients, patients having intolerable side-effects with de-addiction therapies or those requiring long-term maintenance therapies.

Presence of a high-affinity nicotine binding site at α4β2 nAChR, in the descending inhibitory pathways from brainstem has sparked interest in the antinociceptive properties of nicotinic ACh receptor agonists like epibatidine (Decker MW et al., Expert. Opin. Investig. Drugs, 2001, 10, 1819-1830). Several new developments have opened the area for use of nicotinic modulators for therapy of pain (Rowbotham MC et al., Pain,, 2009, 146, 245-252). Appropriate modulation of the nicotinic ACh receptors could provide for remedial approach to pain related states. Thus, this invention may find application in the treatment and prophylaxis of multitude of pain conditions including, either one or combinations of, pain arising from, peripheral nervous system (PNS), post-diabetic neuralgia (PDN), post-herpetic neuralgia (PHN), multiple sclerosis, Parkinson's disease, low-back pain, fibromyalgia, post-operative pain, acute pain, chronic pain, mononeuropathy, primary lateral sclerosis, pseudobulbar palsy, progressive muscular palsy, progressive bulbar palsy, postpolio syndrome, diabetes induced polyneuropathy, acute demyelinating polyneuropathy (Guillain-Barre syndrome), acute spinal muscular atrophy (Werdnig-Hoffman disease) and secondary neurodegeneration (Donnelly-Roberts DL et al., J. Pharmacol. Exp. Ther., 1998, 285, 777-786; Rowley TJ et al., Br. J. Anaesth., 2010, 105, 201-207; Bruchfeld A et al., J. Intern. Med., 2010, 268, 94-101).

Another key role of the α7 nAChR is the ability to modulate the production of pro-inflammatory cytokines, like interleukins (IL), tumor necrosis factor alpha (TNF-α), and high mobility group box (HMGB-1) in the central nervous system. Consequently, an anti-inflammatory and antinociceptive effect in pain disorders have been demonstrated (Damaj MI et al., Neuropharmacology, 2000, 39, 2785-2791). Additionally, 'cholinergic anti-inflammatory pathway' is proposed to be a regulatory of local and systemic inflammation and neuro-immune interactions through neural and humoral pathways (Gallowitsch-Puerta M et al., Life Sci. 2007, 80, 2325-2329; Gallowitsch-Puerta and Pavlov 2007; Rosas-Ballina M et al., Mol. Med. 2009, 15, 195-202; Rosas-Ballina M et al., J. Intern. Med. 2009, 265, 663-679). Selective modulators of nicotinic ACh receptors, particularly α7 type, like GTS-21, attenuate cytokine production and IL-1β after endotoxin exposure. Furthermore, α7 nAChR are understood to have a central role in arthritis pathogenesis and potential therapeutic strategy for treatment of joint inflammation (Westman M et al., Scand J. Immunol. 2009, 70, 136-140). A putative role for α7 nAChR has also been implicated in severe sepsis, endotoxemic shock and systemic inflammation (Jin Y et al. (2010) Int. J. Immunogenet. Liu C et al., Crit. Care Med. 2009, 37, 634-641). This invention may thus find application in the treatment and prophylaxis of plethora of inflammation and pain related states involving TNF-α and thus providing symptomatic relief in either any one or combination of, rheumatoid arthritis, bone resorption diseases, atherosclerosis, inflammatory bowel disease, Crohn's disease, inflammation, cancer pain, muscle degeneration, osteoarthritis, osteoporosis, ulcerative colitis, rhinitis, pancreatitis, spondylitis, acute respiratory distress syndrome (ARDS), joint inflammation, anaphylaxis, ischemia reperfusion injury, multiple sclerosis, cerebral malaria, septic shock, tissue rejection of graft, brain trauma, toxic shock syndrome, herpes virus infection (HSV-1 & HSV-2), herpes zoster infection, sepsis, fever, myalgias, asthma, uveititis, contact dermatitis, obesity-related disease and endotoxemia (Giebelen IA T et al., Shock, 2007, 27, 443-447; Pena G et al., Eur. J. Immunol., 2010, 40, 2580-2589).

Angiogenesis, is a critical physiological process for the cell survival and pathologically important for cancer proliferation; several non-neural nicotinic ACh receptors, particularly α7, α5, α3, β2, β4, are involved (Arias HR et al., Int. J. Biochem. Cell Biol., 2009, 41, 1441-1451; Heeschen C et al., J. Clin. Invest., 2002, 110, 527-536). A role of nicotinic ACh receptors in the development of cervical cancer, lung carcinogenesis and paediatric lung disorders in smoking-exposed population has also been studied (Calleja-Macias IE et al., Int. J. Cancer, 2009, 124, 1090-1096; Schuller HM et al., Eur. J. Pharmacol., 2000, 393, 265-277). It is thus, imperative for the modulators of nicotinic ACh receptors, to have a modulatory role in angiogenesis and cancer cell survival. Thus, this invention may find application in the treatment and prophylaxis of multitude of cancerous conditions including, one or combination of, acute or chronic myelogenous leukemia, multiple myeloma, tumor growth inhibition, angiogenesis and cancer associated-cachexia.

Several α7 nAChR agonists, partial agonists, have been characterized for their efficacy in clinical and preclinical studies. EVP-6124, an agonist at α7 nAChR, has demonstrated significant improvement in sensory processing and cognition biomarkers in Phase Ib study with patients suffering from schizophrenia (EnVivo Pharmaceuticals press release 2009, Jan 12). GTS-21 (DMXB-Anabaseine), an α7 nAChR agonist, in the P II clinical trials, has shown efficacy in improving cognitive deficits in schizophrenia and inhibition of endotoxin-induced TNF-α release (Olincy A et al., Biol. Psychiatry, 2005, 57(8, Suppl.), Abst 44; Olincy A et al., Arch. Gen. Psychiatry, 2006, 63, 630-638; Goldstein R et al., Acad. Emerg. Med., 2007, 14 (15, Suppl. 1), Abst. 474). CP-810123, a α7 nAChR agonist, exhibits protection against the scopolamine-induced dementia and inhibition of amphetamine-induced auditory evoked potentials in preclinical studies (O'Donnell CJ et al., J. Med. Chem., 2010, 53, 1222-1237). SSR-180711A, also an α7 nAChR agonist, enhances learning and memory, and protects against MK-801/Scopolamine-induced memory loss and prepulse inhibition in preclinical studies (Redrobe JP et al., Eur. J. Pharmacol., 2009, 602, 58-65; Dunlop J et al., J. Pharmacol. Exp. Ther., 2009, 328, 766-776; Pichat P et al., Neuropsychopharmacology, 2007, 32, 17-34). SEN-12333, protected against scopolamine-induced amnesia in passive avoidance test in preclinical studies (Roncarati R et al., J. Pharmacol. Exp. Ther., 2009, 329, 459-468). AR-R-17779, an agonist at α7 nAChR, exhibits improvement in the social recognition task performed in rats (Van KM et al., Psychopharmacology (Berl), 2004, 172, 375-383). ABBF, an agonist at α7 nAChR, improves social recognition memory and working memory in Morris maze task in rats (Boess FG et al., J. Pharmacol. Exp. Ther., 2007, 321, 716-725). TC-5619, a selective α7 nAChR agonist has demonstrated efficacy in animal models of positive and negative symptoms and cognitive dysfunction in schizophrenia (Hauser TA et al., Biochem. Pharmacol., 2009, 78, 803-812).

An alternative strategy to reinforce or potentiate the endogenous cholinergic neurotransmission of ACh without directly stimulating the target receptor is the positive allosteric modulation (PAM) of α7 nAChR (Albuquerque EX et al., Alzheimer Dis. Assoc. Disord., 2001, 15 Suppl 1, S19-S25). Several PAMs have been characterized, albeit in the preclinical stages of discovery. A-86774, α7 nAChR PAM, improves sensory gating in DBA/2 mice by significantly reducing the T:C ratio in a preclinical model of schizophrenia (Faghih R et al., J. Med. Chem., 2009, 52, 3377-3384). XY-4083, an α7 nAChR PAM, normalizes the sensorimotor gating deficits in the DBA/2 mice and memory acquisition in 8-arm radial maze without altering the receptor desensitization kinetics (Ng HJ et al., Proc. Natl. Acad. Sci. U. S. A., 2007, 104, 8059-8064). Yet another PAM, PNU-120596, profoundly alters α7 nAChR desensitization kinetics and simultaneously protecting against the disruption of prepulse inhibition by MK-801. NS-1738, another PAM, has exhibited efficacy in-vivo in the animal models of social recognition and spatial memory acquisition in the Morris maze task (Timmermann DB et al., J. Pharmacol. Exp. Ther., 2007, 323, 294-307). In addition, several patents/applications published are listed below - US20060142349, US20070142450, US20090253691, WO2007031440, WO2009115547, WO2009135944, WO2009127678, WO2009127679, WO2009043780, WO2009043784, US7683084, US7741364, WO2009145996, US20100240707, WO2011064288, US20100222398, US20100227869, EP1866314, WO2010130768, WO2011036167, US20100190819 disclose efficacy of allosteric modulators of nicotinic ACh receptors and underscoring their therapeutic potential.

Following are the abbreviations used and meaning thereof in the specification:
ACh: Acetylcholine.
AD: Alzheimer's disease.
ADC: AIDS dementia complex.
ADHD: attention deficit hyperactivity disorder.
AIDS: Acquired immunodeficiency syndrome.
ARDS: acute respiratory distress syndrome.
DCC: 1,3-dicyclohexylcarbodiimide.
DCE: dichloroethane.
DCM: dichloromethane.
DLB: dementia with Lewy bodies.
DMF: N,N-dimethylformamide.
EDCI: 1-(3-dimethylaminopropyl)-3-ethylcarbodimide hydrochloride.
FLIPR: Fluorometric Imaging Plate Reader.
HBSS: Hank's balanced salt solution.
HEPES: 4-(2-hydroxyethyl)piperazine-1-ethanesulfonic acid.
HMGB: high mobility group box.
HOAT: 1-hydroxy-7-azabenzotriazole.
HOBT: hydroxybenzotriazole hydrate.
HPLC: High Performance liquid chromatography.
IL: interleukins.
LDT: laterodorsal tegmental nucleus.
LGIC: ligand-gated ion channels.
MCI: mild cognitive impairment.
NBS: N-bromosuccinamide.
NCS: N-chlorosuccinamide.
NIS: N-iodosuccinamide
NNRs: Neural nicotinic ACh receptors.
PAM: positive allosteric modulation.
PD: Parkinson's disease.
PDN: post-diabetic neuralgia.
PHN: post-herpetic neuralgia.
PMBO: p-methoxy benzyloxy.
PNS: peripheral nervous system.
TBI: traumatic brain injury.
THF: Tetrahydrofuran.
TLC: Thin layer chromatography.
TMS: tetramethylsilane.
TNF-α: tumor necrosis factor alpha.
VTA: ventral tegmental area.
α7 nAChR: nicotinic acetylcholine receptor α7 subunit.

### Objective of the Invention:

The main objective of the present invention is therefore to provide novel compounds of the general formula I, their tautomeric forms, their stereoisomers, their pharmaceutically acceptable salts, pharmaceutical compositions containing them, process and intermediates for the preparation of the above said compounds which have α7 nAChR modulatory activity.

### Summary of the Invention

According to one aspect of the present invention there is provided compounds represented by the general formula I, its tautomeric forms, its pharmaceutically acceptable salts, their combinations with suitable medicament and pharmaceutical compositions containing them.

A process for the preparation of the compounds of the general formula I is also provided herein.

Novel intermediates, a process for their preparation and their use in methods of making compounds of the general formula I is further disclosed herein.

### Detailed Description of the invention:

The present invention relates to a compound of the general formula I, its tautomeric forms, its stereoisomers, its pharmaceutically acceptable salts, their combinations with suitable medicament and pharmaceutical compositions containing them. 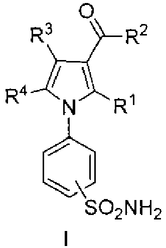 wherein,
R¹ is selected from hydrogen, halogen, optionally substituted alkyl, perhaloalkyl, optionally substituted cycloalkyl, optionally substituted aryl; optionally substituted heterocyclyl, optionally substituted heteroaryl;
R² is selected from optionally substituted alkyl, optionally substituted heteroalkyl, optionally substituted aryl, optionally substituted heteroaryl, optionally substituted cycloalkyl, optionally substituted heterocyclyl, or -NR⁵(R⁶), -A¹R⁵, -N(R⁵)OR⁶;
R³ is selected from hydrogen, optionally substituted alkyl, halo, optionally substituted cycloalkyl, optionally substituted aryl, optionally substituted heterocyclyl, optionally substituted heteroaryl, cyano, nitro or -NR⁵(R⁶), -OR⁵;
R⁴ is 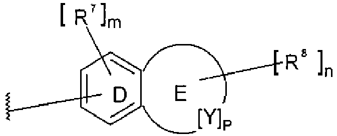
   wherein, phenyl ring `D' is fused with ring 'E', which is a non-aromatic five to eight member ring inclusive of `Y' group(s);
   Y is independently selected at each repetition from -O-, -S-, -NH-, 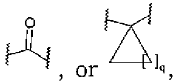 where q = 1 - 4; wherein when Y is selected as -NH- or 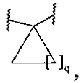 it is optionally substituted by [R⁸]ₙ;
   wherein, R⁵ and R⁶ are independently selected from hydrogen, optionally substituted alkyl, optionally substituted heteroalkyl, optionally substituted aryl, optionally substituted heteroaryl, optionally substituted cycloalkyl, optionally substituted heterocyclyl, R^{9a}C(=A¹)-;
   R⁷ is selected independently at each occurrence from the group consisting of halogen, optionally substituted alkyl, optionally substituted cycloalkyl;
   R⁸ is independently selected at each occurrence from the group consisting of optionally substituted alkyl, R⁹A¹-, R^{9a}C(=A¹)-;
   m = 0 to 2;
   n = 0 to 3;
   p = 0 to 4;
   wherein, R⁹ wherever it appears, is selected from hydrogen, optionally substituted C₁₋₆ alkyl, optionally substituted heteroalkyl, optionally substituted aryl, optionally substituted heteroaryl, optionally substituted cycloalkyl, and optionally substituted heterocyclyl; and A¹ is selected from O and S;
   R^{9a} wherever it appears, is selected from optionally substituted C₁₋₆ alkyl, optionally substituted heteroalkyl, optionally substituted aryl, optionally substituted heteroaryl, optionally substituted cycloalkyl, and optionally substituted heterocyclyl;
   wherein,
   the term "optionally substituted alkyl", means a alkyl group optionally substituted with 1 to 6 substituents selected independently from the group comprising of oxo, halogen, nitro, cyano, aryl, hereroaryl, cycloalkyl, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R^{10a}C(=O)N(H)-, (R¹⁰)(H)N-, (R¹⁰)(alkyl)N-, (R¹⁰)(H)NC(=A¹)N(H)-, (R¹⁰)(Akyl)NC(=A¹)N(H)-;
   the term "optionally substituted heteroalkyl" means a heteroalkyl group optionally substituted with 1 to 6 substituents selected independently from the group comprising of oxo, halogen, nitro, cyano, aryl, hereroaryl, cycloalkyl.
   the term "optionally substituted cycloalkyl" means a cycloalkyl group optionally substituted with 1 to 6 substituents selected independently from the group comprising of oxo, halogen, nitro, cyano, aryl, hereroaryl, alkyl, R^{10a}C(=O)-, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-;
   the term "optionally substituted aryl" means (i) an aryl group optionally substituted with 1 to 3 substituents selected independently from the group comprising of halogen, nitro, cyano, hydroxy, C₁ to C₆ alkyl, C₃ to C₆ cycloalkyl, C₁ to C₆ perhaloalkyl, alkyl-O-, perhaloalkyl-O-, alkyl-N(alkyl)-, alkyl-N(H)-, H₂N-, alkyl-SO₂-, perhaloalkyl-SO₂-, alkyl-C(=O)N(alkyl)-, alkyl-C(=O)N(H)-, alkyl-N(alkyl)C(=O)-, alkyl-N(H)C(=O)-, H₂NC(=O)-, alkyl-N(alkyl)SO₂-, alkyl-N(H)SO₂-, H₂NSO₂-, 3 to 6 membered heterocycle containing 1 to 2 heteroatoms selected from N, O and S optionally substituted with alkyl or alkyl-C(=O)-, (ii) an aryl ring optionally fused with cycloalkane or heterocycle across a bond optionally substituted with oxo, alkyl or alkyl-C(=O)-;
   the term "optionally substituted heterocyclyl" means a (i) heterocyclyl group optionally substituted on ring carbons with 1 to 6 substituents selected independently from the group comprising of oxo, halogen, nitro, cyano, aryl, hereroaryl, alkyl, R¹⁰A¹-, R^{10a}OC(=O)-, R^{10a}C(=O)O-, (R¹⁰)(H)NC(=O)-, (R¹⁰)(alkyl)NC(O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-; (ii) heterocyclyl group optionally substituted on ring nitrogen(s) with substituents selected from the group comprising of aryl, hereroaryl, alkyl, R^{10a}C(=O)-, R^{10a}SO₂-, R^{10a}OC(=O)-, (R¹⁰)(H)NC(=O)-, (R¹⁰)(alkyl)NC(=O)-;
   the term "optionally substituted heteroaryl" means a heteroaryl group optionally substituted with 1 to 3 substituents selected independently from the group comprising of halogen, nitro, cyano, hydroxy, C₁ to C₆ alkyl, C₃ to C₆ cycloalkyl, C₁ to C₆ perhaloalkyl, alkyl-O-, perhaloalkyl-O-, alkyl-N(alkyl)-, alkyl-N(H)-, H₂N-, alkyl-SO₂-, perhaloalkyl-SO₂-, alkyl-C(=O)N(alkyl)-, alkyl-C(=O)N(H)-, alkyl-N(alkyl)C(=O)-, alkyl-N(H)C(=O)-, H₂NC(=O)-, alkyl-N(alkyl)SO₂-, alkyl-N(H)SO₂-, H₂NSO₂-, 3 to 6 membered heterocycle containing 1 to 2 heteroatoms selected from N, O and S optionally substituted with alkyl or alkyl-C(=O)-;
   wherein R¹⁰ is selected from hydrogen, alkyl, aryl, heteroaryl, cycloalkyl or heterocyclyl; and A¹ is selected from S and O; and R^{10a} is selected from alkyl, perhaloalkyl, aryl, heteroaryl, cycloalkyl or heterocyclyl.

Other aspect of the invention of the present invention is compound of formula I as described hereinabove wherein when p is selected as 0 then n is selected from the integers ranging between 1 and 4.

Preferred embodiment of the present invention is compound of formula I as defined herein above, wherein R¹ is selected from methyl.

Other preferred embodiment of the present invention is compound of formula I as defined hereinabove, wherein, R² is selected from ethyl and ethoxy.

Another preferred embodiment of the present invention is compound of formula I as defined hereinabove, wherein, R³ is selected from hydrogen and methyl.

Yet another preferred embodiment of the present invention is compound of formula I as defined hereinabove, wherein, R⁴ is selected from following groups:

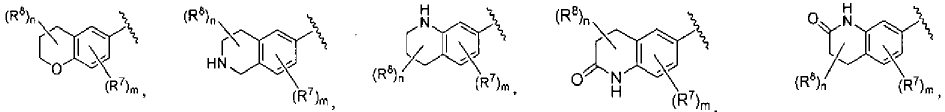

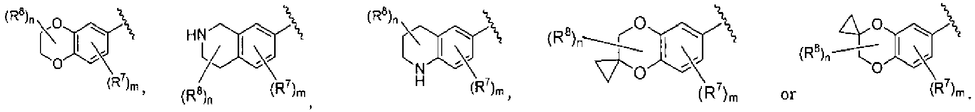

Further preferred embodiment of the present invention is compound of formula I as defined hereinabove, wherein R¹ is selected from methyl; R² is selected from ethyl and ethoxy; R³ is selected from hydrogen and methyl; and R⁴ is selected from following groups:

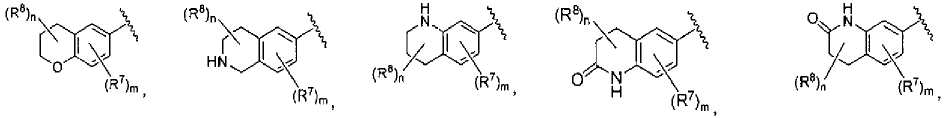

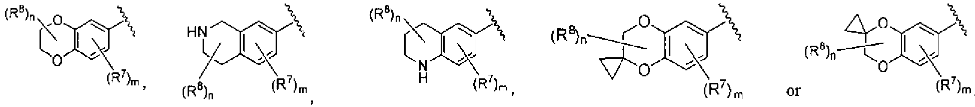

General terms used in formula can be defined as follows; however, the meaning stated hereinbelow should not be interpreted as limiting the scope of the term *per se*.

The term "**alkyl**", as used herein, means a straight or branched chain hydrocarbon containing from 1 to 20 carbon atoms. The term as defined herein also includes unsaturated chains containing 2 to 20 carbon atoms and one or more unsaturations (double or triple bonds) as in alkenyl and alkynyl groups. Preferably the alkyl chain may contain 1 to 10 carbon atoms, and alkenyl and alkynyl chains may contain 2 to 10 carbons. More preferably alkyl chain may contain up to 6 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, allyl, vinyl, acetylene, and n-hexyl.

Alkyl as defined hereinabove may be optionally substituted with one or more substituents selected independently from the group comprising of oxo, halogen, nitro, cyano, aryl, hereroaryl, cycloalkyl, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-; wherein R¹⁰ is selected from hydrogen, alkyl, aryl, heteroaryl, cycloalkyl or heterocyclyl; and A¹ is selected from S and O; and R^{10a} is selected from alkyl, perhaloalkyl, aryl, heteroaryl, cycloalkyl or heterocyclyl.

The term "**perhaloalkyl**" used herein means an alkyl group as defined hereinabove wherein all the hydrogen atoms of the said alkyl group are substituted with halogen. The perhaloalkyl group is exemplified by trifluoromethyl, pentafluoroethyl and the like.

The term "**heteroalkyl**" as used herein means an 'alkyl' group wherein one or more of the carbon atoms replaced by -O-, -S-, -S(O₂)-, -S(O)-, -N(R^{m})-, Si(R^{m})Rⁿ-wherein, R^{m} and Rⁿ are independently selected from hydrogen, alkyl, aryl, heteroaryl, cycloalkyl, and heterocyclyl.

The term "**cycloalkyl**" as used herein, means a monocyclic, bicyclic, or tricyclic non-aromatic ring system containing from 3 to 14 carbon atoms, preferably monocyclic cycloalkyl ring containing 3 to 6 carbon atoms. The ring may contain one or more unsaturations (double or triple bonds). Examples of monocyclic ring systems include cyclopropyl, cyclobutyl, cyclopentyl, cyclohexyl, cycloheptyl, and cyclooctyl. Bicyclic ring systems are also exemplified by a bridged monocyclic ring system in which two non-adjacent carbon atoms of the monocyclic ring are linked by an alkylene bridge. Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane, bicyclo[3.3.2]decane, bicyclo[3.1.0]hexane, bicyclo[410]heptane, bicyclo[3.2.0]heptanes, octahydro-1H-indene. Tricyclic ring systems are also exemplified by a bicyclic ring system in which two non-adjacent carbon atoms of the bicyclic ring are linked by a bond or an alkylene bridge.

Representative examples of tricyclic-ring systems include, but are not limited to, tricyclo[3.3.1.0^{3.7}]nonane and tricyclo[3.3.1.1^{3.7}]decane (adamantane). The term cycloalkyl also include spiro systems wherein one of the ring is annulated on a single carbon atom such ring systems are exemplified by spiro[2.5]octane, spiro[4.5]decane,. spiro[bicyclo[4.1.0]heptane-2,1'-cyclopentane], hexahydro-2'H-spiro[cyclopropane-1,1'-pentalene].

cycloalkyl as defined hereinabove may be optionally substituted with one or more substituents selected independently from the group comprising of oxo, halogen, nitro, cyano, aryl, hereroaryl, alkyl, R^{10a}C(=O)-, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-; wherein R¹⁰ is selected from hydrogen, alkyl, aryl, heteroaryl, cycloalkyl or heterocyclyl; and A¹ is selected from S and O; and R^{10a} is selected from alkyl, perhaloalkyl, aryl, heteroaryl, cycloalkyl or heterocyclyl.

The term "**aryl**" refers to a monovalent monocyclic, bicyclic or tricyclic aromatic hydrocarbon ring system. Examples of aryl groups include but not limited to phenyl, naphthyl, anthracenyl, fluorenyl, indenyl, azulenyl, and the like. The said aryl group also includes aryl rings fused with heteroaryl or heterocyclic rings such as 2,3-dihydro-benzo[1,4]dioxin-6-yl; 2,3-dihydro-benzo[1,4]dioxin-5-yl; 2,3-dihydro-benzofuran-5-yl; 2,3-dihydro-benzofuran-4-yl; 2,3-dihydro-benzofuran-6-yl; 2,3-dihydro-benzofuran-6-yl; 2,3-dihydro-1H-indol-5-yl; 2,3-dihydro-1H-indol-4-yl; 2,3-dihydro-1H-indol-6-yl; 2,3-dihydro-1H-indol-7-yl; benzo[1,3]dioxol-4-yl; benzo[1,3]dioxol-5-yl; 1,2,3,4-tetrahydroquinolinyl; 1,2,3,4-tetrahydroisoquinolinyl; 2,3-dihydrobenzothien-4-yl, 2-oxoindolin-5-yl.

Aryl as defined hereinabove may be optionally substituted with one or more substituents selected independently from the group comprising of halogen, nitro, cyano, hydroxy, C₁ to C₆ alkyl, C₃ to C₆ cycloalkyl, C₁ to C₆ perhaloalkyl, alkyl-O-, perhaloalkyl-O-, alkyl-N(alkyl)-, alkyl-N(H)-, H₂N-, alkyl-SO₂-, perhaloalkyl-SO₂-, alkyl-C(=O)N(alkyl)-, alkyl-C(=O)N(H)-, alkyl-N(alkyl)C(=O)-, alkyl-N(H)C(=O)-, H₂NC(=O)-, alkyl-N(alkyl)SO₂-, alkyl-N(H)SO₂-, H₂NSO₂-, 3 to 6 membered heterocycle containing 1 to 2 heteroatoms selected from N, O and S optionally substituted with alkyl or alkyl-C(=O)-.

The term "**heteroaryl**" refers to a 5-14 membered monocyclic, bicyclic, or tricyclic ring system having 1-4 ring heteroatoms selected from O, N, or S, and the remainder ring atoms being carbon (with appropriate hydrogen atoms unless otherwise indicated), wherein at least one ring in the ring system is aromatic. Heteroaryl groups may be optionally substituted with one or more substituents. In one embodiment, 0, 1, 2, 3, or 4 atoms of each ring of a heteroaryl group may be substituted by a substituent. Examples of heteroaryl groups include but not limited to pyridyl, 1-oxo-pyridyl, furanyl, thienyl, pyrrolyl, oxazolyl, oxadiazolyl, imidazolyl, thiazolyl, isoxazolyl, quinolinyl, pyrazolyl, isothiazolyl, pyridazinyl, pyrimidinyl, pyrazinyl, triazinyl. triazolyl, thiadiazolyl, isoquinolinyl, benzoxazolyl, benzofuranyl, indolizinyl, imidazopyridyl, tetrazolyl, benzimidazolyl, benzothiazolyl, benzothiadiazolyl, benzoxadiazolyl, indolyl, azaindolyl, imidazopyridyl, quinazolinyl, purinyl, pyrrolo[2,3]pyrimidinyl, pyrazolo[3,4]pyrimidinyl, and benzo(b)thienyl, 2,3-thiadiazolyl, 1H-pyrazolo[5,1-c ]-1,2,4-triazolyl, pyrrolo[3,4-d]-1,2,3-triazolyl, cyclopentatriazolyl, 3H-pyrrolo[3,4-c] isoxazolyl and the like.

heteroaryl as defined hereinabove may be optionally substituted with one or more substituents selected independently form the group comprising of halogen, nitro, cyano, hydroxy, C₁ to C₆ alkyl, C₃ to C₆ cycloalkyl, C₁ to C₆ perhaloalkyl, alkyl-O-, perhaloalkyl-O-, alkyl-N(alkyl)-, alkyl-N(H)-, H₂N-, alkyl-SO₂-, perhaloalkyl-SO₂-, alkyl-C(=O)N(alkyl)-, alkyl-C(=O)N(H)-, alkyl-N(alkyl)C(=O)-, alkyl-N(H)C(=O)-, H₂NC(=O)-, alkyl-N(alkyl)SO₂-, alkyl-N(H)SO₂-, H₂NSO₂-, 3 to 6 membered heterocycle containing 1 to 2 heteroatoms selected from N, O and S optionally substituted with alkyl or alkyl-C(=O)-.

The term "**heterocycle**" or "heterocyclic" as used herein, means a 'cycloalkyl' group wherein one or more of the carbon atoms replaced by -O-, -S-, -S(O₂)-, -S(O)-, - N(R^{m})-, -Si(R^{m})Rⁿ-, wherein, R^{m} and Rₙ are independently selected from hydrogen, alkyl, aryl, heteroaryl, cycloalkyl, and heterocyclyl. The heterocycle may be connected to the parent molecular moiety through any carbon atom or any nitrogen atom contained within the heterocycle. Representative examples of monocyclic heterocycle include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl. oxadiazolidinyl, oxazolinyl, oxazolidinyl, piperazinyl, piperidinyl, pyranyl, pyrazolinyl, pyrazolidinyl. pyrrolinyl, pyrrolidinyl, tetrahydrofuranyl, tetrahydrothienyl, thiadiazolinyl, thiadiazolidinyl, thiazolinyl, thiazolidinyl, thiomorpholinyl, 1.1-dioxidothiomorpholinyl (thiomorpholine sulfone). thiopyranyl, and trithianyl. Representative examples of bicyclic heterocycle include, but are not limited to 1,3-benzodioxolyl, 1 ,3-benzodithiolyl, 2,3-dihydro-1,4-benzodioxinyl, 2,3-dihydro-1-benzofuranyl, 2,3-dihydro-1-benzothienyl, 2,3-dihydro-1 H-indolyl and 1,2,3,4-tetrahydroquinolinyl. The term heterocycle also include bridged heterocyclic systems such as azabicyclo[3.2.1]octane, azabicyclo[3.3.1]nonane and the like.

Heterocyclyl group may optionally be substituted on ring carbons with one or more substituents selected independently from the group comprising of oxo, halogen, nitro, cyano, aryl, hereroaryl, alkyl, R¹⁰A¹-, R^{10a}OC(=O)-, R^{10a}C(=O)O-, (R¹⁰)(H)NC(=O)-, (R¹⁰)(alkyl)NC(O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-; wherein R¹⁰ is selected from hydrogen, alkyl, aryl, heteroaryl, cycloalkyl or heterocyclyl; and A¹ is selected from S and O; and R^{10a} is selected from alkyl, perhaloalkyl, aryl, heteroaryl, cycloalkyl or heterocyclyl.

Heterocyclyl group may further optionally be substituted on ring nitrogen(s) with substituents selected from the group comprising of aryl, hereroaryl, alkyl, R^{10a}C(=O)-, R^{10a}SO₂-, R^{10a}OC(=O)-, (R¹⁰)(H)NC(=O)-, (R¹⁰)(alkyl)NC(=O)-; wherein R¹⁰ is selected from hydrogen, alkyl, aryl, heteroaryl, cycloalkyl or heterocyclyl; and R^{10a} is selected from alkyl, perhaloalkyl, aryl, heteroaryl, cycloalkyl or heterocyclyl.

A compound its stereoisomers, pharmaceutically acceptable salt and pharmaceutical composition thereof as described hereinabove wherein the compound of general formula I is selected from:
1. 4-(5-(4,4-dimethylchroman-6-yl)-2-methyl-3-propionyl-1*H*-pyrrol-1-yl)benzenesulfonamide.
2. 4-(5-(2,3-dihydrobenzo[b][1,4]dioxin-6-yl)-2-methyl-3-propionyl-1H-pyrrol-1-yl)benzenesulfonamide.
3. 4-(2-(2,3-dihydrobenzo[b][1,4]dioxin-6-yl)-3,5-dimethyl-4-propionyl-1H-pyrrol-1-yl)benzenesulfonamide.
4. Ethyl 5-(2,3-dihydrobenzo[b][1,4]dioxin-6-yl)-2,4-dimethyl-1-(4-sulfamoylphenyl)-1H-pyrrole-3-carboxylate.
5. 4-(5-(2,2-dimethylchroman-6-yl)-2-methyl-3-propionyl-1H-pyrrol-1-yl)benzenesulfonamide.
6. 4-(5-(8-fluoro-4,4-dimethylchroman-6-yl)-2-methyl-3-propionyl-1H-pyrrol-1-yl) benzenesulfonamide.
7. 4-(5-(2-acetyl-4,4-dimethyl-1,2,3,4-tetrahydroisoquinolin-7-yl)-2-methyl-3-propionyl-1H-pyrrol-1-yl)benzenesulfonamide.
8. 4-(5-(2-acetyl-4,4-dimethyl-1,2,3,4-tetrahydroisoquinolin-6-yl)-2-methyl-3-propionyl-1H-pyrrol-1-yl)benzenesulfonamide.
9. 4-(5-(4,4-dimethyl-1,2,3,4-tetrahydroisoquinolin-7-yl)-2-methyl-3-propionyl-1H-pyrrol-1-yl)benzenesulfonamide.
10. 4-(2-methyl-3-propionyl-5-(3H-spiro[benzo[b][1,4]dioxine-2,1'-cyclopropan]-7-yl)-1H-pyrrol-1-yl)benzenesulfonamide.
11.4-(2-methyl-3-propionyl-5-(3H-spiro[benzo[b][1,4]dioxin-2,1'-cyclopropan]-6-yl)-1H-pyrrol-1-yl)benzenesulfonamide.
12.4-(5-(1-acetyl-4,4-dimethyl-1,2,3,4-tetrahydroquinolin-6-yl)-2-methyl-3-propionyl-1 H-pyrrol-1-yl)benzenesulfonamide.
13.4-(5-(1-acetyl-4,4-dimethyl-1,2,3,4-tetrahydroquinolin-7-yl)-2-methyl-3-propionyl-1H-pyrrol-1-yl)benzenesulfonamide.
14.4-(5-(4,4-dimethyl-1,2,3,4-tetrahydroquinolin-6-yl)-2-methyl-3-propionyl-1H-pyrrol-1-yl)benzenesulfonamide.
15.4-(5-(4,4-dimethyl-1,2,3,4-tetrahydroquinolin-7-yl)-2-methyl-3-propionyl-1 H-pyrrol-1-yl)benzenesulfonamide.
16.4-(5-(4,4-dimethyl-2-oxo-1,2,3,4-tetrahydroquinolin-6-yl)-2-methyl-3-propionyl-1H-pyrrol-1-yl)benzenesulfonamide.
17. 4-(5-(4,4-dimethyl-2-oxo-1,2,3,4-tetrahydroquinolin-7-yl)-2-methyl-3-propionyl-1H-pyrrol-1-yl)benzenesulfonamide.
18. 4-(2-methyl-3-propionyl-5-(1,4,4-trimethyl-2-oxo-1,2,3,4-tetrahydroquinolin-6-yl)-1H-pyrrol-1-yl)benzenesulfonamide.

Use of a compound, its stereoisomers, its pharmaceutically acceptable salt, and its pharmaceutical composition thereof in preventing or treating a disease or its symptoms or a disorder medicated partially or completely by nicotinic acetylcholine receptors wherein the compound is selected form:
1. 4-(5-(4,4-dimethylchroman-6-yl)-2-methyl-3-propionyl-1*H-*pyrrol-1-yl)benzenesulfonamide.
2. 4-(5-(2,3-dihydrobenzo[b][1,4]dioxin-5-yl)-2-methyl-3-propionyl-1H-pyrrol-1-yl)benzenesulfonamide.
3. 4-(2-(2,3-dihydrobenzo[b][1,4]dioxin-5-yl)-3,5-dimethyl-4-propionyl-1H-pyrrol-1-yl)benzenesulfonamide.
4. Ethyl 5-(2,3-dihydrobenzo[b][1,4]dioxin-6-yl)-2,4-dimethyl-1-(4-sulfamoylphenyl)-1 H-pyrrole-3-carboxylate.
5. 4-(5-(2,3-dihydro-1H-inden-4-yl)-2-methyl-3-propionyl-1H-pyrrol-1-yl)benzenesulfonamide.
6. 4-(5-(2,2-dimethylchroman-6-yl)-2-methyl-3-propionyl-1H-pyrrol-1-yl)benzenesulfonamide.
7. 4-(5-(8-fluoro-4,4-dimethylchroman-6-yl)-2-methyl-3-propionyl-1H-pyrrol-1-yl)benzenesulfonamide.
8. 4-(5-(2-acetyl-4,4-dimethyl-1,2,3,4-tetrahydroisoquinolin-7-yl)-2-methyl-3-propionyl-1H-pyrrol-1-yl)benzenesulfonamide.
9. 4-(5-(2-acetyl-4,4-dimethyl-1,2,3,4-tetrahydroisoquinolin-6-yl)-2-methyl-3-propionyl-1H-pyrrol-1-yl)benzenesulfonamide.
10.4-(5-(4,4-dimethyl-1,2,3,4-tetrahydroisoquinolin-7-yl)-2-methyl-3-propionyl-1H-pyrrol-1-yl)benzenesulfonamide.
11.4-(2-methyl-3-propionyl-5-(3H-spiro[benzo[b][1,4]dioxine-2,1'-cyclopropan]-7-yl)-1H-pyrrol-1-yl)benzenesulfonamide.
12.4-(2-methyl-3-propionyl-5-(3H-spiro[benzo[b][1,4]dioxine-2,1'-cyclopropan]-6-yl)-1H-pyrrol-1-yl)benzenesulfonamide.
13.4-(5-(1-acetyl-4,4-dimethyl-1,2,3,4-tetrahydroquinolin-6-yl)-2-methyl-3-propionyl-1H-pyrrol-1-yl)benzenesulfonamide.
14.4-(5-(1-acetyl-4,4-dimethyl-1,2,3,4-tetrahydroquinolin-7-yl)-2-methyl-3-propionyl-1H-pyrrol-1-yl)benzenesulfonamide.
15.4-(5-(4,4-dimethyl-1,2,3,4-tetrahydroquinolin-6-yl)-2-methyl-3-propionyl-1H-pyrrol-1-yl)benzenesulfonamide.
16.4-(5-(4,4-dimethyl-1,2,3,4-tetrahydroquinolin-7-yl)-2-methyl-3-propionyl-1H-pyrrol-1-yl)benzenesulfonamide.
17.4-(5-(4,4-dimethyl-2-oxo-1,2,3,4-tetrahydroquinolin-6-yl)-2-methyl-3-propionyl-1H-pyrrol-1-yl)benzenesulfonamide.
18.4-(5-(4,4-dimethyl-2-oxo-1,2,3,4-tetrahydroquinolin-7-yl)-2-methyl-3-propionyl-1H-pyrrol-1-yl)benzenesulfonamide.
19.4-(2-methyl-3-propionyl-5-(1,4,4-trimethyl-2-oxo-1,2,3,4-tetrahydroquinolin-6-yl)-1H-pyrrol-1-yl)benzenesulfonamide.
20.4-(2-methyl-3-propionyl-5-(5,6,7,8-tetrahydronaphthalen-2-yl)-1H-pyrrol-1-yl)benzenesulfonamide.

The compounds of general formula I where all the symbols are as defined earlier were prepared by method described below in scheme 1. However, the invention may not be limited to these methods; the compounds may also be prepared by using procedures described for structurally related compounds in the literature.

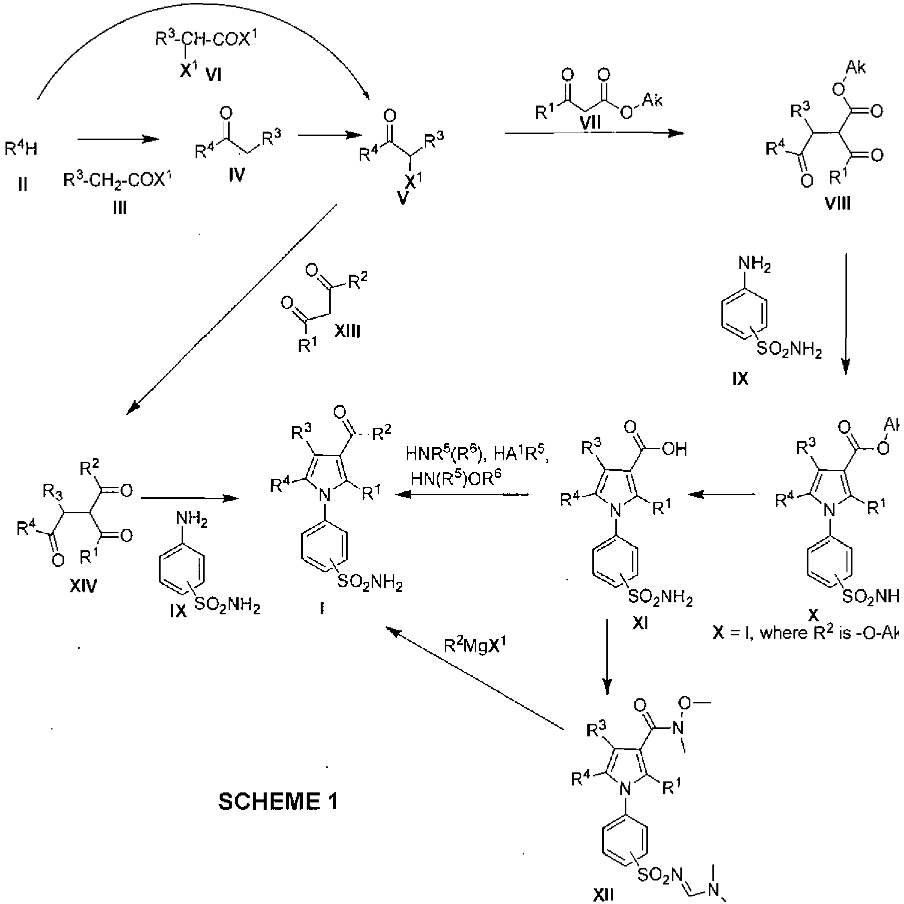

Compound of the formula I can be prepared starting from compounds represented by general formulae II and III by subjecting them to Friedal-Crafts reaction in the presence of Lewis acid as described in the literature EP 2168959 to give the Compounds of formula IV. Friedal Craft reaction can be carried out under different conditions well known in the art.

Alternatively, compound of formula IV can be prepared according to the appropriate procedure given in literature such as US 6313107, US5037825 and Journal of Med. Chemistry, 2006, 49,478 or the like.

Compound of the formula IV where symbols R⁴ is same as defined earlier in general formula and R³ is hydrogen, optionally substituted alkyl, optionally substituted cycloalkyl, optionally substituted aryl, optionally substituted heterocyclyl, optionally substituted heteroaryl, -OR⁶ where R⁶ is not selected as hydrogen undergo for halogenation to provide the compounds of formula V. Halogenation can be carried out under a condition adopting procedure generally used in the synthetic organic chemistry using bromine, iodine, N-halosuccinamide, sufuryl chloride, cupric chloride, cupric bromide or cupric iodide preferably bromine and cupric chloride using a solvent such as ethyl acetate, dichloromethane, methanol, THF, 1,4-dioxane and the like. Preferably dichloromethane or methanol are used.

Alternatively, Compounds of formula V can be prepared starting from compounds represented by general formulae II by reacting it with compound VI under Friedal-Crafts condition in the presence of Lewis acid such as AlCl₃ and the like as described in the literature EP 2168959 to give the compound of formula V. Friedal Craft reaction can be carried out under different conditions well known in the art.

Compound of formula V where symbols R³ and R⁴ are same as defined for compound IV, and X¹ is halogen when treated with base such as potassium carbonate, sodium hydride, preferably pulverized sodium under room temperature to heated conditions in a solvent such as THF, an aromatic hydrocarbon such as benzene, toluene and the like. Preferably toluene and compound of the formula VII where R¹ is optionally substituted alkyl, perhaloalkyl, optionally substituted cycloalkyl, optionally substituted aryl, optionally substituted heterocyclyl, optionally substituted heteroaryl, provide diketo ester compound VIII.

Compound of the formula VII can be prepared according to the procedure given in literature such as Chem. Pharm. Bull. 1982, 30, 2590 and J. of Med. Chem., 1997, 40, 547.

Compound VIII where symbols R¹, R³, R⁴ are same as defined earlier was treated with substituted aniline of formula IX under heating conditions in a solvent such as acetic acid and the like to obtain compound of the formula X.

The compounds of the formula X when R³ = H can be functionalized by electrophilic reagents such as but not limited to I₂, HNO₂, HCHO which would further lead to the formation of compounds of formula X having R³ = aryl, nitro, amino, amino alkyl, halo, hydroxy or cyano by using common functional group transformation procedure well known in the art.

Ester hydrolysis of compound of the formula X gave compound of formula XI. Ester hydrolysis may be carried out using standard procedure generally used in synthetic organic chemistry or well known in the art with reagents such as sodium hydroxide, potassium hydroxide, lithium hydroxide or the like in solvents such as alcohol, THF or the like. Preferably, aqueous solution of sodium hydroxide and ethanol were used for this reaction.

Compound of formula XI where R¹, R³, R⁴ are same as defined earlier was further converted to its corresponding acid chloride using standard procedure known in synthetic organic chemistry or preferably by reaction with oxalyl chloride in dichloromethane along with DMF followed by reaction with *N,O-*dimethylhydroxylamine hydrochloride and triethylamine in dichloromethane to provide compound of formula XII.

Compound of the formula XII was treated with Grignard reagent R²MgX¹ where R² selected from optionally substituted alkyl, optionally substituted heteroalkyl, optionally substituted aryl, optionally substituted heteroaryl, optionally substituted cycloalkyl or optionally substituted heterocyclyl, and X¹ is halogen gave compound of formula I, where R² is optionally substituted alkyl, optionally substituted heteroalkyl, optionally substituted aryl, optionally substituted heteroaryl, optionally substituted cycloalkyl or optionally substituted heterocyclyl. The reaction may be carried out as per the procedure given in literature such as J. Med. Chem., 2009, 52,3377.

Compound of formula XI was alternatively reacted with HNR⁵(R⁶), HA¹R⁵, HN(R⁵)OR⁶ where R⁵, R⁶ and A¹ are same as defined under the general formula I to provide compound of the formula I where R² is -NR⁵(R⁶), -A¹R⁵, -N(R⁵)OR⁶. The reaction was carried out according to the conditions known in converting carboxylic acids to amides and esters as known to one skilled in the art. The reaction may be carried out in the presence of solvents, for example DMF, THF, a halogenated hydrocarbon such as chloroform and dichloromethane, an aromatic hydrocarbon such as xylene, benzene, toluene, or the like, in the presence of suitable base such as triethylamine, diisopropylethylamine, pyridine or mixtures thereof or the like at a temperature between 0-50°C using reagents such as 1-(3-dimethylaminopropyl)-3-ethylcarbodimide hydrochloride (EDCI), 1,3-dicyclohexylcarbodiimide (DCC), auxiliary reagents such as 1-hydroxy-7-azabenzotriazole (HOAT), hydroxybenzotriazole hydrate (HOBT) or the like.

Alternatively, the compounds of the formula 1 where R³ = H; R² is selected from optionally substituted alkyl, optionally substituted heteroalkyl, optionally substituted aryl, optionally substituted heteroaryl, optionally substituted cycloalkyl or optionally substituted heterocyclyl; R¹ is optionally substituted alkyl, perhaloalkyl, optionally substituted cycloalkyl, optionally substituted aryl, optionally substituted heterocyclyl, optionally substituted heteroaryl; and R⁴ is same as defined earlier was prepared from compound of the formula V where R³ is H, R⁴ is same as defined under generic formula I, and X¹ is halogen by reacting it with compound of the formula XIII where R¹ is same as defined earlier and R² is same as defined earlier excluding NR⁵R⁶, -A¹R⁵, -N(R⁵)OR⁶ to give the compound XIV where R³ is H; R² is optionally substituted alkyl, optionally substituted heteroalkyl, optionally substituted aryl, optionally substituted heteroaryl, optionally substituted cycloalkyl or optionally substituted heterocyclyl; R¹ and R⁴ are same as defined earlier in the generic formula I. The reaction may be carried out in the presence of base such as potassium carbonate, sodium hydride, preferably pulverized sodium in a solvent such as THF, an aromatic hydrocarbon such as benzene, toluene or the like, preferably toluene is used.

Cyclization of compound of formula XIV with substituted aniline of formula IX under heating conditions in a solvent such as acetic acid or the like gave compound of formula I.

Compound of the formula XIII was be prepared according to the procedure given in literature such as J. Amer. Chem. Soc. 1945, 67, 9, 1510-1512.

Compound of the formula I where R¹ is hydrogen, R², R³ and R⁴ are same as defined earlier can be synthesized by adopting the chemistry described in Tetrahedron Letters, 1982, 23, 37, 3765-3768 and Helvetica Chimica Acta, 1998, 81, 7, 1207-1214.

Compound of the formula I where R¹ = H, R², R³ and R⁴ are same as defined earlier can be converted to compound of the formula I where R¹ is Halogen, R², R³ and R⁴ are same as defined earlier by halogenation. Halogenation can be carried out under a condition according to a procedure generally used in the synthetic organic chemistry using bromine, iodine, NCS, NBS, NIS, sufuryl chloride, cupric chloride, cupric bromide or cupric iodide preferably bromine and cupric chloride using a solvent such as ethyl acetate, dichloromethane, methanol, THF, 1,4 dioxane , and preferably dichloromethane or methanol.

Compound of formula II where R⁴ is same as defined under compound I can be prepared using process reported in the literature such as J.Med. Chem, 1985, 28, 1, 116-124, Monatshefte fur chemie, 1996, 127, 275-290, J.Med. Chem,, 1997, 40, 16, 2445-2451, US 4808597 and Eur. J. of Med. Chem., 2008, 43, 8, 1730 - 1736, or the like.

Process for synthesis of some of the typical intermediates of formula II is provided hereinbelow in scheme 2.

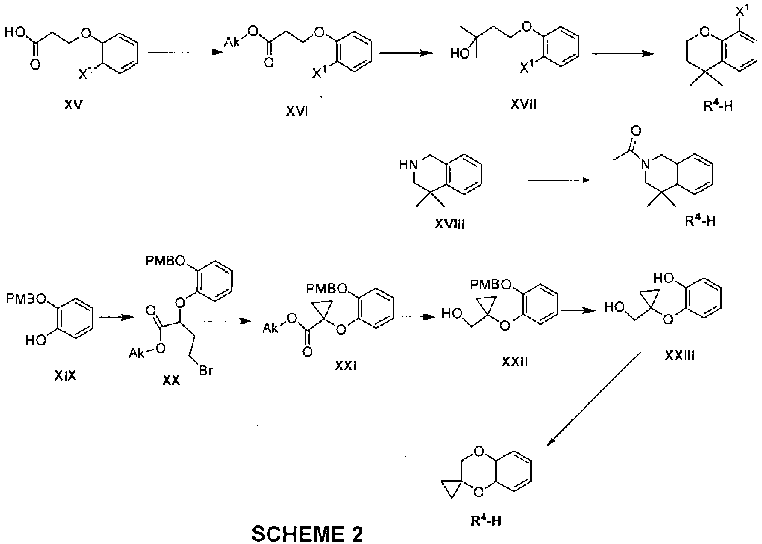

Compound XVII was prepared starting from compounds represented by general formula XV where X¹ is halo, by esterification of carboxylic acid with alcohol in the presence of inorganic acid such as but not limited to catalytic H₂SO₄ under room temperature to heated condition as described in the literature like Journal of the American Chemical Society, 1944, 66, 914-17 to obtain the Compounds of formula XVI. The compounds of the formula XVI was treated with Grignard reagent (MeMgX¹) to provide the compounds of formula XVII. The reaction may be carried out but not limited to the procedure given in literature such as J.Med. Chem, 2009, 52, 3377. The compound XVII was converted to compound of formula II where symbols R⁴ are same as defined for compound I by subjecting them to Friedal-Crafts reaction in the presence of Lewis acid as described in the literature (J.Med. Chem, 1985, 28, 1, 116 - 124).

The compounds of formula II where symbols R⁴ are same as defined for compound 1 was prepared from compound XVIII by acetylating using base such as but not limited to triethyl amine and acetyl chloride as described in J. Med. Chem, 2000, 43, 236-249.

The compound XXIII can be prepared starting from compounds represented by general formulae XIX by treatment of substituted phenol with alkyl 2,4-dibromobutanoate in the presence of base such as K₂CO₃ under room temperature to heated condition as described in the literature such as US2010076027 to give the compound of the formula XX. The compound of formula XX was converted to compound of formula XXI by cyclopropane ring formation using base such as but not limited to potassium t-butoxide as described in the literature such as US2010076027. The compound of formula XXI can be converted into compound of formula XXII using reducing reagent such as but not limited to LiAlH₄ as described in the literature Tetrahedron, 1994, 50, 15, 4311-4322; which was de-protected by method using reagents such as ceric ammonium nitrate, Trifluoromethane sulfonate BF₃-etherate but preferably by hydrogenation using catalytic palladium on carbon to give compound of formula XXIII. The compound XXIII was converted to compound of formula II where symbols R⁴ are same as defined for compound I by subjecting them to mitsunobu reaction in the presence of reagent such as but not limited to Diethyl azo dicarboxylate as described in the literature (Bioorganic & Medicinal Chemistry Letters, 2009, 19(3), 854 - 859).

The intermediates and the compounds of the present invention are obtained in pure form in a manner known *per se*, for example by distilling off the solvent in vacuum and re-crystallizing the residue obtained from a suitable solvent, such as pentane, diethyl ether, isopropyl ether, chloroform, dichloromethane, ethyl acetate, acetone or their combinations or subjecting it to one of the purification methods, such as column chromatography (eg. flash chromatography) on a suitable support material such as alumina or silica gel using eluent such as dichloromethane, ethyl acetate, hexane, methanol, acetone and their combinations. Preparative LC-MS method is also used for the purification of molecules described herein.

Salts of compound of formula I are obtained by dissolving the compound in a suitable solvent, for example in a chlorinated hydrocarbon, such as methyl chloride or chloroform or a low molecular weight aliphatic alcohol, for example, ethanol or isopropanol, which was then treated with the desired acid or base as described in Berge S.M. et al. "Pharmaceutical Salts, a review article in Journal of Pharmaceutical sciences volume 66, page 1-19 (1977)" and in handbook of pharmaceutical salts properties, selection, and use by P.H.Einrich Stahland Camille G.wermuth, Wiley- VCH (2002).

The stereoisomers of the compounds of formula I of the present invention may be prepared by stereospecific syntheses or resolution of the achiral compound using an optically active amine, acid or complex forming agent, and separating the diastereomeric salt/complex by fractional crystallization or by column chromatography.

The present invention further provides a pharmaceutical composition, containing the compounds of the general formula (I) as defined above, its tautomeric forms, its stereoisomers and its pharmaceutically acceptable salts in combination with the usual pharmaceutically employed carriers, diluents and the like are useful for the treatment and/or prophylaxis of diseases or disorder or condition such as Alzheimer's disease (AD), mild cognitive impairment (MCI), senile dementia, vascular dementia, dementia of Parkinson's disease, attention deficit disorder, attention deficit hyperactivity disorder (ADHD), dementia associated with Lewy bodies, AIDS dementia complex (ADC), Pick's disease, dementia associated with Down's syndrome, Huntington's disease, cognitive deficits associated with traumatic brain injury (TBI), cognitive and sensorimotor gating deficits associated with schizophrenia, cognitive deficits associated with bipolar disorder, cognitive impairments associated with depression, acute pain, post-surgical or post-operative pain, chronic pain, inflammation, inflammatory pain, neuropathic pain, smoking cessation, need for new blood vessel growth associated with wound healing, need for new blood vessel growth associated with vascularization of skin grafts, and lack of circulation, arthritis, rheumatoid arthritis, psoriasis, Crohn's disease, ulcerative colitis, pouchitis, inflammatory bowel disease, celiac disease, periodontitis, sarcoidosis, pancreatitis, organ transplant rejection, acute immune disease associated with organ transplantation, chronic immune disease associated with organ transplantation, septic shock, toxic shock syndrome, sepsis syndrome, depression, and rheumatoid spondylitis.

The present invention also provides a pharmaceutical composition, containing the compounds of the general formula (I) as defined above, its tautomeric forms, its stereoisomers and its pharmaceutically acceptable salts in combination with the usual pharmaceutically employed carriers, diluents and the like are useful for the treatment and/or prophylaxis of diseases or disorder or condition classified or diagnosed as major or minor neurocognitive disorders, or disorders arising due to neurodegeneration.

The present invention also provides a compound of formula I, as defined hereinabove in combination with or as adjunct to medications for use in the treatment of attention deficit hyperactivity disorders, schizophrenia, and other cognitive disorders such as Alzheimer's disease, Parkinson's dementia, vascular dementia or dementia associated with Lewy bodies, traumatic brain injury.

The present invention also provides a compound of formula I, as defined hereinabove in combination with or as an adjunct to acetylcholinesterase inhibitors, disease modifying drugs or biologics for neurodegenerative disorders, dopaminergic drugs, antidepressants, typical or an atypical antipsychotic for use in the treatment of a disease or disorder.

Accordingly, compound of formula I is useful for preventing or treating a disorder mediated by nicotinic acetylcholine receptors. Such compounds can be administered to a subject having such a disorder or susceptible to such disorders in a therapeutically effective amount. The compounds are particularly useful for a method of treating a mammal having a condition where modulation of nicotinic acetylcholine receptor activity is of therapeutic benefit, wherein the method is accomplished by administering a therapeutically effective amount of a compound of formula I to a subject having, or susceptible to, such a disorder. The term 'subject' used herein can be defined as any living organism capable of expressing α7 subunit of nicotinic acetylcholine receptor including mammals.

The following examples are provided to further illustrate the present invention and therefore should not be construed to limit the scope of the present invention. All ¹HNMR spectra were determined in the solvents indicated and chemical shifts are reported in δ units downfield from the internal standard tetramethylsilane (TMS) and interproton coupling constants are reported in Hertz (Hz).

### Example 1: Preparation of 4-(5-(4,4-dimethylchroman-6-yl)-2-methyl-3-propionyl-1H-pyrrol-1-yl)benzenesulfonamide.

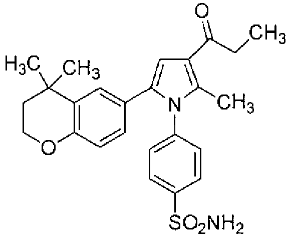

### Step 1: 2-Bromo-1-(4,4-dimethylchroman-6-yl)ethanone

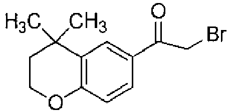

To a stirred solution of 1-(4,4-dimethylchroman-5-yl)ethanone (prepared according to the procedure reported in J.Med. Chem, 1985, 28, 1, 116-124, 2.0g, 9.80 mmol) in methanol (30 ml.) was added bromine (1.57g, 0.5 ml, 9.80 mmol) in a dropwise manner at 10°C. The resulting mixture was stirred at room temperature for 2 hr. The completion of reaction was monitored by TLC. Water (10 ml) was added to it and resultant mixture was stirred for 45 minutes at room temperature. Solvent was evaporated at reduced pressure. Residue so obtained was taken in ehyl acetate (100 ml), washed with water (25 ml) followed by brine (25 ml). Combined organic layer was dried over anhydrous Na₂SO₄. The solvent was evaporated under reduced pressure to obtain a crude product; which was purified by column chromatography over silica gel (100-200 mesh) using 20% ethyl acetate in hexanes as an eluent to yield the title compound (2.3 g, 83%)
MS: *m*/*z* 283 (M+1)
¹HNMR (CDCl₃, 400 MHz): δ 7.98 (d, *J*=2.4Hz, 1H), 7.70 (dd, *J*=8.4,2.4 Hz, 1H), 6.82 (d, *J*=8.4Hz, 1H), 4.38 (s, 2H), 4.26 (dt, *J*=4.4, 1.2 Hz, 2H), 1.85 (dt, *J*=4.4, 1.2 Hz, 2H), 1.37 (s, 6H).

### Step 2: 3-Acetyl-1-(4,4-dimethylchroman-6-yl)hexane-1,4-dione

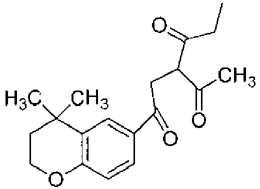

To the stirred solution of pulverized sodium (0.2g , 8.63 mmol) in toluene (40 ml) was added hexane-2,4-dione (prepared according to the procedure given in J. Amer. Chem. Soc., 1945, 67, 9, , 1510-1512, 0.894g, 7.85 mmol) at 0°C and reaction mixture was stirred at room temperature for 2 hr. To this was added solution of 2-bromo-1-(4,4-dimethylchroman-6-yl)ethanone (step 1, 2.0g , 7.07 mmol) in toluene (10 ml) and reaction mixture was heated at 60°C for 2 hr under stirring. The completion of reaction was monitored by TLC. To this reaction mixture was added cold water (15 ml) and extracted with ethyl acetate (2x100 ml) and the combined organic layer was dried over anhydrous Na₂SO₄. The solvent was evaporated under reduced pressure to obtain a crude product; which was purified by column chromatography over silica gel (100-200 mesh) using 20% ethyl acetate in hexanes as an eluent to yield the title compound (1.4g, 62.78%).
MS: *m*/*z* 317 (M+1)
¹HNMR (CDCl₃, 400 MHz): δ 7.90 (d, *J*=2.4Hz, 1H), 7.68 (dd, *J*=8.4,2.4 Hz, 1H), 6.79 (d, *J*=8.4Hz, 1H), 4.3 (t, *J*=6.8Hz, 1H), 4.25 (dt, *J*=4.4, 1.2 Hz, 2H), 3.52 (d, *J*=6.8Hz, 2H), 2.67 (q, *J*=7.2Hz, 2H), 2.31 (s, 3H), 1.84 (dt, *J*=4.4, 1.2 Hz, 2H), 1.33 (s, 6H), 1.08 (t, *J*=7.2Hz, 3H).

### Step 3: 4-(5-(4,4-dimethylchroman-6-yl)-2-methyl-3-propionyl-1H-pyrrol-1-yl)benzenesulfonamide

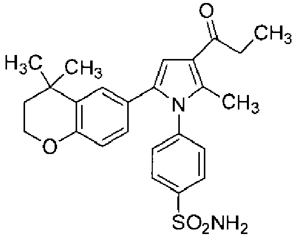

A mixture of 3-acetyl-1-(4,4-dimethylchroman-6-yl)hexane-1,4-dione (step 2, 1.3g , 4.11 mmol) and 4-aminobenzenesulfonamide (0.7g, 4.11mmol) in acetic acid (5 ml) was heated at 110° C for 3 hr. The completion of reaction was monitored by TLC. Solvent was evaporated at reduced pressure. Residue so obtained was taken in solution of ammonia in chloroform (20 ml) and stirred for 10 minutes. Reaction mixture was again concentrated at reduced pressure. Ethyl acetate (100 ml) was added to the residue, washed with water (10 ml). Combined organic layer was dried over anhydrous Na₂SO₄. The solvent was evaporated under reduced pressure to obtain a crude product; which was purified by column chromatography over silica gel (100-200 mesh) using 4% methanol in dichloromethane as an eluent to yield the title compound (0.460 g, 24.8%)
MS: *m*/*z* 453 (M+1)
¹HNMR (CDCl₃, 400 MHz): δ 7.95 (d, *J*=8.8Hz, 2H), 7.26 (d, *J*=8.8,Hz, 2H), 6.89 (dd, *J*=8.4, 2.0 Hz, 1H), 6.64 (m, 3H), 5.07 (bs, exchanged with D₂O 2H), 4.09 (t, *J*=5.2Hz, 2H), 2.85 (q, *J*=7.2Hz, 2H), 2.41 (s, 3H), 1.70 (t, *J*= 5.2 Hz, 2H), 1.19 (t, *J*=7.2Hz, 3H), 1.00 (s, 6H).

### Example 2: Preparation of 4-(5-(4,4-dimethylchroman-6-yl)-2-methyl-3-propionyl-1H-pyrrol-1-yl)benzenesulfonamide [Alternative Method]

### Step 1: Ethyl 2-acetyl-4-(4,4-dimethylchroman-6-yl)-4-oxobutanoate

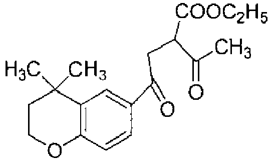

To the stirred solution of pulverized sodium (0.35g, 15.61 mmol) in toluene (40 ml) was added ethyl-3-oxobutanoate (3.05g, 2.97 ml, 23.46 mmol) at 0°C and reaction mixture was stirred at room temperature for 2 hr. To this was added solution of 2-bromo-1-(4,4-dimethylchroman-6-yl)ethanone (4.41g , 15.61 mmol) in toluene (25ml) and reaction mixture was stirred at room temperature for 2 hr . The completion of reaction was monitored by TLC. To this reaction mixture was added cold water (30 ml) and extracted with ethyl acetate (2x250 ml) and the combined organic layer was dried over anhydrous Na₂SO₄. The solvent was evaporated under reduced pressure to obtain a crude product; which was purified by column chromatography over silica gel (100-200 mesh) using DCM as an eluent to yield the title compound (3.5g, 67.3%).
MS: *m*/*z* 333 (M+1)

### Step 2: Ethyl 5-(4,4-dimethylchroman-6-yl)-2-methyl-1-(4-sulfamoylphenyl)-1H-pyrrole-3-carboxylate

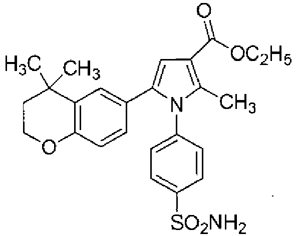

A mixture of ethyl 2-acetyl-4-(4,4-dimethylchroman-6-yl)-4-oxobutanoate (Step 1, 3.5g, 10.53 mmol) and 4-aminobenzenesulfonamide (2.18g, 12.64 mmol) in acetic acid (35 ml) was heated at 110° C for 15 hr. The completion of reaction was monitored by TLC. Reaction mixture was concentrated at reduced pressure. Ethyl acetate (250 ml) was added to the residue, washed with water (1x 30 ml). Organic layer was dried over anhydrous Na₂SO₄. The solvent was evaporated under reduced pressure to obtain a crude product; which was purified by column chromatography over silica gel (100-200 mesh) using 0.1% methanol in dichloromethane as an eluent to yield the title compound (2.5 g, 50.80%)
MS: *m*/*z* 469 (M+1)

### Step 3: 5-(4,4-dimethylchroman-6-yl)-2-methyl-1-(4-sulfamoylphenyl)-1H-pyrrole-3-carboxylic acid

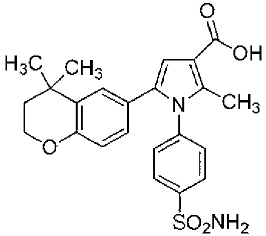

Ethyl 5-(4,4-dimethylchroman-6-yl)-2-methyl-1-(4-sulfamoylphenyl)-1*H*-pyrrole-3-carboxylate (Step 2, 2.5g , 5.34 mmol) was suspended in ethanol (100 ml) and treated with 2M solution of NaOH (25 ml) at 0°C. the reaction mixture was refluxed for 3 hr. The completion of reaction was monitored by TLC. Reaction mixture was concentrated at reduced pressure. Residue was diluted with water (10 ml) and neutralized with 10 % HCl upto pH7, aqueous layer was extracted with ethyl acetate (2 x 100 ml). Combined organic layer was dried over anhydrous Na₂SO₄. The solvent was evaporated under reduced pressure to obtain a product. (1.7g, 72.3%)
MS: *m*/*z* 441 (M+1)

### Step 4: 1-(4-(N-((dimethylamino)methylene)sulfamoyl)phenyl)-5-(4,4-dimethylchroman-6-yl)-N-methoxy-N,2-dimethyl-1H-pyrrole-3-carboxamide

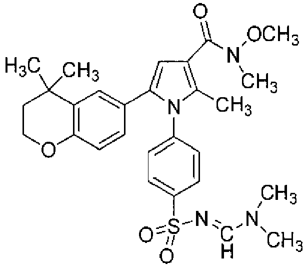

Oxalyl chloride (0.98g, 0.65ml, 7.72 mmol) was added dropwise at 0°C to a solution of 5-(4,4-dimethylchroman-6-yl)-2-methyl-1-(4-sulfamoylphenyl)-1*H-*pyrrole-3-carboxylic acid (step 3,1.7g, 3.86 mmol) in dichloromethane (100 ml)and DMF (0.56g, 0.59 ml,7.72 m mol). Mixture was allowed to come at room temperature and stirred for 2 hr. under nitrogen atmosphere. The completion of reaction was monitored by TLC. The mixture was concentrated under reduced pressure and used directly for further reaction.

To this residue was added *N*,*O*-dimethylhydroxylamine hydrochloride (0.75g, 7.72 mmol) in dry dichloromethane (50 ml) at 0°C followed by the addition of triethylamine (1.56g, 2.05 ml, 15.44 mmol,) under stirring. The reaction mixture was stirred at room temperature for 2 hr. The completion of reaction was monitored by TLC. The solvent was removed under reduced pressure. The residue so obtained was taken in dichloromethane (100 ml), washed with water (2x 10 ml.) and organic layers separated were dried over anhydrous sodium sulphate, filtered and concentrated at reduced pressure to get a crude product. This crude product was purified by column chromatography over silica gel (100-200 mesh) using 0.2% methanol in dichloromethane as an eluent to yield the title compound (1.67g, 80.6%).
MS: *m*/*z* 539 (M+1)

### Step 5: 4-(5-(4,4-dimethylchroman-6-yl)-2-methyl-3-propionyl-1H-pyrrol-1-yl)benzenesulfonamide

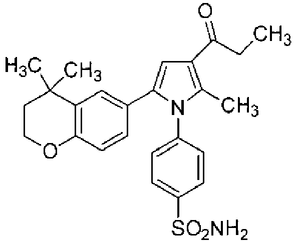

To a solution of 1-4-(*N*-((dimethylamino)methylene)sulfamoyl)phenyl)-5-(4,4-dimethylchroman-6-yl)-*N*-methoxy-*N*,2-dimethyl-1H-pyrrole-3-carboxamide (Step 4, 1.67g , 3.10 mmol) in anhydrous THF (25 ml) at 0°C, Grignard reagent [ethyl magnesium bromide, 2.06g , 15.5 ml (1 M soln. in THF), 15.52 mmol] was added dropwise and reaction mixture was heated to reflux for 30 minutes. The completion of reaction was monitored by TLC. After cooling, reaction mixture was quenched by addition of solution of saturated ammonium chloride (20 ml) and extracted with ethyl acetate (2 x100 ml) . Combined organic layer was dried over anhydrous Na₂SO₄. The solvent was evaporated under reduced pressure to obtain a crude product; which was purified by column chromatography over silica gel (100-200 mesh) using 0.1% methanol in dichloromethane as an eluent to yield the title compound which was finally purified by preparative HPLC (0.100g, 7.1 %)
MS: *m*/*z* 453 (M+1)
¹HNMR (CDCl₃, 400 MHz): δ 7.95 (d, *J*=8.8Hz, 2H), 7.27 (d, *J*=8.8,Hz, 2H), 6.90 (dd, *J*=8.4, 2.0 Hz, 1H), 6.65 (m, 3H), 4.90 (bs, exchanged with D₂O 2H), 4.11 (t, *J*=5.2Hz, 2H), 2.86 (q, *J*=7.2Hz, 2H), 2.44 (s, 3H), 1.71 (t, *J*= 5.2 Hz, 2H), 1.21(t, *J*=7.2Hz, 3H), 1.02 (s, 6H).

**Example 3:** Following compounds of the present inventions were prepared using a process analogous to Example 1 and 2 by appropriately changing the reactants required.

### 4-(5-(2,3-dihydrobenzo[b][1,4]dioxin-6-yl)-2-methyl-3-propionyl-1 H-pyrrol-1-yl)benzenesulfonamide

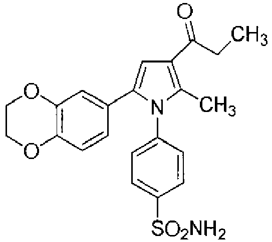

MS: *m*/*z* 427 (M+1),
¹HNMR (CDCl₃, 400 MHz): δ 7.96 (d, *J*=8.4Hz, 2H), 7.28 (d, *J*=8.4,Hz, 2H), 6.64-6.66 (m, 2H), 6.58 (d, *J*=2.0 Hz, 1H), 6.40 (dd, *J*=8.4, 2.0 Hz, 1H), 4.87 (bs, exchanged with D₂O 2H), 4.19-4.22 (m, 4H), 2.86 (q, *J*=7.2Hz, 2H), 2.42 (s, 3H), 1.20 (t, *J*=7.2Hz, 3H).

### 4-(2-(2,3-dihydrobenzo[b][1,4]dioxin-6-yl)-3,5-dimethyl-4-propionyl-1 H-pyrrol-1-yl)benzenesulfonamide

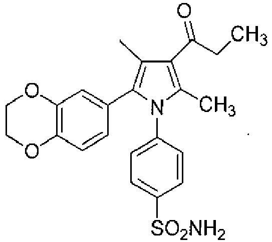

MS: m/z 441 (M+1);
¹HNMR (CDCl₃, 400 MHz): δ 7.87 (d, *J*=8.4Hz, 2H), 7.19 (d, J=8.4,Hz, 2H), 6.67 (d, J=8.0 Hz, 1H), 6.56 (d, *J*=2.0 Hz, 1H), 6.39 (dd, *J*=8.0, 2.0 Hz, 1H), 4.88 (bs, exchanged with D₂O 2H), 4.13-4.22 (m, 4H), 2.86 (q, *J*=7.2Hz, 2H), 2.33 (s, 3H), 2.24 (s, 3H), 1.20 (t, *J*=7.2Hz, 3H).

### Ethyl 5-(2,3-dihydrobenzo[b][1,4]dioxin-6-yl)-2,4-dimethyl-1-(4-sulfamoylphenyl)-1H-pyrrole-3-carboxylate

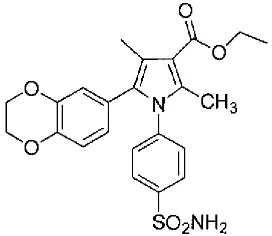

MS: *m*/*z* 457 (M+1),
¹HNMR (CDCl₃, 400 MHz): δ 7.87 (d, *J*=8.4Hz, 2H), 7.17 (d, *J*=8.4,Hz, 2H), 6.65 (d, *J*=8.4 Hz, 1H), 6.56 (d, *J*=2.0 Hz, 1H), 6.39 (dd, *J*=8.4, 2.0 Hz, 1H), 4.95 (bs, exchanged with D₂O 2H), 4.22 (q, *J*=6.8Hz, 2H), 4.14-4.20 (m, 4H), 2.35 (s, 3H), 2.22 (s, 3H), 1.36 (t, *J*=6.8Hz, 3H).

### 4-(2-methyl-3-propionyl-5-(5,6,7,8-tetrahydronaphthalen-2-yl)-1H-pyrrol-1-yl)benzenesulfonamide.

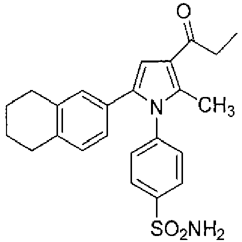

MS: m/z 423 (M+1),
¹HNMR (CDCl₃, 400 MHz): δ 7.95 (d, *J*=8.4Hz, 2H), 7.28 (d, *J*=8.4,Hz, 2H), 6.80-6.83 (m, 2H), 6.67 (s, 1H), 6.59 (dd, *J*=8.0, 2.0 Hz, 1H), 4.99 (bs, exchanged with D₂O 2H), 2.87 (q, *J*=7.2Hz, 2H), 2.60-2.68 (m, 4H), 2.42 (s, 3H), 1.72-1.75 (m, 4H), 1.20 (t, *J*=7.2Hz, 3H).

### Example 4: Preparation of 4-(5-(2,3-dihydro-1H-inden-4-yl)-2-methyl-3-propionyl-1H-pyrrol-1-yl)benzenesulfonamide.

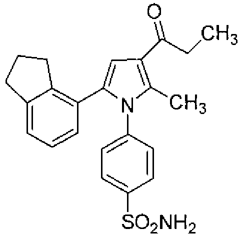

### Step 1: 2-bromo-1-(2,3-dihydro-1H-inden-4-yl)ethanone

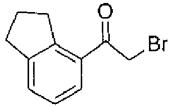

To a stirred solution of 1-(2,3-dihydro-1H-inden-4-yl)ethanone (prepared according to the procedure reported in Monatshefte fur chemie 1996, 127, 275-290, 0.8 gm, 5.00 mmol) in diethyl ether (8 ml) were added AlCl₃ (0.73 gm, 5.5 mmol) and bromine (0.96 gm, 0.31 ml, 6.00 mmol) in a drop wise manner at 0°C. The resulting mixture was stirred at room temperature for 1 hr. The completion of reaction was monitored by TLC. Reaction mixture was poured into cold water (10 ml). Aqueous layer was extracted with ethyl acetate (2 x 30 ml). Organic layers separated were dried over anhydrous sodium sulphate, filtered and concentrated at reduced pressure to get a crude product; which was purified by column chromatography using 1% ethyl acetate in hexanes as an eluent to yield the title compound (0.76 gm, 63.8%).
MS: *m*/*z* 240 (M+1),
¹HNMR (CDCl₃, 400 MHz): δ 7.66 (d, *J*=7.2 Hz, 1H), 7.43 (d, *J*=7.2 Hz, 1H), 7.23-7.27 (m, 1H), 4.49 (s, 2H), 3.25 (t, *J*=7.6 Hz, 2H), 2.92 (t, *J*=7.6 Hz, 2H), 2.08 (quintet, *J*=7.6 Hz, 2H).

### Step 2: 3-acetyl-1-(2,3-dihydro-1H-inden-4-yl)hexane-1,4-dione

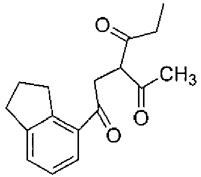

To the stirred solution of pulverized sodium (0.046 gm, 2.00 mmol) in toluene (5 ml) was added hexane-2,4-dione (prepared according to the procedure given in J. Amer. Chem. Soc., 1945, 67, 9, , 1510-1512, 0.21 gm, 1.85 mmol) at 0°C and reaction mixture was stirred at room temperature for 2 hr. To this was added solution of 2-bromo-1-(2,3-dihydro-1H-inden-4-yl)ethanone (step 1, 0.4 gm, 1.67 mmol) in toluene (5 ml) and reaction mixture was heated at 60°C for 2 hr under stirring. The completion of reaction was monitored by TLC. To this reaction mixture was added cold water (5 ml) and extracted with ethyl acetate (2x30 ml) and the combined organic layer was dried over anhydrous Na₂SO₄. The solvent was evaporated under reduced pressure to obtain a crude product; which was purified by column chromatography using 10% ethyl acetate in hexanes as an eluent to yield the title compound (0.196 gm, 39.12%).
MS: *m*/*z* 273 (M+1),
¹HNMR (CDCl₃, 400 MHz): δ 7.72 (d, *J*=7.2 Hz, 1H), 7.41 (d, *J*=7.2 Hz, 1H), 7.23-7.26 (m, 1H), 4.36 (t, *J*=7.2 Hz, 1H), 3.56 (d, *J*=7.2 Hz, 2H), 3.23 (t, *J*=7.6 Hz, 2H), 2.91 (t, *J*=7.6 Hz, 2H), 2.69 (q, *J*=7.6 Hz, 2H), 2.31 (s, 3H), 2.07 (quintet, *J*=7.2 Hz, 2H), 1.08 (t, *J*=7.2 Hz, 3H).

### Step 3: 4-(5-(2,3-dihydro-1H-inden-4-yl)-2-methyl-3-propionyl-1H-pyrrol-1-yl)benzenesulfonamide

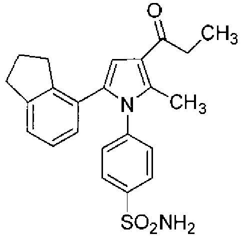

To the solution of 3-acetyl-1-(2,3-dihydro-1H-inden-4-yl)hexane-1,4-dione (step 2, 0.18 gm, 0.68 mmol) in acetic acid (5 ml) was added 4-aminobenzenesulfonamide (0.12 gm, 0.68 mmol) at room temperature. Reaction mixture was heated at 110° C for 3 hr. The completion of reaction was monitored by TLC. Solvent was evaporated at reduced pressure. Residue so obtained was taken in solution of ammonia in chloroform (10 ml) and stirred for 10 minutes. Reaction mixture was again concentrated at reduced pressure. Ethyl acetate (30 ml) was added to the residue, washed with water (5 ml). Combined organic layer was dried over anhydrous Na₂SO₄. The solvent was evaporated under reduced pressure to obtain a crude product; which was purified by column chromatography using 5% methanol in DCM as an eluent to yield the title compound (0.041 gm, 14.8%).
MS: *m*/*z* 409 (M+1),
¹HNMR (DMSO, 400 MHz): δ 7.80 (d, *J*=8.4 Hz, 2H), 7.48 (bs-exchanges with D₂O, 2H), 7.41 (d, *J*=8.4 Hz, 2H), 7.07 (d, *J*=7.6 Hz, 1H), 6.93 (t, *J*=7.6 Hz, 1H), 6.79 (s, 1H), 6.64 (d, *J*=7.6 Hz, 1H), 2.77-2.85 (m, 6H), 2.34 (s, 3H), 1.91 (quintet, *J*=7.2 Hz, 2H), 1.08 (t, *J*=7.2 Hz, 3H).

### Example 5: Preparation of 4-(5-(2,2-dimethylchroman-6-yl)-2-methyl-3-propionyl-1H-pyrrol-1-yl)benzenesulfonamide.

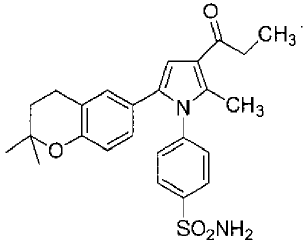

### Step 1: 2-bromo-1-(2,2-dimethylchroman-6-yl)ethanone

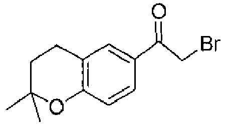

To a stirred solution of 1-(2,2-dimethylchroman-6-yl)ethanone (prepared according to the procedure reported in J.Med. Chem,, 1997, 40, 16, 2445-2451, 2.5 gm, 12.25 mmol) in methanol (25 ml) was and bromine (1.96 gm, 0.63ml, 12.25 mmol) in a drop wise manner at 0°C. The resulting mixture was stirred at room temperature for 2 hr. The completion of reaction was monitored by TLC. Reaction mixture was concentrated at reduced pressure and dissolved in DCM (100 ml). Organic layer was washed with water (2x 25 ml), dried over anhydrous sodium sulphate, filtered and concentrated at reduced pressure to get a crude product; which was purified by column chromatography using 1% ethyl acetate in hexanes as an eluent to yield the title compound (1.50 gm, 43.22%).
MS: *m*/*z* 284 (M+1),
¹HNMR (CDCl₃, 400 MHz): δ 7.71-7.76 (m, 2H), 6.80 (d, *J*=8.4 Hz, 1H), 4.35 (s, 2H), 2.82 (t, *J*=6.8 Hz, 2H), 1.84 (t, *J*=6.8 Hz, 2H), 1.35 (s, 6H).

### Step 2: 3-acetyl-1-(2,2-dimethylchroman-6-yl)hexane-1,4-dione

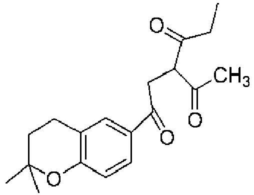

To the stirred solution of pulverized sodium (0.37 gm, 16.08 mmol) in toluene (10 ml) was added hexane-2,4-dione (prepared according to the procedure given in J. Amer. Chem. Soc., 1945, 67, 9, , 1510-1512, 1.82 gm, 15.96 mmol) at 0°C and reaction mixture was stirred at room temperature for 2 hr. To this was added solution of 2-bromo-1-(2,2-dimethylchroman-6-yl)ethanone (step 1, 3.0 gm, 10.60 mmol) in toluene (10 ml) and reaction mixture was heated at 60°C for 2 hr under stirring. The completion of reaction was monitored by TLC. To this reaction mixture was added cold water (15 ml) and extracted with ethyl acetate (2x 100 ml) and the combined organic layer was dried over anhydrous Na₂SO₄. The solvent was evaporated under reduced pressure to obtain a crude product; which was purified by column chromatography using 5% ethyl acetate in hexanes as an eluent to yield the title compound (1.00 gm, 29.9%).
MS: *m*/*z* 317 (M+1),
¹HNMR (CDCl₃, 400 MHz): δ 7.67-7.77 (m, 2H), 6.72-6.79 (m, 1H), 4.36 (t, *J*=6.8 Hz, 1H), 3.51 (d, *J*=6.8 Hz, 2H), 2.72-2.85 (m, 4H), 2.31 (s, 3H), 1.82 (q, *J*=7.2 Hz, 2H), 1.35 (s, 6H), 1.06 (t, *J*=7.2 Hz, 3H).

### Step 3: 4-(5-(2,2-dimethylchroman-6-yl)-2-methyl-3-propionyl-1H-pyrrol-1-yl)benzenesulfonamide

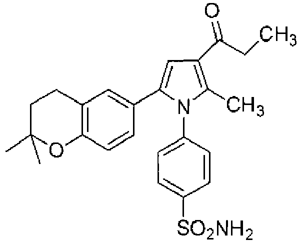

To the solution of 3-acetyl-1-(2,2-dimethylchroman-6-yl)hexane-1,4-dione (step 2, 0.33 gm, 1.05 mmol) in acetic acid (5 ml) was added 4-aminobenzenesulfonamide (0.22 gm, 1.25 mmol) at room temperature. Reaction mixture was heated at 110° C for 3 hr. The completion of reaction was monitored by TLC. Solvent was evaporated at reduced pressure. Residue so obtained was taken in solution of ammonia in chloroform (10 ml) and stirred for 10 minutes. Reaction mixture was again concentrated at reduced pressure. Ethyl acetate (30 ml) was added to the residue, washed with water (5 ml). Combined organic layer was dried over anhydrous Na₂SO₄. The solvent was evaporated under reduced pressure to obtain a crude product; which was purified by column chromatography using 5% methanol in DCM as an eluent to yield the title compound (0.10 gm, 21.27%).
MS: *m*/*z* 453 (M+1),
¹HNMR (DMSO, 400 MHz): δ 7.87 (d, *J*=8.4 Hz, 2H), 7.50 (bs-exchanges with D₂O, 2H), 7.44 (d, *J*=8.4 Hz, 2H), 6.93 (d, *J*=2.0 Hz, 1H), 6.78 (s, 1H), 6.57 (dd, *J*=8.4, 2.0 Hz, 1H), 6.47 (d, *J*=8.4 Hz, 1H), 2.82 (q, *J*=7.2 Hz, 2H), 2.60 (t, *J*=6.8 Hz, 2H), 2.31 (s, 3H), 1.70 (t, *J*=6.8 Hz, 2H), 1.22 (s, 6H), 1.07 (t, *J*=7.2 Hz, 3H).

### Example 6: Preparation of 4-(5-(8-fluoro-4,4-dimethylchroman-6-yl)-2-methyl-3-propionyl-1H-pyrrol-1-yl)benzenesulfonamide.

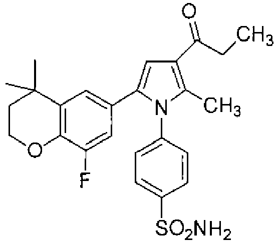

### Step 1: Methyl 3-(2-fluorophenoxy)propanoate

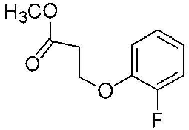

To a stirred solution of 3-(2-fluorophenoxy)propanoic acid (prepared according to the procedure reported in WO2010013794, 14.0 gm, 76.08 mmol) in methanol (140 ml) was added thionyl chloride (13.57 gm, 8.5 ml, 114.12 mmol) in a drop wise manner at 0°C. The resulting mixture was stirred at room temperature for 2 hr. The completion of reaction was monitored by TLC. Reaction mixture was concentrated at reduced pressure and dissolved in Ethyl acetate (300 ml). Organic layer was washed with water (2x 50 ml), dried over anhydrous sodium sulphate, filtered and concentrated at reduced pressure to get a crude product; which was purified by column chromatography using 20% ethyl acetate in hexanes as an eluent to yield the title compound (12.9 gm, 85.66%).
MS: *m*/*z* 221 (M+23),
¹HNMR (CDCl₃, 400 MHz): δ 6.88 - 7.09 (m, 4H), 4.32 (t, *J*=6.4 Hz, 2H), 3.72 (s, 3H), 2.84 (t, *J*=6.4 Hz, 2H).

### Step 2: 4-(2-fluorophenoxy)-2-methylbutan-2-ol

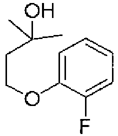

To a stirred solution of methyl 3-(2-fluorophenoxy)propanoate (Step-1, 12.0 gm, 60.60 mmol) in THF (25 ml) was added methyl magnesium bromide (21.67 gm, 60.72 ml 3M solution in diethyl ether, 181.80 mmol) in a drop wise manner at 0°C under nitrogen atmosphere. The resulting mixture was stirred at 90°C for 2 hr. The completion of reaction was monitored by TLC. Reaction mixture was quenched by addition of saturated NH₄Cl solution (100 ml). Aquous layer was extracted with ethyl acetate (2x 200 ml). Organic layers was washed with water (2x 50 ml), dried over anhydrous sodium sulphate, filtered and concentrated at reduced pressure to get a crude product; which was purified by column chromatography using 12% ethyl acetate in hexanes as an eluent to yield the title compound (8.60 gm, 71.66%).
MS: *m*/*z* 221 (M+23),
¹HNMR (CDCl₃, 400 MHz): δ 6.88 - 7.09 (m, 4H), 4.24 (t, *J*=6.4 Hz, 2H), 2.35 (bs, exchanges with D₂O 1H), 2.02 (t, *J*=6.4 Hz, 2H), 1.31 (s, 6H).

### Step 3: 8-fluoro-4,4-dimethylchroman

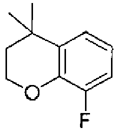

To a stirred solution of AlCl₃ (8.67 gm, 65.05 mmol) in nitromethane (50 ml) was added solution of 4-(2-fluorophenoxy)-2-methylbutan-2-ol (Step-2, 8.5 gm, 43.36 mmol) in nitromethane (20 ml) in a drop wise manner at 0°C. The resulting mixture was stirred at room temperature for 3 hr. The completion of reaction was monitored by TLC. Reaction mixture was quenched with 2N HCl (50 ml) at 0°C. Aqueous layer was extracted with ethyl acetate (2 x 100 ml). Organic layers separated were dried over anhydrous sodium sulphate, filtered and concentrated at reduced pressure to get a crude product; which was purified by column chromatography using 1% ethyl acetate in hexanes as an eluent to yield the title compound (5.80 gm, 74.35%).
MS: *m*/*z* No ionization,
¹HNMR (CDCl₃, 400 MHz): δ 7.03 - 7.76 (dt, J=1.6 Hz, 8.0 Hz, 1H), 6.85 - 6.88 (m, 1H), 6.77 - 6.8 (m, 1H), 4.24-4.26 (m, 2H), 1.85-1.87 (m, 2H), 1.33 (s, 6H).

### Step 4: 2-bromo-1-(8-fluoro-4,4-dimethylchroman-6-yl)ethanone

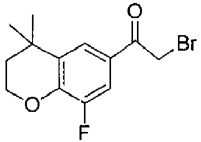

To a stirred solution of AlCl₃ (4.88 gm, 36.73 mmol) in DCE (60 ml) was added solution of 8-fluoro-4,4-dimethylchroman (Step-3, 5.8 gm, 32.22 mmol) in DCE (20 ml) and 2-bromoacetyl bromide (7.80 gm, 3.35 ml, 38.66 mmol) in a drop wise manner at 0°C. The resulting mixture was stirred at room temperature for 3 hr. The completion of reaction was monitored by TLC. Reaction mixture was quenched with water (70 ml) at 0°C. Aqueous layer was extracted with ethyl acetate (2 x 100 ml). Organic layers washed with 1N HCl (50 ml), water (50 ml). Organic layer separated was dried over anhydrous sodium sulphate, filtered and concentrated at reduced pressure to get a crude product; which was purified by column chromatography using 6% ethyl acetate in hexanes as an eluent to yield the title compound (6.20 gm, 64.18%).
MS: m/z 301 (M+1),
¹HNMR (CDCl₃, 400 MHz): δ 7.75 - 7.76 (m, 1H), 7.28 (d, *J*=11.2 Hz, 2 Hz, 1H), 4.33 -4.35 (m, 4H), 1.89 (dd, *J*=6.0, 5.6 Hz, 2H), 1.37 (s, 6H).

### Step 5: 3-acetyl-1-(8-fluoro-4,4-dimethylchroman-6-yl)hexane-1,4-dione

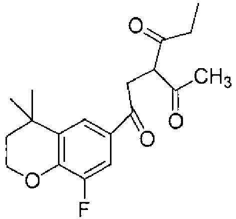

To the stirred solution of pulverized sodium (0.057 gm, 2.49 mmol) in toluene (5 ml) was added hexane-2,4-dione (prepared according to the procedure given in J. Amer. Chem. Soc., 1945, 67, 9, , 1510-1512, 0.23 gm, 1.99 mmol) at 0°C and reaction mixture was stirred at room temperature for 2 hr. To this was added solution of 2-bromo-1-(8-fluoro-4,4-dimethylchroman-6-yl)ethanone (step 4, 0.5 gm, 1.66 mmol) in toluene (5 ml) and reaction mixture was heated at 60°C for 2 hr under stirring. The completion of reaction was monitored by TLC. To this reaction mixture was added cold water (10 ml) and extracted with ethyl acetate (2x 30 ml) and the combined organic layer was dried over anhydrous Na₂SO₄. The solvent was evaporated under reduced pressure to obtain a crude product; which was purified by column chromatography using 20% ethyl acetate in hexanes as an eluent to yield the title compound (0.32 gm, 60.37%).
MS: *m*/*z* 373 (M+39),
¹HNMR (CDCl₃, 400 MHz): δ 7.34-7.43 (m, 1H), 7.12-7.21 (m, 1H), 5.12-5.15(m, 1H), 4.12-4.34 (m, 4H), 2.04 (s, 3H), 1.84-1.91 (m, 4H), 1.35 (s, 6H), 1.21 (t, *J*=7.2 Hz, 3H).

### Step 6: 4-(5-(8-fluoro-4,4-dimethylchroman-6-yl)-2-methyl-3-propionyl-1H-pyrrol-1-yl)benzenesulfonamide

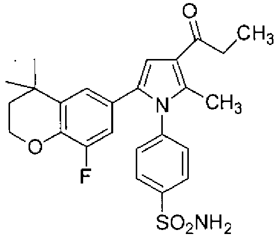

To the solution of 3-acetyl-1-(8-fluoro-4,4-dimethylchroman-6-yl)hexane-1,4-dione (step 2, 0.30 gm, 0.94 mmol) in acetic acid (10 ml) was added 4-aminobenzenesulfonamide (0.24 gm, 1.41 mmol) at room temperature. Reaction mixture was heated at 110° C for 24 hr. The completion of reaction was monitored by TLC. Solvent was evaporated at reduced pressure. Residue so obtained was taken in solution of ammonia in chloroform (10 ml) and stirred for 10 minutes. Reaction mixture was again concentrated at reduced pressure. Ethyl acetate (30 ml) was added to the residue, washed with water (5 ml). Combined organic layer was dried over anhydrous Na₂SO₄. The solvent was evaporated under reduced pressure to obtain a crude product; which was purified by column chromatography using 30% ethyl acetate in hexanes as an eluent to yield the title compound (0.54 gm, 12.27%).
MS: *m*/*z* 471 (M+1),
¹HNMR (CDCl₃, 400 MHz): δ 7.99 (d, *J*=8.8 Hz, 2H), 7.28 (d, *J*=8.8 Hz, 2H), 6.74 (dd, *J*=11.b, 2.0 Hz, 1H), 6.66 (s, 1H), 6.42 (t, *J*=2.0 Hz, 1H), 5.02 (bs-exchanges with D₂O, 2H), 4.19 (t, *J*=5.2 Hz, 2H), 2.86 (q, *J*=7.2 Hz, 2H), 2.43 (s, 3H), 1.75 (t, *J*=5.2 Hz, 2H), 1.21 (t, *J*=7.2 Hz, 3H), 1.02 (s, 6H).

### Example 7: Preparation of

### 4-(5-(2-acetyl-4,4-dimethyl-1,2,3,4-tetrahydroisoquinolin-7-yl)-2-methyl-3-propionyl-1H-pyrrol-1-yl)benzenesulfonamide.

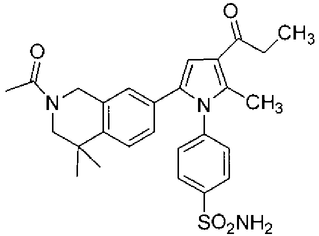

**And**

### 4-(5-(2-acetyl-4,4-dimethyl-1,2,3,4-tetrahydroisoquinolin-6-yl)-2-methyl-3-propionyl-1H-pyrrol-1-yl)benzenesulfonamide.

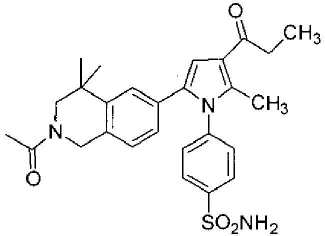

### Step 1: 1-(4,4-dimethyl-3,4-dihydroisoquinolin-2(1H)-yl)ethanone

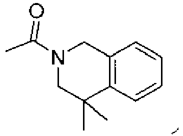

To a stirred solution of 4,4-dimethyl-1,2,3,4-tetrahydroisoquinoline (prepared according to the procedure reported in WO20050037214 A2, 4.0 gm , 24.84 mmol) in DCM (100 ml.) was added triethyl amine (2.76 gm, 3.9 ml, 27.32 mmol) in a dropwise manner at 0°C followed by addition of acetyl chloride (2.14 gm, 1.9 ml, 27.32 mmol). The resulting mixture was stirred at room temperature for 2 hr. The completion of reaction was monitored by TLC. Reaction mixture was diluted with DCM (100 ml), washed with water (2x 25 ml) followed by brine (25 ml). Combined organic layer was dried over anhydrous Na₂SO₄. The solvent was evaporated under reduced pressure to obtain a crude product; which was purified by column chromatography over silica gel (100-200 mesh) using 2.5% methanol in DCM as an eluent to yield the title compound (4.9 g, 97%)
MS: *m*/*z* 204 (M+1),
¹HNMR (DMSO, 400 MHz): δ 7.04-7.36 (m, 4H), 4.76 (s, 2H), 3.42 (s, 2H), 2.18 (s, 3H), 1.30 (s, 3H), 1.27 (s, 3H).

### Step 2: Mixture of 1-(2-acetyl-4,4-dimethyl-1,2,3,4-tetrahydroisoquinolin-7-yl)-2-bromoethanone

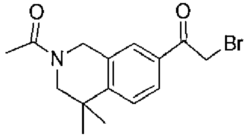

**And**

### 1-(2-acetyl-4,4-dimethyl-1,2,3,4-tetrahydroisoquinolin-6-yl)-2-bromoethanone

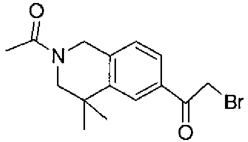

To a stirred solution of AlCl₃ (1.84 gm, 13.79 mmol) in DCE (30 ml) was added solution of 1-(4,4-dimethyl-3,4-dihydroisoquinolin-2(1H)-yl)ethanone (Step-1, 2.0 gm, 9.85 mmol) in DCE (10 ml) and 2-bromoacetyl bromide (2.60 gm, 1.13 ml, 12.80 mmol) in a drop wise manner at 0°C. The resulting mixture was stirred at room temperature for 2 hr. The completion of reaction was monitored by TLC. Reaction mixture was poured into cold water (50 ml). Aqueous layer was extracted with DCM (2 x 100 ml). Organic layers separated were dried over anhydrous sodium sulphate, filtered and concentrated at reduced pressure to get a crude product; which was purified by column chromatography over silica gel (100-200 mesh) using 2.5% methanol in DCM as an eluent to yield mixture of 1-(2-acetyl-4,4-dimethyl-1,2,3,4-tetrahydroisoquinolin-7-yl)-2- bromoethanone and 1-(2-acetyl-4,4-dimethyl-1,2,3,4-tetrahydroisoquinolin-6-yl)-2-bromoethanone (2.1 gm, 65.83%)

### Step 3: Mixture of 3-acetyl-1-(2-acetyl-4,4-dimethyl-1,2,3,4-tetrahydroisoquinolin-7-yl)hexane-1,4-dione

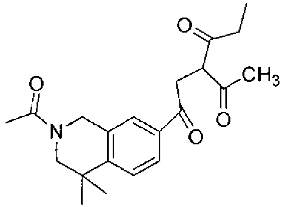

**And**

### 3-acetyl-1-(2-acetyl-4,4-dimethyl-1,2,3,4-tetrahydroisoquinolin-6-yl)hexane-1,4-dione

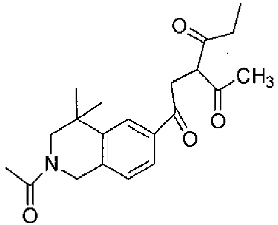

To the stirred solution of pulverized sodium (0.22 gm, 9.42 mmol) in toluene (40 ml) was added hexane-2,4-dione (prepared according to the procedure given in J. Amer. Chem. Soc., 1945, 67, 9, , 1510-1512, 0.98 gm, 8.56 mmol) at 0°C and reaction mixture was stirred at room temperature for 2 hr. To this was added solution of mixture of the 1-(2-acetyl-4,4-dimethyl-1,2,3,4-tetrahydroisoquinolin-7-yl)-2-bromoethanone and 1-(2-acetyl-4,4-dimethyl-1,2,3,4-tetrahydroisoquinolin-6-yl)-2-bromoethanone (step 2, 2.5 gm , 7.71 mmol) in toluene (10 ml) and reaction mixture was heated at 60°C for 2 hr under stirring. The completion of reaction was monitored by TLC. To this reaction mixture was added cold water (15 ml) and extracted with ethyl acetate (2x100 ml) and the combined organic layer was dried over anhydrous Na₂SO₄. The solvent was evaporated under reduced pressure to obtain a crude product; which was purified by column chromatography over silica gel (100-200 mesh) using 2.5% methanol in DCM as an eluent to yield mixture of 3-acetyl-1-(2-acetyl-4,4-dimethyl-1,2,3,4-tetrahydroisoquinolin-7-yl)hexane-1,4-dione and 3-acetyl-1-(2-acetyl-4,4-dimethyl-1,2,3,4-tetrahydroisoquinolin-6-yl)hexane-1,4-dione (1.45 gm, 47.5%).

### Step 4: 4-(5-(2-acetyl-4,4-dimethyl-1,2,3,4-tetrahydroisoquinolin-7-yl)-2-methyl-3-propionyl-1H-pyrrol-1-yl)benzenesulfonamide

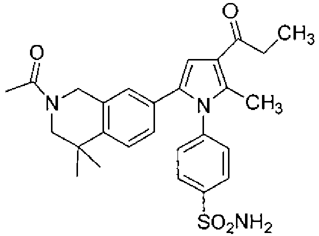

**And**

### 4-(5-(2-acetyl-4,4-dimethyl-1,2,3,4-tetrahydroisoquinolin-6-yl)-2-methyl-3-propionyl-1H-pyrrol-1-yl)benzenesulfonamide

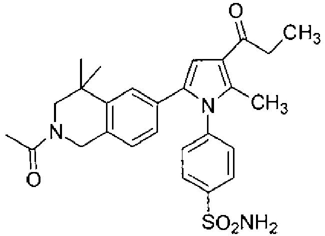

To the solution of the mixture of 3-acetyl-1-(2-acetyl-4,4-dimethyl-1,2,3,4-tetrahydroisoquinolin-7-yl)hexane-1,4-dione and 3-acetyl-1-(2-acetyl-4,4-dimethyl-1,2,3,4-tetrahydroisoquinolin-6-yl)hexane-1,4-dione (step 3, 1.4 gm, 3.92 mmol) in acetic acid (5 ml) was added 4-aminobenzenesulfonamide (0.68 gm, 3.92 mmol) at room temperature. Reaction mixture was heated at 110° C for 3 hr. The completion of reaction was monitored by TLC. Solvent was evaporated at reduced pressure. Residue so obtained was taken in solution of ammonia in chloroform (20 ml) and stirred for 10 minutes. Reaction mixture was again concentrated at reduced pressure. Ethyl acetate (100 ml) was added to the residue, washed with water (10 ml). Combined organic layer was dried over anhydrous Na₂SO₄. The solvent was evaporated under reduced pressure to obtain a crude product; which was purified by column chromatography over silica gel (100-200 mesh) using 50 % ethyl acetate in hexanes as an eluent to yield mixture of the 4-(5-(2-acetyl-4,4-dimethyl-1,2,3,4-tetrahydroisoquinolin-7-yl)-2-methyl-3-propionyl-1 H-pyrrol-1-yl)benzenesulfonamide and 4-(5-(2-acetyl-4,4-dimethyl-1,2,3,4-tetrahydroisoquinolin-6-yl)-2-methyl-3-propionyl 1H-pyrrol-1-yl)benzenesulfonamide. The mixture was separated by preparative HPLC to yield the first title compound (0.31 gm, 16.0%) and second title compound (0.21 gm, 10.89%).

### First title compound: 4-(5-(2-acetyl-4,4-dimethyl-1,2,3,4-tetrahydroisoquinolin-7-yl)-2-methyl-3-propionyl-1H-pyrrol-1-yl)benzenesulfonamide

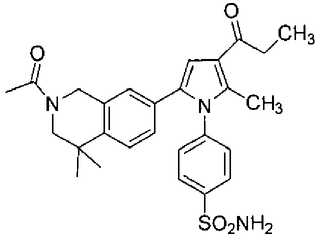

MS: *m*/*z* 494 (M+1),
¹HNMR (DMSO, 400 MHz): δ 7.89-7.92 (m, 2H), 7.54 (bs-exchanges with D₂O, 2H), 7.49 (d, *J*=8.4 Hz, 2H), 7.18 (d, *J*=8.0 Hz, 1H), 7.01 (s, 1H), 6.92 (d, *J*=2.4 Hz, 1H), 6.72-6.74 (m, 1H), 4.56 (s, 2H), 3.42 (s, 2H), 2.84 (q, *J*=7.2 Hz, 2H), 2.30 (s, 3H), 2.05 (s, 3H), 1.17 (s, 3H), 1.11 (s, 3H), 1.05 (t, *J*=7.2 Hz, 3H).

### Second title compound: 4-(5-(2-acetyl-4,4-dimethyl-1,2,3,4-tetrahydroisoquinolin-6-yl)-2-methyl-3-propionyl-1H-pyrrol-1-yl)benzenesulfonamide

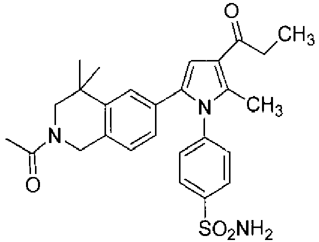

MS: m/z 494 (M+1),
¹HNMR (DMSO, 400 MHz): δ 7.89 (d, *J*=8.4 Hz, 2H), 7.51 (bs-exchanges with D₂O, 2H), 7.48 (d, *J*=8.4 Hz, 2H), 7.05-7.12 (m, 2H), 6.94 (s, 1H), 6.78-6.81 (m, 1H), 4.57 (s, 2H), 3.17 (s, 2H), 2.85 (q, *J*=7.2 Hz, 2H), 2.34 (s, 3H), 2.04 (s, 3H), 1.08 (t, *J*=7.2 Hz, 3H), 0.94 (s, 3H), 0.89 (s, 3H).

### Example 8: Preparation of 4-(5-(4,4-dimethyl-1,2,3,4-tetrahydroisoquinolin-7-yl)-2-methyl-3-propionyl-1H-pyrrol-1-yl)benzenesulfonamide.

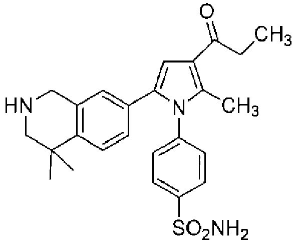

To the solution of the 4-(5-(2-acetyl-4,4-dimethyl-1,2,3,4-tetrahydroisoquinolin-7-yl)-2-methyl-3-propionyl-1H-pyrrol-1-yl)benzenesulfonamide (First title compound of step 4 in Example-7, 0.2 gm, 0.40 mmol) in acetonitrile (8 ml) was 6M HCl (10 ml) at room temperature. Reaction mixture was heated at 80° C for 15 hr. The completion of reaction was monitored by TLC. Solvent was evaporated at reduced pressure. Residue so obtained was taken in solution of ammonia in chloroform (20 ml) and stirred for 10 minutes. Reaction mixture was again concentrated at reduced pressure. Ethyl acetate (50 ml) was added to the residue, washed with water (10 ml). Combined organic layer was dried over anhydrous Na₂SO₄. The solvent was evaporated under reduced pressure to obtain a crude product; which was purified by preparative HPLC to yield the title compound (0.060 gm, 32.96%).
MS: *m*/*z* 452 (M+1),
¹HNMR (DMSO, 400 MHz): δ 8.23 (bs-exchanges with D₂O, 1H), 7.89 (d, *J*=8.4 Hz, 2H), 7.57 (bs-exchanges with D₂O, 2H), 7.19 (d, *J*=8.4 Hz, 2H), 6.88 (s, 1H), 6.85 (d, *J*=2.0 Hz, 1H), 6.76 (dd, *J*=8.4, 2.0 Hz, 2H), 3.81 (s, 2H), 2.84 (q, *J*=7.2 Hz, 2H), 2.78 (s, 2H), 2.30 (s, 3H), 1.17 (s, 6H), 1.05 (t, *J*=7.2 Hz, 3H).

### Example 9: Preparation of 4-(2-methyl-3-propionyl-5-(3H-spiro[benzo[b][1,4]dioxine-2,1'-cyclopropan]-7-yl)-1H-pyrrol-1-yl)benzenesulfonamide.

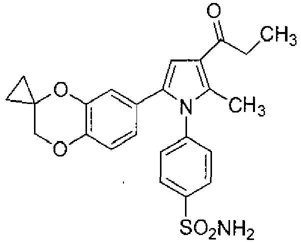

**And**

### 4-(2-methyl-3-propionyl-5-(3H-spiro[benzo[b][1,4]dioxine-2,1'-cyclopropan]-6-yl)-1H-pyrrol-1-yl)benzenesulfonamide.

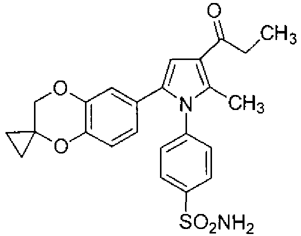

### Step 1: Methyl 4-bromo-2-(2-((4-methoxybenzyl)oxy)phenoxy)butanoate

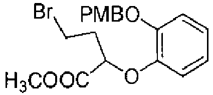

To a stirred solution of 2-((4-methoxybenzyl)oxy)phenol (prepared according to the procedure reported in JOC, 1994, 59, 22, 6567-6587, 10.0 gm , 43.48 mmol) in DMF (100 ml) were added K₂CO₃ (7.81 gm, 56.52 mmol) and methyl 2,4-dibromobutanoate (14.58 gm, ml, 56.52 mmol) at 25°C. The resulting mixture was stirred at 150°C for 3 hr. The completion of reaction was monitored by TLC. Reaction mixture was diluted with ethyl acetate (200 ml), washed with water (2x 50 ml) followed by brine (25 ml). Combined organic layer was dried over anhydrous Na₂SO₄. The solvent was evaporated under reduced pressure to obtain a crude product; which was purified by column chromatography over silica gel (100-200 mesh) using 10% ethyl acetate in hexanes as an eluent to yield the title compound (10.0 g, 56.24%)
MS: m/z 410 (M+1),
¹HNMR (CDCl₃, 400 MHz): δ 7.34 (d, *J*=8.4 Hz, 2H), 6.90-6.98 (m, 6H), 5.00-5.07 (m, 2H), 4.78 (dd, *J*=8.8. 4.0 Hz, 1H), 3.81 (s, 3H), 3.70 (s, 3H), 3.53-3.57 (m, 2H), 2.39-2.52 (m, 2H).

### Step 2: Methyl 1-(2-((4-methoxybenzyl)oxy)phenoxy)cyclopropanecarboxylate

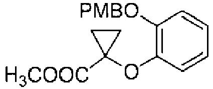

To a stirred solution of methyl 4-bromo-2-(2-((4-methoxybenzyl)oxy)phenoxy)butanoate (Step-1, 8.0 gm, 19.60 mmol) in THF (100 ml) was added potassium t-butoxide (2.41 gm, 21.56 mmol) at 0°C under nitrogen atmosphere. The resulting mixture was stirred at room temperature for 3 hr. The completion of reaction was monitored by TLC. Excess of reagent was quenched with saturated NH₄Cl solution (20 ml) at 0°C. Aqueous layer was extracted with ethyl acetate (2 x 150 ml). Organic layers separated were dried over anhydrous sodium sulphate, filtered and concentrated under reduced pressure to get a crude product; which was purified by column chromatography over silica gel (100-200 mesh) using 25% ethyl acetate in hexanes as an eluent to yield the title compound (2.5 g, 38.88%)
MS: *m*/*z* 351 (M+23),
¹HNMR (CDCl₃, 400 MHz): δ 7.34 (d, *J*=8.4 Hz, 2H), 6.84-6.97 (m, 6H), 5.06 (s, 2H), 3.77 (s, 3H), 3.69 (s, 3H), 1.59 (t, *J*=4.4 Hz, 2H), 1.25 (t, *J*=4.4 Hz, 2H).

### Step 3: (1-(2-((4-methoxybenzyl)oxy)phenoxy)cyclopropyl)methanol

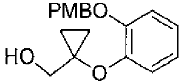

To a stirred solution of methyl 1-(2-((4-methoxybenzyl)oxy)phenoxy)cyclopropanecarboxylate (Step-2, 2.4 gm, 7.31 mmol) in THF (50 ml) was added LAH (0.41 gm, 10.97 mmol) at 0°C under nitrogen atmosphere. The resulting mixture was stirred at room temperature for 3 hr. The completion of reaction was monitored by TLC. Excess of reagent was quenched with saturated NH₄Cl solution (10 ml) at 0°C. Reaction mixture was filtered through bed of Na₂SO₄; washed with ethyl acetate (2 x 50 ml). Filtrate was dried over anhydrous sodium sulphate and concentrated under reduced pressure to get a crude product; which was purified by column chromatography over silica gel (100-200 mesh) using 35% ethyl acetate in hexanes as an eluent to yield the title compound (2.0 g, 91.3%)
MS: *m*/*z* 323 (M+23),
¹HNMR (CDCl₃, 400 MHz): δ 7.36 (d, *J*=8.4 Hz, 2H), 7.13 (d, *J*=7.6 Hz, 1H), 6.88-6.97 (m, 5H), 5.04 (s, 2H), 3.80 (s, 3H), 3.66 (d, *J*=6.0 Hz, 2H), 2.60 (t-exchanges with D₂O, *J*=6.0 Hz, 1H), 1.14 (t, *J*=6.4 Hz, 2H), 0.79 (t, *J*=6.4 Hz, 2H).

### Step 4: 2-(1-(hydroxymethyl)cyclopropoxy)phenol

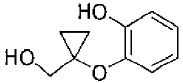

To a stirred solution of 10% palladium on carbon (1.5 gm) in methanol (25 ml), was added solution of (1-(2-((4-methoxybenzyl)oxy)phenoxy)cyclopropyl)methanol (Step-3, 1.5 gm, 5.00 mmol) in methanol (25 ml). To this mixture ammonium formate (12.60 gm, 200.00 mmol) was added at 25°C under nitrogen atmosphere. The resulting mixture was stirred at 60°C for 3 hr. The completion of reaction was monitored by TLC. Reaction mixture was cooled to room temperature and filtered through bed of celite; washed with DCM (2 x 50 ml). Filtrate was concentrated under reduced pressure to get a crude product; which was purified by again it dissolved in DCM (100 ml) and resulting solid was filtered. Filtrate was concentrated under reduced pressure to yield the title compound (0.85 g, 94.4%)
MS: *m*/*z* 203 (M+23),
¹HNMR (CDCl₃, 400 MHz): δ 7.02 (d, *J*=8.4 Hz, 1H), 6.89-6.93 (m, 2H), 6.76-6.81 (m, 1H), 3.78 (s, 2H), 2.30 (bs-exchanges with D₂O, 2H), 1.08 (t, *J*=6.4 Hz, 2H), 0.82 (t, *J*=6.4 Hz, 2H).

### Step 5: 3H-spiro[benzo[b][1,4]dioxine-2,1'-cyclopropane]

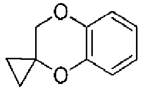

To a stirred solution of 2-(1-(hydroxymethyl)cyclopropoxy)phenol (Step-4, 1.2 gm , 6.66 mmol) in DCM (30 ml.) was added triphenyl phospine (1.92 gm, 7.32 mmol) at 0°C followed by addition of diethyl azodicarboxylate (1.39 gm, 1.26 ml, 7.99 mmol) under nitrogen atmosphere. The resulting mixture was stirred at room temperature for 2 hr. The completion of reaction was monitored by TLC. Reaction mixture was diluted with DCM (50 ml), washed with water (2x 20 ml) followed by brine (20 ml). Combined organic layer was dried over anhydrous Na₂SO₄. The solvent was evaporated under reduced pressure to obtain a crude product; which was purified by column chromatography over silica gel (100-200 mesh) using 10% ethyl acetate in hexanes as an eluent to yield the title compound (0.91 g, 84.2%)
MS: *m*/*z* No Ionization,
¹HNMR (CDCl₃, 400 MHz): δ 6.78-6.93 (m, 4H), 4.14 (s, 2H), 1.09 (t, *J*=6.4 Hz, 2H), 0.79 (t, *J*=6.4 Hz, 2H).

### Step 6: mixture of 2-bromo-1-(3H-spiro[benzo[b][1,4]dioxine-2,1'-cyclopropan]-7-yl)ethanone

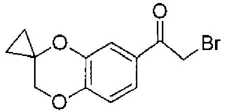

And

### 2-bromo-1-(3H-spiro[benzo[b][1,4]dioxine-2,1'-cyclopropan]-6-yl)ethanone

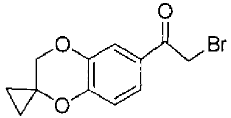

To a stirred solution of AlCl₃ (0.88 gm, 6.66 mmol) in CS₂ (5 ml) was added solution of 3H-spiro[benzo[b][1,4]dioxine-2,1'-cyclopropane] (Step-5, 0.9 gm, 5.55 mmol) in CS₂ (5 ml) and 2-bromoacetyl bromide (1.35 gm, 0.58 ml, 6.66 mmol) in a drop wise manner at 0°C. The resulting mixture was stirred at room temperature for 2 hr. The completion of reaction was monitored by TLC. Reaction mixture was quenched by addition of cold water (10 ml). Aqueous layer was extracted with DCM (2 x 50 ml). Organic layers separated were dried over anhydrous sodium sulphate, filtered and concentrated at reduced pressure to get a crude product; which was purified by column chromatography over silica gel (100-200 mesh) using 20% ethyl acetate in hexanes as an eluent to yield mixture of 2-bromo-1-(3H-spiro[benzo[b][1,4]dioxine-2,1'-cyclopropan]-7-yl)ethanone and 2-bromo-1-(3H-spiro[benzo[b][1,4]dioxine-2,1'-cyclopropan]-6-yl)ethanone (0.4 gm, 25.47%)

### Step 7: Mixture 3-acetyl-1-(3H-spiro[benzo[b][1,4]dioxine-2,1'-cyclopropan]-7-yl)hexane-1,4-dione

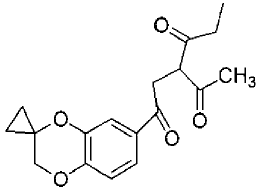

And

### 3-acetyl-1-(3H-spiro[benzo[b][1,4]dioxine-2,1'-cyclopropan]-6-yl)hexane-1,4-dione

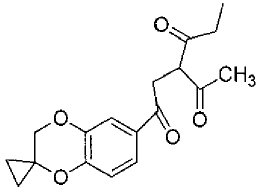

To the stirred solution of pulverized sodium (0.034 gm, 1.47 mmol) in toluene (5 ml) was added hexane-2,4-dione (prepared according to the procedure given in J. Amer. Chem. Soc., 1945, 67, 9, , 1510-1512, 0.15 gm, 1.36 mmol) at 0°C and reaction mixture was stirred at room temperature for 2 hr. To this was added solution of mixture of 2-bromo-1-(3H-spiro[benzo[b][1,4]dioxine-2,1'-cyclopropan]-7-yl)ethanone and 2-bromo-1-(3H-spiro[benzo[b][1,4]dioxine-2,1'-cyclopropan]-6-yl)ethanone (step 6, 0.35 gm, 1.23 mmol) in toluene (5 ml) and reaction mixture was heated at 60°C for 2 hr under stirring. The completion of reaction was monitored by TLC. To this reaction mixture was added cold water (5 ml) and extracted with ethyl acetate (2x 30 ml) and the combined organic layer was dried over anhydrous Na₂SO₄. The solvent was evaporated under reduced pressure to obtain a crude product; which was purified by column chromatography over silica gel (100-200 mesh) using 25% ethyl acetate in hexanes as an eluent to yield mixture of 3-acetyl-1-(3H-spiro[benzo[b][1,4]dioxine-2,1'-cyclopropan]-7-yl)hexane-1,4-dione and 3-acetyl-1-(3H-spiro[benzo[b][1,4]dioxine-2,1'-cyclopropan]-6-yl)hexane-1,4-dione (0.23 gm, 58.9%).

### Step 8: 4-(2-methyl-3-propionyl-5-(3H-spiro[benzo[b][1,4]dioxine-2,1'-cyclopropan]-7-yl)-1H-pyrrol-1-yl)benzenesulfonamide

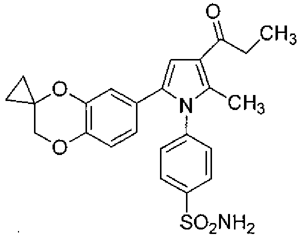

And

### 4-(2-methyl-3-propionyl-5-(3H-spiro[benzo[b][1,4]dioxine-2,1'-cyclopropan]-6-yl)-1 H-pyrrol-1-yl)benzenesulfonamide

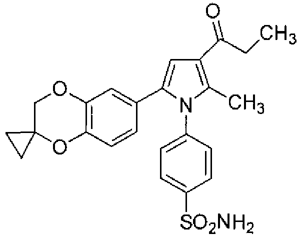

To the solution of the mixture of 3-acetyl-1-(3H-spiro[benzo[b][1,4]dioxine-2,1'-cyclopropan]-7-yl)hexane-1,4-dione and 3-acetyl-1-(3H-spiro[benzo[b][1,4]dioxine-2,1'-cyclopropan]-6-yl)hexane-1,4-dione (step 7, 0.2 gm, 0.63 mmol) in toluene:acetic acid (5:0.5 ml) was added 4-aminobenzenesulfonamide (0.13 gm, 0.75 mmol) at room temperature under nitrogen atmosphere. To this reaction mixture *p*-toluene sulphonic acid (0.015 gm, 0.09 mmol) was added and heated at 110° C for 3 hr. The completion of reaction was monitored by TLC. Solvent was evaporated at reduced pressure. Residue so obtained was taken in solution of ammonia in chloroform (10 ml) and stirred for 10 minutes. Reaction mixture was again concentrated at reduced pressure. Ethyl acetate (50 ml) was added to the residue, washed with water (10 ml). Combined organic layer was dried over anhydrous Na₂SO₄. The solvent was evaporated under reduced pressure to obtain a crude product; which was purified by column chromatography over silica gel (100-200 mesh) using 40 % ethyl acetate in hexanes as an eluent to yield mixture of the 4-(2-methyl-3-propionyl-5-(3H-spiro[benzo[b][1,4]dioxine-2,1'-cyclopropan]-7-yl)-1 H-pyrrol-1-yl)benzenesulfonamide and 4-(2-methyl-3-propionyl-5-(3H-spiro[benzo[b][1,4]dioxine-2,1'-cyclopropan]-6-yl)-1H-pyrrol-1-yl)benzenesulfonamide. The mixture was separated by preparative HPLC to yield the first title compound (0.035 gm, 12.2%) and second title compound (0.05 gm, 17.48%).

### First title compound: 4-(2-methyl-3-propionyl-5-(3H-spiro[benzo[b][1,4]dioxine-2,1'-cyclopropan]-7-yl)-1H-pyrrol-1-yl)benzenesulfonamide

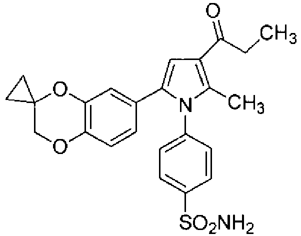

MS: m/z 453 (M+1),
¹HNMR (DMSO, 400 MHz): δ 7.88 (d, *J*=8.4 Hz, 2H), 7.52 (bs-exchanges with D₂O, 2H), 7.44 (d, *J*=8.4 Hz, 2H), 6.80 (s, 1H), 6.72 (d, *J*=8.4 Hz, 1H), 6.55 (d, *J*=2.0 Hz, 1H), 6.47 (dd, *J*=8.4, 2.0 Hz, 1H), 4.14 (s, 2H), 2.81 (q, *J*=7.2 Hz, 2H), 2.29 (s, 3H), 1.05 (t, *J*=7.2 Hz, 3H), 0.94 (t, *J*=5.6 Hz, 2H), 0.80 (t, *J*=5.6 Hz, 2H).

### Second title compound: 4-(2-methyl-3-propionyl-5-(3H-spiro[benzo[b][1,4]dioxine-2, 1'-cyclopropan]-6-yl)-1H-pyrrol-1-yl)benzenesulfonamide

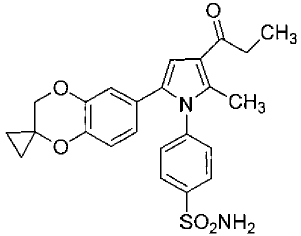

MS: m/z 453 (M+1),
¹HNMR (DMSO, 400 MHz): δ 7.89 (d, *J*=8.4 Hz, 2H), 7.53 (bs-exchanges with D₂O, 2H), 7.47 (d, *J*=8.4 Hz, 2H), 6.82 (s, 1H), 6.70 (d, *J*=2.0 Hz, 1H), 6.61 (d, *J*=8.4 Hz, 1H), 6.42 (dd, *J*=8.4, 2.0 Hz, 1H), 4.13 (s, 2H), 2.82 (q, *J*=7.2 Hz, 2H), 2.29 (s, 3H), 1.06 (t, *J*=7.2 Hz, 3H), 0.96 (t, *J*=5.6 Hz, 2H), 0.85 (t, *J*=5.6 Hz, 2H).

### Example 10: Preparation of 4-(5-(1-acetyl-4,4-dimethyl-1,2,3,4-tetrahydroquinolin-6-yl)-2-methyl-3-propionyl-1H-pyrrol-1-yl)benzenesulfonamide.

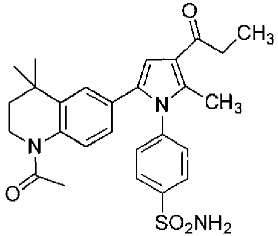

**And**

### 4-(5-(1-acetyl-4,4-dimethyl-1,2,3,4-tetrahydroquinolin-7-yl)-2-methyl-3-propionyl-1H-pyrrol-1-yl)benzenesulfonamide.

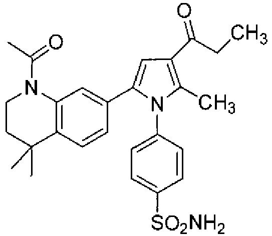

### Step 1: Mixture of 1-(1-acetyl-4,4-dimethyl-1,2,3,4-tetrahydroquinolin-6-yl)-2-bromoethanone

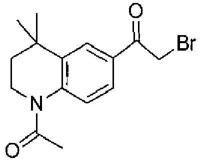

And

### 1-(1-acetyl-4,4-dimethyl-1,2,3,4-tetrahydroquinolin-7-yl)-2-bromoethanone

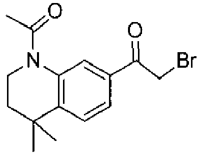

To a stirred solution of AlCl₃ (1.31 gm, 6.40 mmol) in DCE (30 ml) was added solution of 1-(4,4-dimethyl-3,4-dihydroquinolin-1(2H)-yl)ethanone (prepared according to the procedure reported in US 4808597, 1.2 gm, 5.91 mmol) in DCE (10 ml) and 2-bromoacetyl bromide (0.94 gm, 0.41 ml, 7.00 mmol) in a drop wise manner at 0°C. The resulting mixture was stirred at room temperature for 2 hr. The completion of reaction was monitored by TLC. Reaction mixture was poured into cold water (30 ml). Aqueous layer was extracted with DCM (2 x 50 ml). Organic layers separated were dried over anhydrous sodium sulphate, filtered and concentrated at reduced pressure to get a crude product; which was purified by column chromatography using 30% ethyl acetate in hexanes as an eluent to yield the mixture of 1-(1-acetyl-4,4-dimethyl-1,2,3,4-tetrahydroquinolin-6-yl)-2-bromoethanone and 1-(1-acetyl-4,4-dimethyl-1,2,3,4-tetrahydroquinolin-7-yl)-2-bromoethanone (0.80 gm, 42.10%).

### Step 2: Mixture of 3-acetyl-1-(1-acetyl-4,4-dimethyl-1,2,3,4-tetrahydroquinolin-6-yl)hexane-1,4-dione

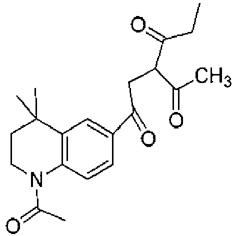

And

### 3-acetyl-1-(1-acetyl-4,4-dimethyl-1,2,3,4-tetrahydroquinolin-7-yl)hexane-1,4-dione

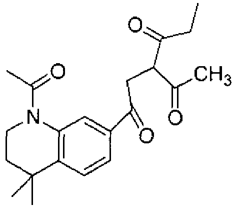

To the stirred solution of pulverized sodium (0.085 gm, 3.69 mmol) in toluene (10 ml) was added hexane-2,4-dione (prepared according to the procedure given in J. Amer. Chem. Soc., 1945, 67, 9, , 1510-1512, 0.30 gm, 2.71 mmol) at 0°C and reaction mixture was stirred at room temperature for 2 hr. To this was added solution of mixture of 1-(1-acetyl-4,4-dimethyl-1,2,3,4-tetrahydroquinolin-6-yl)-2-bromoethanone and 1-(1-acetyl-4,4-dimethyl-1,2,3,4-tetrahydroquinolin-7-yl)-2-bromoethanone (step 1, 0.80 gm, 2.47 mmol) in toluene (10 ml) and reaction mixture was heated at 60°C for 2 hr under stirring. The completion of reaction was monitored by TLC. To this reaction mixture was added cold water (10 ml) and extracted with ethyl acetate (2x 50 ml) and the combined organic layer was dried over anhydrous Na₂SO₄. The solvent was evaporated under reduced pressure to obtain a crude product; which was purified by column chromatography using 35% ethyl acetate in hexanes as an eluent to yield mixture of 3-acetyl-1-(1-acetyl-4,4-dimethyl-1,2,3,4-tetrahydroquinolin-6-yl)hexane-1,4-dione and 3-acetyl-1-(1-acetyl-4,4-dimethyl-1,2,3,4-tetrahydroquinolin-7-yl)hexane-1,4-dione (0.60 gm, 54.5%).

### Step 3: 4-(5-(1-acetyl-4,4-dimethyl-1,2,3,4-tetrahydroquinolin-6-yl)-2-methyl-3-propionyl-1H-pyrrol-1-yl)benzenesulfonamide

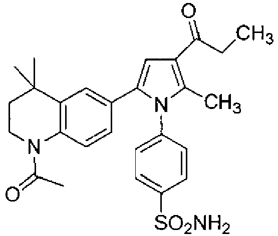

And

### 4-(5-(1-acetyl-4,4-dimethyl-1,2,3,4-tetrahydroquinolin-7-yl)-2-methyl-3-propionyl-1 H-pyrrol-1-yl)benzenesulfonamide

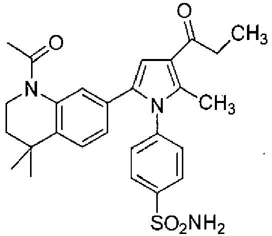

To the solution of the mixture of 3-acetyl-1-(1-acetyl-4,4-dimethyl-1,2,3,4-tetrahydroquinolin-6-yl)hexane-1,4-dione and 3-acetyl-1-(1-acetyl-4,4-dimethyl-1,2,3,4-tetrahydroquinolin-7-yl)hexane-1,4-dione (step 2, 0.25 gm, 0.70 mmol) in acetic acid (5 ml) was added 4-aminobenzenesulfonamide (0.24 gm, 1.40 mmol) at room temperature. Reaction mixture was heated at 110° C for 3 hr. The completion of reaction was monitored by TLC. Solvent was evaporated at reduced pressure. Residue so obtained was taken in solution of ammonia in chloroform (10 ml) and stirred for 10 minutes. Reaction mixture was again concentrated at reduced pressure. Ethyl acetate (50 ml) was added to the residue, washed with water (10 ml). Combined organic layer was dried over anhydrous Na₂SO₄. The solvent was evaporated under reduced pressure to obtain a crude product; which was purified by column chromatography over silica gel (100-200 mesh) using 50 % ethyl acetate in hexanes as an eluent to yield mixture of the 4-(5-(1-acetyl-4,4-dimethyl-1,2,3,4-tetrahydroquinolin-6-yl)-2-methyl-3-propionyl-1 H-pyrrol-1-yl)benzenesulfonamide and 4-(5-(1-acetyl-4,4-dimethyl-1,2,3,4-tetrahydroquinolin-7-yl)-2-methyl-3-propionyl-1H-pyrrol-1-yl)benzenesulfonamide. The mixture was separated by preparative HPLC to yield the first title compound (0.045 gm, 13.0%) and second title compound (0.030 gm, 8.69%).

### First title compound: 4-(5-(1-acetyl-4,4-dimethyl-1,2,3,4-tetrahydroquinolin-6-yl)-2-methyl-3-propionyl-1H-pyrrol-1-yl) benzenesulfonamide

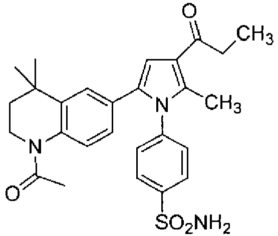

MS: *m*/*z* 494 (M+1),
¹HNMR (DMSO, 400 MHz): δ 7.90 (d, *J*=8.4 Hz, 2H), 7.47-7.53 (m, 5H), 7.05 (d, *J*=8.0 Hz, 1H), 6.94 (s, 1H), 6.75 (s, 1H), 3.62 (t, *J*=5.6 Hz, 2H), 2.85 (q, *J*=7.2 Hz, 2H), 2.34 (s, 3H), 2.08 (s, 3H), 1.60 (t, *J*=5.6 Hz, 2H), 1.08 (t, *J*=7.2 Hz, 3H), 0.92 (s, 6H).

### Second title compound: 4-(5-(1-acetyl-4,4-dimethyl-1,2,3,4-tetrahydroquinolin-7-yl)-2-methyl-3-propionyl-1H-pyrrol-1-yl)benzenesulfonamide

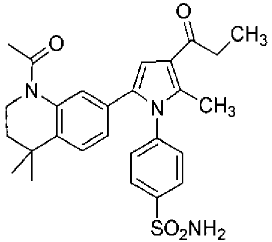

MS: *m*/*z* 494 (M+1),
¹HNMR (DMSO, 400 MHz): δ 7.89 (d, *J*=8.4 Hz, 2H), 7.48-7.54 (m, 4H), 7.29 (d, *J*=8.0 Hz, 1H), 6.86-6.94 (m, 3H), 3.63 (t, *J*=6.0 Hz, 2H), 2.85 (q, *J*=7.2 Hz, 2H), 2.50 (s, 3H), 2.30 (s, 3H), 1.65 (t, *J*=6.0 Hz, 2H), 1.18 (s, 6H), 1.06 (t, *J*=7.2 Hz, 3H).

### Example 11: Preparation of 4-(5-(4,4-dimethyl-1,2,3,4-tetrahydroquinolin-6-yl)-2-methyl-3-propionyl-1H-pyrrol-1-yl)benzenesulfonamide.

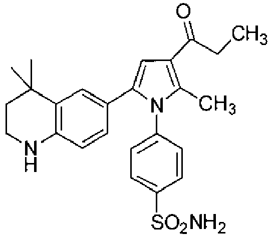

**And**

### 4-(5-(4,4-dimethyl-1,2,3,4-tetrahydroquinolin-7-yl)-2-methyl-3-propionyl-1H-pyrrol-1-yl)benzenesulfonamide.

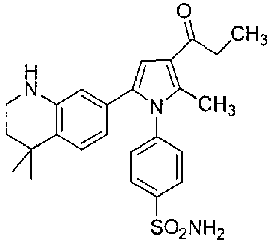

To the stirred solution of mixture of the 4-(5-(1-acetyl-4,4-dimethyl-1,2,3,4-tetrahydroquinolin-6-yl)-2-methyl-3-propionyl-1H-pyrrol-1-yl)benzenesulfonamide and 4-(5-(1-acetyl-4,4-dimethyl-1,2,3,4-tetrahydroquinolin-7-yl)-2-methyl-3-propionyl-1H-pyrrol-1-yl)benzenesulfonamide (step 3 in Example-8, 0.1 gm, 0.20 mmol) in acetonitrile (5 ml) was 6M HCl (10 ml) at room temperature. Reaction mixture was heated at 100° C for 4 hr. The completion of reaction was monitored by TLC. Solvent was evaporated at reduced pressure. Residue so obtained was taken in solution of ammonia in chloroform (20 ml) and stirred for 10 minutes. Reaction mixture was again concentrated at reduced pressure. Ethyl acetate (30 ml) was added to the residue, washed with water (10 ml). Combined organic layer was dried over anhydrous Na₂SO₄. The solvent was evaporated under reduced pressure to obtain a crude product; which was purified by column chromatography using 55% ethyl acetate in hexanes as an eluent to yield the first title compound (0.035 gm, 38.46%) and second title compound (0.025 gm, 27.47%).

### First title compound: 4-(5-(4,4-dimethyl-1,2,3,4-tetrahydroquinolin-6-yl)-2-methyl-3-propionyl-1H-pyrrol-1-yl)benzenesulfonamide

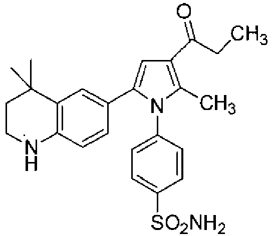

MS: m/z 452 (M+1),
¹HNMR (CDCl₃, 400 MHz): δ 7.96 (d, *J*=8.4 Hz, 2H), 7.28 (d, *J*=8.4 Hz, 2H), 6.79 (dd, *J*=8.0, 2.0 Hz, 1H), 6.60 (s, 1H), 6.58 (d, *J*=2.0 Hz, 1H), 6.31 (d, *J*=8.0 Hz, 1H), 4.87 (bs-exchanges with D₂O, 2H), 3.24 (t, *J*=5.6 Hz, 2H), 2.85 (q, *J*=7.2 Hz, 2H), 2.44 (s, 3H), 1.62 (t, *J*=5.6 Hz, 2H), 1.59 (bs-exchanges with D₂O, 1H), 1.21 (t, *J*=7.2 Hz, 3H), 0.98 (s, 6H).

### Second title compound: 4-(5-(4,4-dimethyl-1,2,3,4-tetrahydroquinolin-7-yl)-2-methyl-3-propionyl-1H-pyrrol-1-yl)benzenesulfonamide

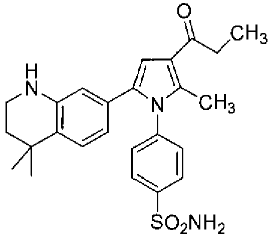

MS: *m*/*z* 452 (M+1),
¹HNMR (CDCl₃, 400 MHz): δ 7.95 (d, *J*=8.4 Hz, 2H), 7.29 (d, *J*=8.4 Hz, 2H), 6.93 (d, *J*=8.0 Hz, 1H), 6.65 (s, 1H), 6.19 (d, *J*= 2.0 Hz, 1H), 6.16 (dd, *J*=8.0, 2.0 Hz, 1H), 4.99 (bs-exchanges with D₂O, 2H), 3.25 (t, *J*=5.6 Hz, 2H), 2.84 (q, *J*=7.2 Hz, 2H), 2.40 (s, 3H), 1.68 (t, *J*=5.6 Hz, 2H), 1.66 (bs-exchanges with D₂O, 1H), 1.22 (s, 6H), 1.20 (t, *J*=7.2 Hz, 3H).

### Example 12: Preparation of 4-(5-(4,4-dimethyl-2-oxo-1,2,3,4-tetrahydroquinolin-6-yl)-2-methyl-3-propionyl-1H-pyrrol-1-yl)benzenesulfonamide.

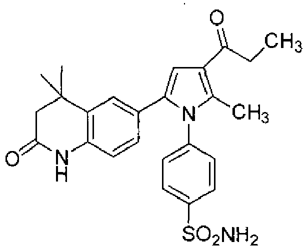

**And**

### 4-(5-(4,4-dimethyl-2-oxo-1,2,3,4-tetrahydroquinolin-7-yl)-2-methyl-3-propionyl-1H-pyrrol-1-yl)benzenesulfonamide.

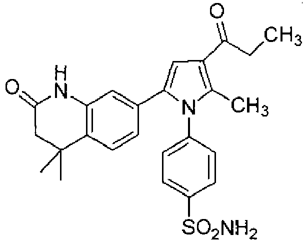

### Step 1: Mixture of 6-(2-bromoacetyl)-4,4-dimethyl-3,4-dihydroquinolin-2(1H)-one

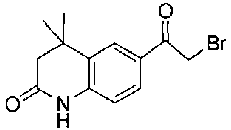

And

### 7-(2-bromoacetyl)-4,4-dimethyl-3,4-dihydroquinolin-2(1H)-one

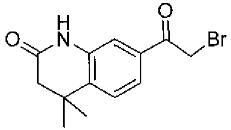

To a stirred solution of AlCl₃ (7.50 gm, 56.25 mmol) in CS₂ (30 ml) was added solution of 4,4-dimethyl-3,4-dihydroquinolin-2(1H)-one (prepared according to the procedure reported in US 4808597, 2.5 gm, 14.20 mmol) in CS₂ (20 ml) and 2-bromoacetyl bromide (4.32 gm, 1.88 ml, 21.70 mmol) in a drop wise manner at 0°C. The resulting mixture was stirred at reflux temperature for 3 hr. The completion of reaction was monitored by TLC. Reaction mixture was poured into cold 2N HCl (30 ml). Aqueous layer was extracted with ethyl acetate (2 x 100 ml). Organic layers separated were dried over anhydrous sodium sulphate, filtered and concentrated at reduced pressure to get a crude product; which was purified by column chromatography using 35% ethyl acetate in hexanes as an eluent to yield the mixture of 6-(2-bromoacetyl)-4,4-dimethyl-3,4-dihydroquinolin-2(1H)-one and 7-(2-bromoacetyl)-4,4-dimethyl-3,4-dihydroquinolin-2(1H)-one (2.00 gm, 47.4%).

### Step 2: Mixture of 3-acetyl-1-(4,4-dimethyl-2-oxo-1,2,3,4-tetrahydroquinolin-6-yl)hexane-1,4-dione

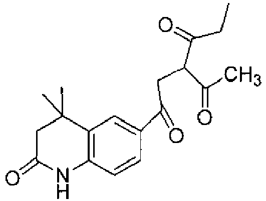

And

### 3-acetyl-1-(4,4-dimethyl-2-oxo-1,2,3,4-tetrahydroquinolin-7-yl)hexane-1,4-dione

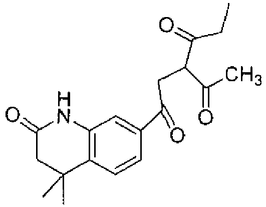

To the stirred solution of pulverized sodium (0.184 gm, 8.00 mmol) in toluene (15 ml) was added hexane-2,4-dione (prepared according to the procedure given in J. Amer. Chem. Soc., 1945, 67, 9, , 1510-1512, 0.77 gm, 6.70 mmol) at 0°C and reaction mixture was stirred at room temperature for 2 hr. To this was added solution of mixture of 6-(2-bromoacetyl)-4,4-dimethyl-3,4-dihydroquinolin-2(1H)-one and 7-(2-bromoacetyl)-4,4-dimethyl-3,4-dihydroquinolin-2(1H)-one (step 1, 2.00 gm, 6.70 mmol) in toluene (15 ml) and reaction mixture was heated at 60°C for 2 hr under stirring. The completion of reaction was monitored by TLC. To this reaction mixture was added cold water (20 ml) and extracted with ethyl acetate (2x 100 ml) and the combined organic layer was dried over anhydrous Na₂SO₄. The solvent was evaporated under reduced pressure to obtain a crude product; which was purified by column chromatography using 35% ethyl acetate in hexanes as an eluent to yield mixture of 3-acetyl-1-(4,4-dimethyl-2-oxo-1,2,3,4-tetrahydroquinolin-6-yl)hexane-1,4-dione and 3-acetyl-1-(4,4-dimethyl-2-oxo-1,2,3,4-tetrahydroquinolin-7-yl)hexane-1,4-dione (1.30 gm, 58.55%).

### Step 3: 4-(5-(4,4-dimethyl-2-oxo-1,2,3,4-tetrahydroquinolin-6-yl)-2-methyl-3-propionyl-1H-pyrrol-1-yl)benzenesulfonamide

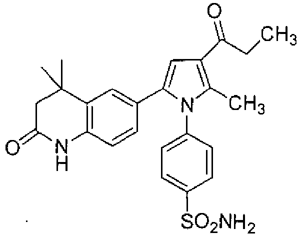

And

### 4-(5-(4,4-dimethyl-2-oxo-1,2,3,4-tetrahydroquinolin-7-yl)-2-methyl-3-propionyl-1H-pyrrol-1-yl)benzenesulfonamide

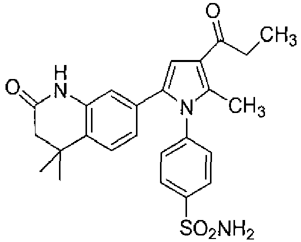

To the solution of the mixture of 3-acetyl-1-(4,4-dimethyl-2-oxo-1,2,3,4-tetrahydroquinolin-6-yl)hexane-1,4-dione and 3-acetyl-1-(4,4-dimethyl-2-oxo-1,2,3,4-tetrahydroquinolin-7-yl)hexane-1,4-dione (step 2, 1.30 gm, 3.95 mmol) in acetic acid (20 ml) was added 4-aminobenzenesulfonamide (1.35 gm, 7.90 mmol) at room temperature. Reaction mixture was heated at 110° C for 3 hr. The completion of reaction was monitored by TLC. Solvent was evaporated at reduced pressure. Residue so obtained was taken in solution of ammonia in chloroform (30 ml) and stirred for 10 minutes. Reaction mixture was again concentrated at reduced pressure. Ethyl acetate (100 ml) was added to the residue, washed with water (30 ml). Combined organic layer was dried over anhydrous Na₂SO₄. The solvent was evaporated under reduced pressure to obtain a crude product; which was purified by column chromatography over silica gel (100-200 mesh) using 50 % ethyl acetate in hexanes as an eluent to yield mixture of the 4-(5-(4,4-dimethyl-2-oxo-1,2,3,4-tetrahydroquinolin-6-yl)-2-methyl-3-propionyl-1H-pyrrol-1-yl)benzenesulfonamide and 4-(5-(4,4-dimethyl-2-oxo-1,2,3,4-tetrahydroquinolin-7-yl)-2-methyl-3-propionyl-1H-pyrrol-1-yl)benzenesulfonamide (0.6 gm, 32.78%). 0.150 gm of the mixture was separated by preparative HPLC to yield the first title compound (0.035 gm, 23.33%) and second title compound (0.025 gm, 16.66%).

### First title compound: 4-(5-(4,4-dimethyl-2-oxo-1,2,3,4-tetrahydroquinolin-6-yl)-2-methyl-3-propionyl-1 H-pyrrol-1-yl)benzenesulfonamide

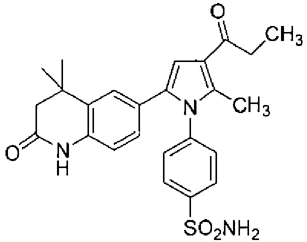

MS: m/z 466 (M+1),
¹HNMR (DMSO, 400 MHz): δ 10.46 (bs-exchanges with D₂O, 1H), 7.99 (d, *J*=8.4 Hz, 2H), 7.56-7.72 (m, 6H), 6.97 (d, *J*=8.4 Hz, 1H), 6.21 (s, 1H), 2.44 (s, 2H), 2.30 (q, *J*=7.2 Hz, 2H), 2.21 (s, 3H), 1.27 (s, 6H), 1.01 (t, *J*=7.2 Hz, 3H).

### Second title compound: 4-(5-(4,4-dimethyl-2-oxo-1,2,3,4-tetrahydroquinolin-7-yl)-2-methyl-3-propionyl-1H-pyrrol-1-yl)benzenesulfonamide

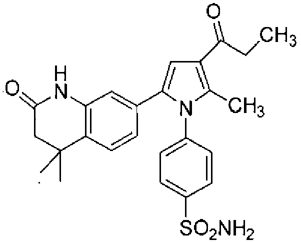

MS: *m*/*z* 466 (M+1),
¹HNMR (DMSO, 400 MHz): δ 10.13 (bs-exchanges with D₂O, 1H), 7.89 (d, *J*=8.4 Hz, 2H), 7.46-7.51 (m, 4H), 7.03 (dd, *J*=8.0, 2.0 Hz, 1H), 6.87 (s, 1H), 6.71-6.74 (m, 2H), 2.83 (q, *J*=7.2 Hz, 2H), 2.33 (s, 3H), 2.21 (s, 2H), 1.07 (t, *J*=7.2 Hz, 3H), 0.94 (s, 6H).

### Example 13: Preparation of 4-(2-methyl-3-propionyl-5-(1,4,4-trimethyl-2-oxo-1,2,3,4-tetrahydroquinolin-6-yl)-1H-pyrrol-1-yl)benzenesulfonamide.

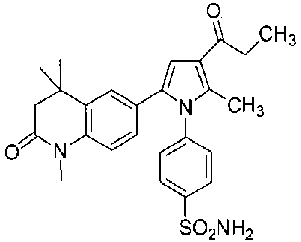

### Step 1: 6-(2-bromoacetyl)-1,4,4-trimethyl-3,4-dihydroquinolin-2(1H)-one

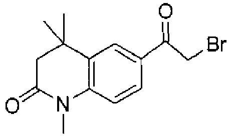

To a stirred solution of AlCl₃ (2.36 gm, 17.7 mmol) in CS₂ (30 ml) was added solution of 1,4,4-trimethyl-3,4-dihydroquinolin-2(1H)-one, (prepared according to the procedure reported in European Journal of Medicinal Chemistry, 2008, 43, 8, 1730 - 1736, 2.8 gm, 14.80 mmol) in CS₂ (20 ml) and 2-bromoacetyl bromide (3.26 gm, 1.42 ml, 16.20 mmol) in a drop wise manner at 0°C. The resulting mixture was stirred at reflux temperature for 4 hr. The completion of reaction was monitored by TLC. Reaction mixture was poured into cold water (50 ml). Aqueous layer was extracted with ethyl acetate (2 x 100 ml). Organic layers separated were dried over anhydrous sodium sulphate, filtered and concentrated at reduced pressure to get a crude product; which was purified by column chromatography using 45% ethyl acetate in hexanes as an eluent to yield the title compound (2.00 gm, 43.57%).
MS: **m/z** 311 (M+1),

### Step 2: 3-acetyl-1-(1,4,4-trimethyl-2-oxo-1,2,3,4-tetrahydroquinolin-6-yl)hexane-1,4-dione

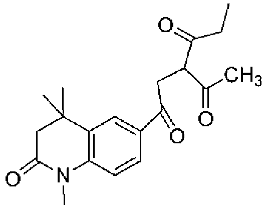

To the stirred solution of pulverized sodium (0.220 gm, 9.56 mmol) in toluene (15 ml) was added hexane-2,4-dione (prepared according to the procedure given in J. Amer. Chem. Soc., 1945, 67, 9, , 1510-1512, 0.87 gm, 7.60 mmol) at 0°C and reaction mixture was stirred at room temperature for 2 hr. To this was added solution of 6-(2-bromoacetyl)-1,4,4-trimethyl-3,4-dihydroquinolin-2(1H)-one (step 1, 2.00 gm, 6.40 mmol) in toluene (15 ml) and reaction mixture was heated at 60°C for 2 hr under stirring. The completion of reaction was monitored by TLC. To this reaction mixture was added cold water (20 ml) and extracted with ethyl acetate (2x 100 ml) and the combined organic layer was dried over anhydrous Na₂SO₄. The solvent was evaporated under reduced pressure to obtain a crude product; which was purified by column chromatography using 35% ethyl acetate in hexanes as an eluent to yield title compound (0.72 gm, 32.57%).
MS: *m*/*z* 344 (M+1),

### Step 3: 4-(2-methyl-3-propionyl-5-(1,4,4-trimethyl-2-oxo-1,2,3,4-tetrahydroquinolin-6-yl)-1H-pyrrol-1-yl)benzenesulfonamide

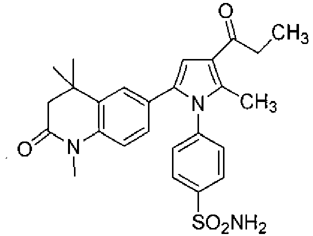

To the solution of the 3-acetyl-1-(1,4,4-trimethyl-2-oxo-1,2,3,4-tetrahydroquinolin-6-yl)hexane-1,4-dione (step 2, 0.70 gm, 2.04 mmol) in acetic acid (15 ml) was added 4-aminobenzenesulfonamide (0.70 gm, 4.08 mmol) at room temperature. Reaction mixture was heated at 110° C for 3 hr. The completion of reaction was monitored by TLC. Solvent was evaporated at reduced pressure. Residue so obtained was taken in solution of ammonia in chloroform (30 ml) and stirred for 10 minutes. Reaction mixture was again concentrated at reduced pressure. Ethyl acetate (100 ml) was added to the residue, washed with water (30 ml). Combined organic layer was dried over anhydrous Na₂SO₄. The solvent was evaporated under reduced pressure to obtain a crude product; which was purified by preparative HPLC to yield the title compound (0.110 gm, 11.2%).
MS: *m*/*z* 480 (M+1),
¹HNMR (CDCl₃, 400 MHz): δ 7.99 (d, *J*=8.4 Hz, 2H), 7.32 (d, *J*=8.4 Hz, 2H), 7.03 (dd, *J*=8.4, 2.0 Hz, 1H), 6.84 (d, *J*=8.4 Hz, 1H), 6.77 (d, *J*=2.0 Hz, 1H), 6.73 (s, 1H), 4.98 (bs-exchanges with D₂O, 2H), 3.33 (s, 3H), 2.89 (q, *J*=7.2 Hz, 2H), 2.46 (s, 3H), 2.41 (s, 2H), 1.22 (t, *J*=7.2 Hz, 3H), 1.07 (s, 6H).

### Example 14: Pharmacological screening

Compounds were tested in a cell-based real-time kinetic assay in human IMR-32 cells with native expression of α7nAChR. The increase in intracellular Ca²⁺ levels was measured in a Fluorometric Imaging Plate Reader (FLIPR). Test compound and agonist solutions were made in assay buffer (HBSS, pH 7.4, 20 mM HEPES, and 10 mM CaCl₂). Briefly, cells were plated into Poly-D-Lysine coated back-walled clear-bottom 96-well microplates at a density of 80,000 to 100,000 cells/well and incubated at 37°C/5% CO₂ for 40-48 h prior to the experiment. For evaluation of compound mediated potentiation of agonist response, growth media was removed from the wells and 200 µl of FLIPR calcium 4 dye (Molecular Devices), reconstituted in assay buffer, and was added to the wells. After dye loading, microplates were incubated for 30 min at 37°C and 30 min at room temperature and then directly transferred to the FLIPR. Baseline fluorescence was monitored for the first 10 to 30 s followed by the addition of 25 µl of test compound solution and subsequent monitoring of fluorescence changes for up to 10 min. This was followed by addition of 25 µl of agonist (PNU-282987, 10 µM) solution and measurement of fluorescence for 4 min. (Faghih R. et al. 2009, J. Med. Chem. 52, 3377 - 84.)

The compound induced fold increase in agonist response (fold PAM activity) was computed by dividing the maximum effect (Max-Min fluorescence) obtained with test compound in presence of agonist with the agonist-alone effect. EC₅₀ of the compound was calculated using GraphPad Prism software version 5.0, by plotting compound concentrations against fold PAM activity.

The compounds of the present invention showed 2 to 30 fold activation at 1 µM concentration.

## Claims

1. A compound of the general formula I, its tautomeric forms, its stereoisomers and its pharmaceutically acceptable salts; 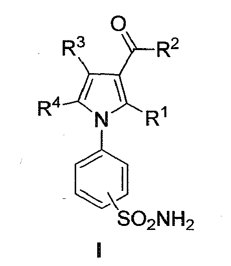 wherein,
R¹ is selected from hydrogen, halogen, optionally substituted alkyl, perhaloalkyl, optionally substituted cycloalkyl, optionally substituted aryl; optionally substituted heterocyclyl, optionally substituted heteroaryl;
R² is selected from optionally substituted alkyl, optionally substituted heteroalkyl, optionally substituted aryl, optionally substituted heteroaryl, optionally substituted cycloalkyl, optionally substituted heterocyclyl, or -NR⁵(R⁶), -A¹R⁵, -N(R⁵)OR⁶;
R³ is selected from hydrogen, optionally substituted alkyl, halo, optionally substituted cycloalkyl, optionally substituted aryl, optionally substituted heterocyclyl, optionally substituted heteroaryl, cyano, nitro or -NR⁵(R⁶), -OR⁵;
R⁴ is 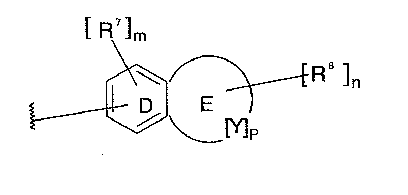
wherein, phenyl ring 'D' is fused with ring 'E', which is a non-aromatic five to eight member ring inclusive of 'Y' group(s);
Y is independently selected at each repetition from -O-, -S-, -NH-, 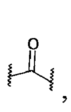 or 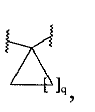 where q = 1 - 4; wherein when Y is selected as -NH- or 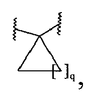 it is optionally substituted by [R⁸]ₙ;
wherein, R⁵ and R⁶ are independently selected from hydrogen, optionally substituted alkyl, optionally substituted heteroalkyl, optionally substituted aryl, optionally substituted heteroaryl, optionally substituted cycloalkyl, optionally substituted heterocyclyl, R^{9a}C(=A¹)-;
R⁷ is selected independently at each occurrence from the group consisting of halogen, optionally substituted alkyl, optionally substituted cycloalkyl;
R⁸ is independently selected at each occurrence from the group consisting of optionally substituted alkyl, R⁹A¹-, R^{9a}C(=A¹)-;
m = 0 to 2;
n = 0 to 3;
p = 0 to 4;
such that, when p = 0 then n ≠ 0;
wherein, R⁹ wherever it appears, is selected from hydrogen, optionally substituted C₁₋₆ alkyl, optionally substituted heteroalkyl, optionally substituted aryl, optionally substituted heteroaryl, optionally substituted cycloalkyl, and optionally substituted heterocyclyl; and A¹ is selected from O and S;
R^{9a} wherever it appears, is selected from optionally substituted C₁₋₆ alkyl, optionally substituted heteroalkyl, optionally substituted aryl, optionally substituted heteroaryl, optionally substituted cycloalkyl, and optionally substituted heterocyclyl;
wherein,
"optionally substituted alkyl", means a alkyl group optionally substituted with 1 to 6 substituents selected independently from the group consisting of oxo, halogen, nitro, cyano, aryl, hereroaryl, cycloalkyl, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-;
"optionally substituted heteroalkyl" means a heteroalkyl group optionally substituted with 1 to 6 substituents selected independently from the group consisting of oxo, halogen, nitro, cyano, aryl, hereroaryl, cycloalkyl.
"optionally substituted cycloalkyl" means a cycloalkyl group optionally substituted with 1 to 6 substituents selected independently from the group consisting of oxo, halogen, nitro, cyano, aryl, hereroaryl, alkyl, R^{10a}C(=O)-, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-;
"optionally substituted aryl" means (i) an aryl group optionally substituted with 1 to 3 substituents selected independently from the group consisting of halogen, nitro, cyano, hydroxy, C₁ to C₆ alkyl, C₃ to C₆ cycloalkyl, C₁ to C₆ perhaloalkyl, alkyl-O-, perhaloalkyl-O-, alkyl-N(alkyl)-, alkyl-N(H)-, H₂N-, alkyl-SO₂-, perhaloalkyl-SO₂-, alkyl-C(=O)N(alkyl)-, alkyl-C(=O)N(H)-, alkyl-N(alkyl)C(=O)-, alkyl-N(H)C(=O)-, H₂NC(=O)-, alkyl-N(alkyl)SO₂-, alkyl-N(H)SO₂-, H₂NSO₂-, 3 to 6 membered heterocycle containing 1 to 2 heteroatoms selected from N, O and S optionally substituted with alkyl or alkyl-C(=O)-, (ii) an aryl ring optionally fused with cycloalkane or heterocycle across a bond optionally substituted with oxo, alkyl or alkyl-C(=O)-;
"optionally substituted heterocyclyl" means a (i) heterocyclyl group optionally substituted on ring carbons with 1 to 6 substituents selected independently from the group consisting of oxo, halogen, nitro, cyano, aryl, hereroaryl, alkyl, R¹⁰A¹-, R^{10a}OC(=O)-, R^{10a}C(=O)O-, (R¹⁰)(H)NC(=O)-, (R¹⁰)(alkyl)NC(O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-; (ii) heterocyclyl group optionally substituted on ring nitrogen(s) with substituents selected from the group consisting of aryl, hereroaryl, alkyl, R^{10a}C(=O)-, R^{10a}SO₂-, R^{10a}OC(=O)-, (R¹⁰)(H)NC(=O)-, (R¹⁰)(alkyl)NC(=O)-;
"optionally substituted heteroaryl" means a heteroaryl group optionally substituted with 1 to 3 substituents selected independently from the group consisting of halogen, nitro, cyano, hydroxy, C₁ to C₆ alkyl, C₃ to C₆ cycloalkyl, C₁ to C₆ perhaloalkyl, alkyl-O-, perhaloalkyl-O-, alkyl-N(alkyl)-, alkyl-N(H)-, H₂N-, alkyl-SO₂-, perhaloalkyl-SO₂-, alkyl-C(=O)N(alkyl)-, alkyl-C(=O)N(H)-, alkyl-N(alkyl)C(=O)-, alkyl-N(H)C(=O)-, H₂NC(=O)-, alkyl-N(alkyl)SO₂-, alkyl-N(H)SO₂-, H₂NSO₂-, 3 to 6 membered heterocycle containing 1 to 2 heteroatoms selected from N, O and S optionally substituted with alkyl or alkyl-C(=O)-;
wherein R¹⁰ is selected from hydrogen, alkyl, aryl, heteroaryl, cycloalkyl or heterocyclyl; and A¹ is selected from S and O; and R^{10a} is selected from alkyl, perhaloalkyl, aryl, heteroaryl, cycloalkyl or heterocyclyl.

2. The compound of formula I as claimed in claim 1, wherein R¹ is selected as methyl.

3. The compound of formula I as claimed in claim 1, wherein R² is selected from ethyl and ethoxy.

4. The compound of formula I as claimed in claim 1, wherein R³ is selected from hydrogen and methyl.

5. The compound of formula I as claimed in claim 1, wherein R⁴ is selected from

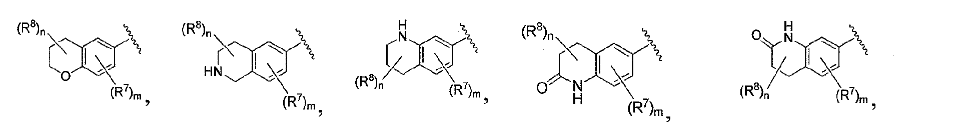

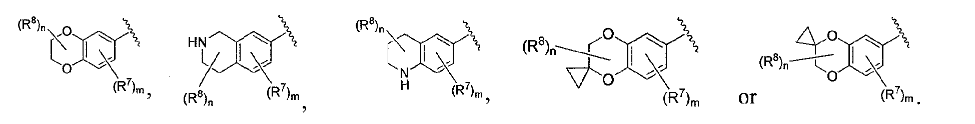

6. The compound of formula I as claimed in claim 1, wherein R¹ is selected from methyl, R² is selected from ethyl and ethoxy, R³ is selected from hydrogen and methyl, and R⁴ is selected from

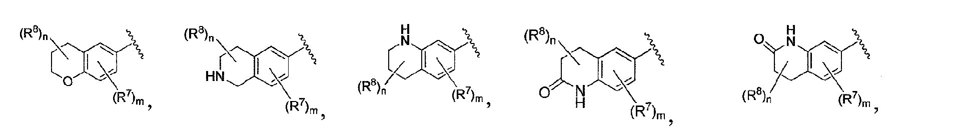

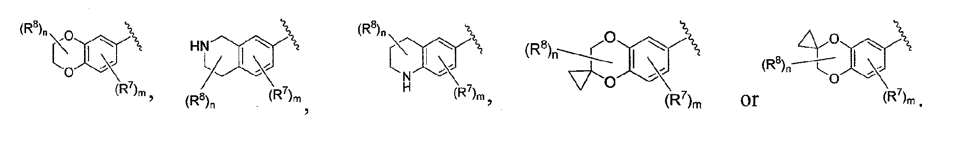

7. The compound of formula I as claimed in claim 1, wherein the compound is selected from-
4-(5-(4,4-dimethylchroman-6-yl)-2-methyl-3-propionyl-1H-pyrrol-1-yl)benzenesulfonamide;
4-(5-(2,3-dihydrobenzo[b][1,4]dioxin-6-yl)-2-methyl-3-propionyl-1H-pyrrol-1-yl)benzenesulfonamide;
4-(2-(2,3-dihydrobenzo[b][1,4]dioxin-6-yl)-3,5-dimethyl-4-propionyl-1H-pyrrol-1-yl)benzenesulfonamide;
Ethyl 5-(2,3-dihydrobenzo[b][1,4]dioxin-6-yl)-2,4-dimethyl-1-(4-sulfamoylphenyl)-1H-pyrrole-3-carboxylate;
4-(5-(2,2-dimethylchroman-6-yl)-2-methyl-3-propionyl-1H-pyrrol-1-yl)benzenesulfonamide;
4-(5-(8-fluoro-4,4-dimethylchroman-6-yl)-2-methyl-3-propionyl-1H-pyrrol-1-yl)benzenesulfonamide;
4-(5-(2-acetyl-4,4-dimethyl-1,2,3,4-tetrahydroisoquinolin-7-yl)-2-methyl-3-propionyl-1H-pyrrol-1-yl)benzenesulfonamide;
4-(5-(2-acetyl-4,4-dimethyl-1,2,3,4-tetrahydroisoquinolin-6-yl)-2-methyl-3-propionyl-1H-pyrrol-1-yl)benzenesulfonamide;
4-(5-(4,4-dimethyl-1,2,3,4-tetrahydroisoquinolin-7-yl)-2-methyl-3-propionyl-1H-pyrrol-1-yl)benzenesulfonamide;
4-(2-methyl-3-propionyl-5-(3H-spiro[benzo [b] [1,4]dioxine-2,1'-cyclopropan]-7-yl)-1H-pyrrol-1-yl)benzenesulfonamide;
4-(2-methyl-3-propionyl-5-(3H-spiro [benzo [b][1,4]dioxine-2,1'-cyclopropan]-6-yl)-1H-pyrrol-1-yl)benzenesulfonamide;
4-(5-(1-acetyl-4,4-dimethyl-1,2,3,4-tetrahydroquinolin-6-yl)-2-methyl-3-propionyl-1H-pyrrol-1-yl)benzenesulfonamide;
4-(5-(1-acetyl-4,4-dimethyl-1,2,3,4-tetrahydroquinolin-7-yl)-2-methyl-3-propionyl-1H-pyrrol-1-yl)benzenesulfonamide;
4-(5-(4,4-dimethyl-1,2,3,4-tetrahydroquinolin-6-yl)-2-methyl-3-propionyl-1H-pyrrol-1-yl)benzenesulfonamide;
4-(5-(4,4-dimethyl-1,2,3,4-tetrahydroquinolin-7-yl)-2-methyl-3-propionyl-1H-pyrrol-1-yl)benzenesulfonamide;
4-(5-(4,4-dimethyl-2-oxo-1,2,3,4-tetrahydroquinolin-6-yl)-2-methyl-3-propionyl-1H-pyrrol-1-yl)benzenesulfonamide;
4-(5-(4,4-dimethyl-2-oxo-1,2,3,4-tetrahydroquinolin-7-yl)-2-methyl-3-propionyl-1H-pyrrol-1-yl)benzenesulfonamide;
4-(2-methyl-3-propionyl-5-(1,4,4-trimethyl-2-oxo-1,2,3,4-tetrahydroquinolin-6-yl)-1H-pyrrol-1-yl)benzenesulfonamide.

8. A pharmaceutical composition comprising a compound of claim 1 and a pharmaceutically acceptable carrier.

9. A compound of claim 1 for use in preventing or treating a disease or its symptoms or a disorder mediated partially or completely by nicotinic acetylcholine receptors.

10. A compound of formula I for use in treating a disease or disorder or condition, 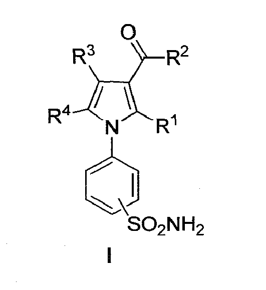 wherein,
R¹ is selected from hydrogen, halogen, optionally substituted alkyl, perhaloalkyl, optionally substituted cycloalkyl, optionally substituted aryl; optionally substituted heterocyclyl, optionally substituted heteroaryl;
R² is selected from optionally substituted alkyl, optionally substituted heteroalkyl, optionally substituted aryl, optionally substituted heteroaryl, optionally substituted cycloalkyl, optionally substituted heterocyclyl, or -NR⁵(R⁶), -A¹R⁵, -N(R⁵)OR⁶;
R³ is selected from hydrogen, optionally substituted alkyl, halo, optionally substituted cycloalkyl, optionally substituted aryl, optionally substituted heterocyclyl, optionally substituted heteroaryl, cyano, nitro or -NR⁵(R⁶), -OR⁵;
R⁴ is 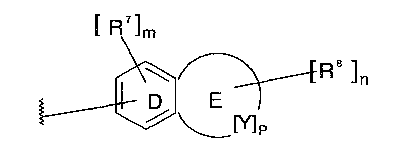
wherein, phenyl ring 'D' is fused with ring `E', which is a non-aromatic five to eight member ring inclusive of `Y' group(s);
Y is independently selected at each repetition from -O-, -S-, -NH-, 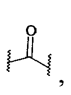 or 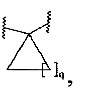 where q = 1 - 4; wherein when Y is selected as -NH- or 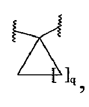 it is optionally substituted by [R⁸]ₙ;
wherein, R⁵ and R⁶ are independently selected from hydrogen, optionally substituted alkyl, optionally substituted heteroalkyl, optionally substituted aryl, optionally substituted heteroaryl, optionally substituted cycloalkyl, optionally substituted heterocyclyl, R^{9a}C(=A¹)-;
R⁷ is selected independently at each occurrence from the group consisting of halogen, optionally substituted alkyl, optionally substituted cycloalkyl;
R⁸ is independently selected at each occurrence from the group consisting of optionally substituted alkyl, R⁹A¹-, R^{9a}C(=A¹)-;
m = 0 to 2;
n = 0 to 3;
p = 0 to 4;
wherein, R⁹ wherever it appears, is selected from hydrogen, optionally substituted C₁₋₆ alkyl, optionally substituted heteroalkyl, optionally substituted aryl, optionally substituted heteroaryl, optionally substituted cycloalkyl, and optionally substituted heterocyclyl; and A¹ is selected from O and S;
R^{9a} wherever it appears, is selected from optionally substituted C₁₋₆ alkyl, optionally substituted heteroalkyl, optionally substituted aryl, optionally substituted heteroaryl, optionally substituted cycloalkyl, and optionally substituted heterocyclyl;
wherein,
"optionally substituted alkyl", means an alkyl group optionally substituted with 1 to 6 substituents selected independently from the group consisting of oxo, halogen, nitro, cyano, aryl, hereroaryl, cycloalkyl, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-;
"optionally substituted heteroalkyl" means a heteroalkyl group optionally substituted with 1 to 6 substituents selected independently from the group consisting of oxo, halogen, nitro, cyano, aryl, hereroaryl, cycloalkyl.
"optionally substituted cycloalkyl" means a cycloalkyl group optionally substituted with 1 to 6 substituents selected independently from the group consisting of oxo, halogen, nitro, cyano, aryl, hereroaryl, alkyl, R^{10a}C(=O)-, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-;
"optionally substituted aryl" means (i) an aryl group optionally substituted with 1 to 3 substituents selected independently from the group consisting of halogen, nitro, cyano, hydroxy, C₁ to C₆ alkyl, C₃ to C₆ cycloalkyl, C₁ to C₆ perhaloalkyl, alkyl-O-, perhaloalkyl-O-, alkyl-N(alkyl)-, alkyl-N(H)-, H₂N-, alkyl-SO₂-, perhaloalkyl-SO₂-, alkyl-C(=O)N(alkyl)-, alkyl-C(=O)N(H)-, alkyl-N(alkyl)C(=O)-, alkyl-N(H)C(=O)-, H₂NC(=O)-, alkyl-N(alkyl)SO₂-, alkyl-N(H)SO₂-, H₂NSO₂-, 3 to 6 membered heterocycle containing 1 to 2 heteroatoms selected from N, O and S optionally substituted with alkyl or alkyl-C(=O)-, (ii) an aryl ring optionally fused with cycloalkane or heterocycle across a bond optionally substituted with oxo, alkyl or alkyl-C(=O)-;
"optionally substituted heterocyclyl" means a (i) heterocyclyl group optionally substituted on ring carbons with 1 to 6 substituents selected independently from the group consisting of oxo, halogen, nitro, cyano, aryl, hereroaryl, alkyl, R¹⁰A¹-R^{10a}OC(=O)-, R^{10a}C(=O)O-, (R¹⁰)(H)NC(=O)-, (R¹⁰)(alkyl)NC(O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-; (ii) heterocyclyl group optionally substituted on ring nitrogen(s) with substituents selected from the group consisting of aryl, hereroaryl, alkyl, R^{10a}C(=O)-, R^{10a}SO₂-, R^{10a}OC(=O)-, (R¹⁰)(H)NC(=O)-, (R¹⁰)(alkyl)NC(=O)-;
"optionally substituted heteroaryl" means a heteroaryl group optionally substituted with 1 to 3 substituents selected independently from the group consisting of halogen, nitro, cyano, hydroxy, C₁ to C₆ alkyl, C₃ to C₆ cycloalkyl, C₁ to C₆ perhaloalkyl, alkyl-O-, perhaloalkyl-O-, alkyl-N(alkyl)-, alkyl-N(H)-, H₂N-, alkyl-SO₂-, perhaloalkyl-SO₂-, alkyl-C(=O)N(alkyl)-, alkyl-C(=O)N(H)-, alkyl-N(alkyl)C(=O)-, alkyl-N(H)C(=O)-, H₂NC(=O)-, alkyl-N(alkyl)SO₂-, alkyl-N(H)SO₂-, H₂NSO₂-, 3 to 6 membered heterocycle containing 1 to 2 heteroatoms selected from N, O and S optionally substituted with alkyl or alkyl-C(=O)-;
wherein R¹⁰ is selected from hydrogen, alkyl, aryl, heteroaryl, cycloalkyl or heterocyclyl; and A¹ is selected from S and O; and R^{10a} is selected from alkyl, perhaloalkyl, aryl, heteroaryl, cycloalkyl or heterocyclyl.

11. The compound for use in treating a disease or disorder or condition according to claim 10, wherein the compounds are selected from,
4-(5-(4,4-dimethylchroman-6-yl)-2-methyl-3-propionyl-1H-pyrrol-1-yl)benzenesulfonamide;
4-(5-(2,3-dihydrobenzo[b][1,4]dioxin-6-yl)-2-methyl-3-propionyl-1H-pyrrol-1-yl)benzenesulfonamide;
4-(2-(2,3-dihydrobenzo[b][1,4]dioxin-6-yl)-3,5-dimethyl-4-propionyl-1H-pyrrol-1-yl)benzenesulfonamide;
Ethyl 5-(2,3-dihydrobenzo[b][1,4]dioxin-6-yl)-2,4-dimethyl-1-(4-sulfamoylphenyl)-1H-pyrrole-3-carboxylate;
4-(5-(2,3-dihydro-1H-inden-4-yl)-2-methyl-3-propionyl-1H-pyrrol-1-yl)benzenesulfonamide;
4-(5-(2,2-dimethylchroman-6-yl)-2-methyl-3-propionyl-1H-pyrrol-1-yl)benzenesulfonamide;
4-(5-(8-fluoro-4,4-dimethylchroman-6-yl)-2-methyl-3-propionyl-1H-pyrrol-1-yl)benzenesulfonamide;
4-(5-(2-acetyl-4,4-dimethyl-1,2,3,4-tetrahydroisoquinolin-7-yl)-2-methyl-3-propionyl-1H-pyrrol-1-yl)benzenesulfonamide;
4-(5-(2-acetyl-4,4-dimethyl-1,2,3,4-tetrahydroisoquinolin-6-yl)-2-methyl-3-propionyl-1H-pyrrol-1-yl)benzenesulfonamide;
4-(5-(4,4-dimethyl-1,2,3,4-tetrahydroisoquinolin-7-yl)-2-methyl-3-propionyl-1H-pyrrol-1-yl)benzenesulfonamide;
4-(2-methyl-3-propionyl-5-(3H-spiro[benzo[b] [1,4]dioxin-2,1'-cyclopropan]-7-yl)-1H-pyrrol-1-yl)benzenesulfonamide;
4-(2-methyl-3-propionyl-5-(3H-spiro[benzo[b][1,4]dioxine-2,1'-cyclopropan]-6-yl)-1H-pyrrol-1-yl)benzenesulfonamide;
4-(5-(1-acetyl-4,4-dimethyl-1,2,3,4-tetrahydroquinolin-6-yl)-2-methyl-3-propionyl-1H-pyrrol-1-yl)benzenesulfonamide;
4-(5-(1-acetyl-4,4-dimethyl-1,2,3,4-tetrahydroquinolin-7-yl)-2-methyl-3-propionyl-1H-pyrrol-1-yl)benzenesulfonamide;
4-(5-(4,4-dimethyl-1,2,3,4-tetrahydroquinolin-6-yl)-2-methyl-3-propionyl-1H-pyrrol-1-yl)benzenesulfonamide;
4-(5-(4,4-dimethyl-1,2,3,4-tetrahydroquinolin-7-yl)-2-methyl-3-propionyl-1H-pyrrol-1-yl)benzenesulfonamide;
4-(5-(4,4-dimethyl-2-oxo-1,2,3,4-tetrahydroquinolin-6-yl)-2-methyl-3-propionyl-1H-pyrrol-1-yl)benzenesulfonamide;
4-(5-(4,4-dimethyl-2-oxo-1,2,3,4-tetrahydroquinolin-7-yl)-2-methyl-3-propionyl-1H-pyrrol-1-yl)benzenesulfonamide;
4-(2-methyl-3-propionyl-5-(1,4,4-trimethyl-2-oxo-1,2,3,4-tetrahydroquinolin-6-yl)-1H-pyrrol-1-yl)benzenesulfonamide;
4-(2-methyl-3-propionyl-5-(5,6,7,8-tetrahydronaphthalen-2-yl)-1H-pyrrol-1-yl)benzenesulfonamide.

12. The compound for use in treating a disease or disorder or condition according to claim 10, wherein the disorder or condition or disease is selected from the group consisting of Alzheimer's disease, mild cognitive impairment, senile dementia, vascular dementia, dementia of Parkinson's disease, attention deficit disorder, attention deficit hyperactivity disorder, dementia associated with Lewy bodies, AIDS dementia complex, Pick's disease, dementia associated with Down's syndrome, Huntington's disease, cognitive deficits associated with traumatic brain injury, cognitive and sensorimotor gating deficits associated with schizophrenia, cognitive deficits associated with bipolar disorder, cognitive impairments associated with depression, acute pain, post-surgical or post-operative pain, chronic pain, inflammation, inflammatory pain, neuropathic pain, smoking cessation, need for new blood vessel growth associated with wound healing, need for new blood vessel growth associated with vascularization of skin grafts, and lack of circulation, arthritis, rheumatoid arthritis, psoriasis, Crohn's disease, ulcerative colitis, pouchitis, inflammatory bowel disease, celiac disease, periodontitis, sarcoidosis, pancreatitis, organ transplant rejection, acute immune disease associated with organ transplantation, chronic immune disease associated with organ transplantation, septic shock, toxic shock syndrome, sepsis syndrome, depression, and rheumatoid spondylitis, comprising the step of administering a compound of formula I.

13. The compound for use in treating a disease or disorder or condition according to claim 10, wherein the disease or disorder or condition is selected from the group classified or diagnosed as major or minor neurocognitive disorders, or disorders arising due to neurodegeneration.

14. The compound for use in treating a disease or disorder or condition according to claim 10, comprising administering a compound of formula I in combination with or as adjunct to medications used in the treatment of attention deficit hyperactivity disorders, schizophrenia, and other cognitive disorders such as Alzheimer's disease, Parkinson's dementia, vascular dementia or dementia associated with Lewy bodies, traumatic brain injury.

15. The compound for use in treating a disease or disorder or condition according to claim 10, further comprising administering a compound of formula I in combination with or as an adjunct to acetylcholinesterase inhibitors, disease modifying drugs or biologics for neurodegenerative disorders, dopaminergic drugs, antidepressants, typical or an atypical antipsychotic.

## Patentansprüche

1. Eine Verbindung der allgemeinen Formel I, ihre tautomeren Formen, ihre Stereoisomere und ihre pharmazeutisch zulässigen Salze; 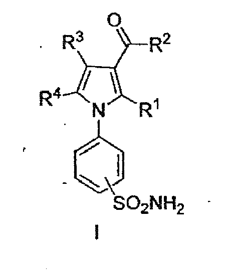 wobei gilt,
R¹ ist ausgewählt aus Wasserstoff, Halogen, optional substituiertem Alkyl, Perhaloalkyl, optional substituiertem Zykloalkyl, optional substituiertem Aryl; optional substituiertem Heterocyclyl, optional substituiertem Heteroaryl;
R² ist ausgewählt aus optional substituiertem Alkyl, optional substituiertem Heteroalkyl, optional substituiertem Aryl, optional substituiertem Heteroaryl, optional substituiertem Zykloalkyl, optional substituiertem Heterocyclyl oder -NR⁵(R⁶), -A¹R⁵, -N(R⁵)OR⁶;
R³ ist ausgewählt aus Wasserstoff, optional substituiertem Alkyl, Halo, optional substituiertem Zykloalkyl, optional substituiertem Aryl, optional substituiertem Heterocyclyl, optional substituiertem Heteroaryl, Cyano, Nitro oder -NR⁵(R⁶), -OR⁵;
R⁴ ist 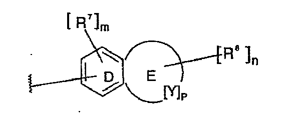
wobei, der Phenylring 'D' mit dem Ring 'E' fusioniert ist, der einen nicht-aromatischen fünfbis achtgliedrigen Ring, der zu der/den 'Y'-Gruppe(n) gehört, darstellt;
Y ist unabhängig ausgewählt bei jeder Wiederholung von -O-, -S-, -NH-, 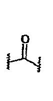 oder 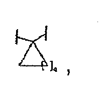 wenn q = 1 - 4 ist; wobei gilt, wenn Y als -NH- oder 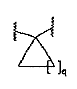 ausgewählt wird, wird es optional substituiert durch [R⁸]ₙ;
wobei gilt, R⁵ und R⁶ sind unabhängig ausgewählt aus Wasserstoff, optional substituiertem Alkyl, optional substituiertem Heteroalkyl, optional substituiertem Aryl, optional substituiertem Heteroaryl, optional substituiertem Zykloalkyl, optional substituiertem Heterocyclyl, R^{9a}C(=A¹)-;
R⁷ wird bei jedem Auftreten unabhängig ausgewählt aus der Gruppe, bestehend aus Halogen, optional substituiertem Alkyl, optional substituiertem Zykloalkyl;
R⁸ wird bei jedem Auftreten unabhängig ausgewählt aus der Gruppe, bestehend aus optional substituiertem Alkyl, R⁹A¹-, R^{9a}C(=A¹)-;
m = 0 bis 2;
n = 0 bis 3;
p = 0 bis 4;
so dass gilt, wenn p = 0 dann n ≠ 0;
wobei R⁹, wo immer es auftritt, ausgewählt wird aus Wasserstoff, optional substituiertem C₁₋₆-Alkyl, optional substituiertem Hereroalkyl, optional substituiertem Aryl, optional substituiertem Heteroaryl, optional substituiertem Zykloalkyl und optional substituiertem Heterocyclyl; und A¹ ist ausgewählt aus O und S;
R^{9a} ist, wo immer es auftritt, ausgewählt aus optional substituiertem C₁₋₆Alkyl, optional substituiertem Heteroalkyl, optional substituiertem Aryl, optional substituiertem Heteroaryl, optional substituiertem Zykloalkyl und optional substituiertem Heterocyclyl;
wobei gilt,
"optional substituiertes Alkyl" bedeutet eine Alkyl-Gruppe, optional substituiert durch 1 bis 6 Substituenten, unabhängig ausgewählt aus der Gruppe, bestehend aus Oxo, Halogen, Nitro, Cyano, Aryl, Hereroaryl, Zykloalkyl, R^{10a}SO₂-, R¹⁰A¹-, R^{10a}OC(=O)-, R^{10a}C(=O)O-, (R¹⁰)(H)NC(=O)-, (R¹⁰)(alkyl)NC(=O)-, R¹⁰aC(=O)N(H)-, (R¹⁰)(H)N-, (R¹⁰)(alkyl)N-, (R¹⁰)(H)NC(=A¹)N(H)-, (R¹⁰)(alkyl)NC(-A¹)N(H)-;
"optional substituiertes Heteroalkyl" bedeutet eine Heteroalkyl-Gruppe, optional substituiert durch 1 bis 6 Substituenten, unabhängig ausgewählt aus der Gruppe, bestehend aus Oxo, Halogen, Nitro, Cyano, Aryl, Heteroaryl, Zykloalkyl.
"optional substituiertes Zykloalkyl" bedeutet eine Zykloalkyl-Gruppe, optional substituiert durch 1 bis 6 Substituenten, unabhängig ausgewählt aus der Gruppe, bestehend aus Oxo, Halogen, Nitro, Cyano, Aryl, Hereroaryl, Alkyl, R^{10a}C(=O)-, R^{10a}SO₂-, R¹⁰A¹-, R^{10a}OC(=O)-, R¹⁰aC(=O)O-, (R¹⁰)(H)NC(=O)-, (R¹⁰)(alkyl)NC(=O)-, R^{10a}C(=O)N(H-, (R¹⁰)(H)N-, (R¹⁰)(alkyl)N-, (R¹⁰)(H)NC(=A¹)N(H)-, (R¹⁰)(alkyl)NC(=A¹)N(H)-;
"optional substituiertes Aryl" bedeutet (i) eine Aryl-Gruppe, optional substituiert durch 1 bis 3 Substituenten, unabhängig ausgewählt aus der Gruppe, bestehend aus Halogen, Nitro, Cyano, Hydroxy, C₁ bis C₆-Alkyl, C₃ bis C₆-Zykloalkyl, C₁ bis C₆-Perhaloalkyl, Alkyl-O-, Perhaloalkyl-O-, Alkyl-N(alkyl)-, Alkyl-N(H)-, H₂N-, Alkyl-SO₂-, Perhaloalkyl-SO₂-, Alkyl-C(=O)N(alkyl)-, Alkyl-C(=O)N(H)-, Alkyl-N(alkyl)C(=O)-, Alkyl-N(H)C(=O)-, H₂NC(=O)-, Alkyl-N(alkyl)SO₂-, Alkyl-N(H)SO₂-, H₂NSO₂-, einem 3- bis 6-gliedrigen Heterozyklus, der 1 bis 2 Heteroatome enthält, ausgewählt aus N, O und S, optional substituiert durch Alkyl oder Alkyl-C(=O)-, (ii) einen Arylring, optional mit Zykloalkan oder einem Heterozyklus über eine Bindung fusioniert, optional substituiert durch Oxo, Alkyl oder Alkyl-C(=O)-;
"optional substituiertes Heterocyclyl" bedeutet (i) eine Heterocyclyl-Gruppe, optional substituiert auf Ringkohlenstoffen mit 1 bis 6 Substituenten, unabhängig ausgewählt aus der Gruppe, bestehend aus Oxo, Halogen, Nitro, Cyano, Aryl, Hereroaryl, Alkyl, R¹⁰A¹-, R^{10a}OC(=O)-, R^{10a}C(=O)O-, (R¹⁰)(H)NC(=O)-, (R¹⁰)(alkyl)NC(O)-, R^{10a}C(=O)N(H)-, (R¹⁰)(H)N-, (R¹⁰)(alkyl)N-, (R¹⁰)(H)NC(=A¹)N(H)-, (R¹⁰)(alkyl)NC(=A¹)N(H)-; (ii) eine Heterocyclyl-Gruppe optional substituiert auf Ring-Stickstoff(en) mit Substituenten, ausgewählt aus der Gruppe, bestehend aus Aryl, Hereroaryl, Alkyl, R^{10a}C(=O)-, R^{10a}SO₂-, R^{10a}OC(=O)-, (R¹⁰)(H)NC(=O)-, (R¹⁰)(alkyl)NC(=O)-;
"optional substituiertes Heteroaryl" bedeutet eine Heteroaryl-Gruppe, optional substituiert durch 1 bis 3 Substituenten, unabhängig ausgewählt aus der Gruppe, bestehend aus Halogen, Nitro, Cyano, Hydroxy, C₁ bis C₆-Alkyl, C₃ bis C₆-Zykloalkyl, C₁ bis C₆-Perhaloalkyl, Alkyl-O-, Perhaloalkyl-O-, Alkyl-N(alkyl)-, Alkyl-N(H)-, H₂N-, Alkyl-SO₂-, Perhaloalkyl-SO₂-, Alkyl-C(=O)N(alkyl), Alkyl-C(=O)N(H)-, Alkyl-N(alkyl)C(=O)-, Alkyl-N(H)C(=O)-, H₂NC(=O)-, Alkyl-N(alkyl)SO₂-, Alkyl-N(H)SO₂-, H₂NSO₂-, einem 3- bis 6-gliedrigen Heterozyklus, der 1 bis 2 Heteroatome enthält, ausgewählt aus N, O und S, optional substituiert durch Alkyl oder Alkyl-C(=O)-;
wobei gilt R¹⁰ ist ausgewählt aus Wasserstoff, Alkyl, Aryl, Heteroaryl, Zykloalkyl oder Heterocyclyl; und A1 ist ausgewählt aus S und O; und R^{10a} ist ausgewählt aus Alkyl, Perhaloalkyl, Aryl, Heteroaryl, Zykloalkyl oder Heterocyclyl.

2. Die Verbindung der Formel I gemäß Anspruch 1, wobei R¹ als Methyl ausgewählt ist.

3. Die Verbindung der Formel I gemäß Anspruch 1, wobei R² aus Ethyl und Ethoxy ausgewählt ist.

4. Die Verbindung der Formel I gemäß Anspruch 1, wobei R³ aus Wasserstoff und Methyl ausgewählt ist.

5. Die Verbindung der Formel I gemäß Anspruch 1, wobei R⁴ ausgewählt ist aus

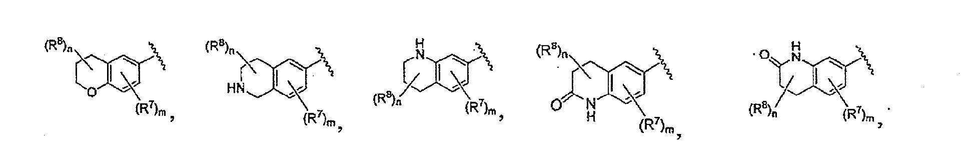

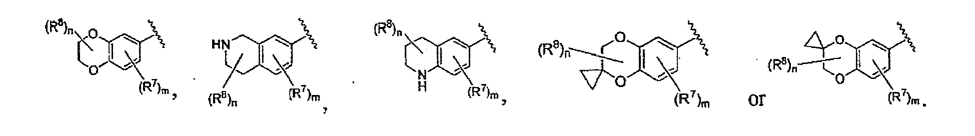

6. Die Verbindung der Formel I gemäß Anspruch 1, wobei R¹ ausgewählt ist aus Methyl, R² ausgewählt ist aus Ethyl und Ethoxy, R³ ausgewählt ist aus Wasserstoff und Methyl und R⁴ ausgewählt ist aus

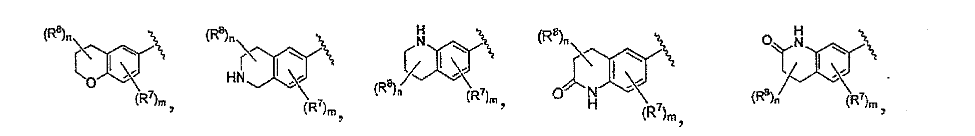

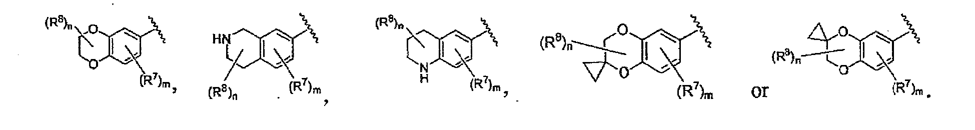

7. Die Verbindung der Formel I gemäß Anspruch 1, wobei die Verbindung ausgewählt ist aus
4-(5-(4,4-dimethylchroman-6-yl)-2-methyl-3-propionyl-1*H*-pyrrol-1-yl)benzenesulfonamid;
4-(5-(2,3-dihydrobenzo[b][1,4]dioxin-6-yl)-2-methyl-3-propionyl-1H-pyrrol-1-yl)benzenesulfonamid;
4-(2-(2,3-dihydrobenzo[b][1,4]dioxin-6-yl)-3,5-dimethyl-4-propionyl-1H-pyrrol-1-yl)benzenesulfonamid;
Ethyl 5-(2,3-dihydrobenzo[b][1,4]dioxin-6-yl)-2,4-dimethyl-1-(4-sulfamoylphenyl)-1H-pyrrol-3-carboxylat;
4-(5-(2,2-dimethylchroman-6-yl)-2-methyl-3-propionyl-1H-pyrrol-1-yl)benzenesulfonamid;
4-(5-(8-fluor-4,4-dimethylchroman-6-yl)-2-methyl-3-propionyl-1H-pyrrol-1-yl)benzenesulfonamid;
4-(5-(2-acetyl-4,4-dimethyl-1,2,3,4-tetrahydroisoquinolin-7-yl)-2-methyl-3-propionyl-1H-pyrrol-1-yl)benzenesulfonamid;
4-(5-(2-acetyl-4,4-dimethyl-1,2,3,4-tetrahydroisoquinolin-6-yl)-2-methyl-3-propionyl-1 H-pyrrol-1-yl)benzenesulfonamid;
4-(5-(4,4-dimethyl-1,2,3,4-tetrahydroisoquinolin-7-yl)-2-methyl-3-propionyl-1H-pyrrol-1-yl)benzenesulfonamid;
4-(2-methyl-3-propionyl-5-(3H-spiro[benzo[b][1,4]dioxin-2,1'-cyclopropan]-7-yl)-1H-pyrrol-1-yl)benzenesulfonamid;
4-(2-methyl-3-propionyl-5-(3H-spiro[benzo[b][1,4]dioxin-2,1'-cyclopropan]-6-yl)-1H-pyrrol-1-yl)benzenesulfonamid;
4-(5-(1-acetyl-4,4-dimethyl-1,2,3,4-tetrahydroquinolin-6-yl)-2-methyl-3-propionyl-1H-pyrrol-1-yl)benzenesulfonamid;
4-(5-(1-acetyl-4,4-dimethyl-1,2,3,4-tetrahydroquinolin-7-yl)-2-methyl-3-propionyl-1H-pyrrol-1-yl)benzenesulfonamid;
4-(5-(4,4-dimethyl-1,2,3,4-tetrahydroquinolin-6-yl)-2-methyl-3-propionyl-1H-pyrrol-1-yl)benzenesulfonamid;
4-(5-(4,4-dimethyl-1,2,3,4-tetrahydroquinolin-7-yl)-2-methyl-3-propionyl-1H-pyrrol-1-yl)benzenesulfonamid;
4-(5-(4,4-dimethyl-2-oxo-1,2,3,4-tetrahydroquinolin-6-yl)-2-methyl-3-propionyl-1 H-pyrrol-1-yl)benzenesulfonamid;
4-(5-(4,4-dimethyl-2-oxo-1,2,3,4-tetrahydroquinolin-7-yl)-2-methyl-3-propionyl-1H-pyrrol-1-yl)benzenesulfonamid;
4-(2-methyl-3-propionyl-5-(1,4,4-trimethyl-2-oxo-1.2,3,4-tetrahydroquinolin-6-yl)-1H-pyrrol-1-yl)benzenesulfonamid.

8. Eine pharmazeutische Zusammensetzung, bestehend aus einer Verbindung gemäß Anspruch 1 und einem pharmazeutisch zulässigen Trägerstoff.

9. Eine Verbindung gemäß Anspruch 1 zur Verwendung bei der Prävention oder Behandlung einer Krankheit oder ihrer Symptome oder einer Störung, die teilweise oder vollständig durch nikotinische Acetylcholinrezeptoren vermittelt ist.

10. Eine Verbindung der Formel I zur Verwendung bei der Behandlung einer Krankheit oder einer Störung oder eines Zustandes, 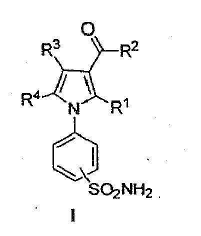 wobei gilt,
R¹ ist ausgewählt aus Wasserstoff, Halogen, optional substituiertem Alkyl, Perhaloalkyl, optional substituiertem Zykloalkyl, optional substituiertem Aryl; optional substituiertem Heterocyclyl, optional substituiertem Heteroaryl;
R² ist ausgewählt aus optional substituiertem Alkyl, optional substituiertem Heteroalkyl, optional substituiertem Aryl, optional substituiertem Heteroaryl, optional substituiertem Zykloalkyl, optional substituiertem Heterocyclyl oder-NR⁵(R⁶), -NR¹, -N(R⁵)OR⁶;
R³ ist ausgewählt aus Wasserstoff, optional substituiertem Alkyl, Halo, optional substituiertem Zykloalkyl, optional substituiertem Aryl, optional substituiertem Heterocyclyl, optional substituiertem Heteroaryl, Cyano, Nitro oder -NR⁵(R⁶), -OR⁵;
R⁴ ist 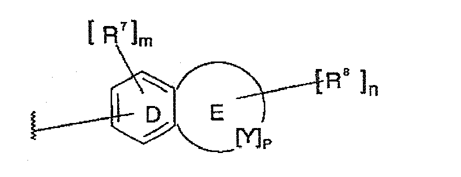
wobei, der Phenylring 'D' mit dem Ring 'E' fusioniert ist, der einen nicht-aromatischen fünfbis achtgliedrigen Ring, der zu der/den 'Y'-Gruppe(n) gehört, darstellt;
Y ist unabhängig ausgewählt bei jeder Wiederholung von -O-, -S-, -NH-, 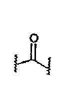 oder 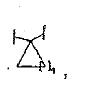
wenn q = 1 - 4 ist; wobei gilt, wenn Y als -NH- oder 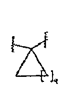 ausgewählt wird, ist es optional substituiert durch [R⁸]ₙ;
wobei gilt, R⁵ und R⁶ sind unabhängig ausgewählt aus Wasserstoff, optional substituiertem Alkyl, optional substituiertem Heteroalkyl, optional substituiertem Aryl, optional substituiertem Heteroaryl, optional substituiertem Zykloalkyl, optional substituiertem Heterocyclyl, R⁹aC(=A¹)-;
R⁷ wird bei jedem Auftreten unabhängig ausgewählt aus der Gruppe, bestehend aus Halogen, optional substituiertem Alkyl, optional substituiertem Zykloalkyl;
R⁸ wird bei jedem Auftreten unabhängig ausgewählt aus der Gruppe, bestehend aus optional substituiertem Alkyl, R⁹A¹-, R^{9a}C(=A¹)-;
m = 0 bis 2;
n = 0 bis 3;
p = 0 bis 4;
wobei R⁹, wo immer es auftritt, ausgewählt wird aus Wasserstoff, optional substituiertem C₁₋₆-Alkyl, optional substituiertem Hereroalkyl, optional substituiertem Aryl, optional substituiertem Heteroaryl, optional substituiertem Zykloalkyl und optional substituiertem Heterocyclyl; und A¹ ausgewählt ist aus O und S;
R^{9a} ist, wo immer es auftritt, ausgewählt aus optional substituiertem C₁₋₆Alkyl, optional substituiertem Heteroalkyl, optional substituiertem Aryl, optional substituiertem Heteroaryl, optional substituiertem Zykloalkyl und optional substituiertem Heterocyclyl;
wobei gilt,
"optional substituiertes Alkyl", bedeutet eine Alkyl-Gruppe, optional substituiert durch 1 bis 6 Substituenten, unabhängig ausgewählt aus der Gruppe, bestehend aus Oxo, Halogen, Nitro, Cyano, Aryl, Hereroaryl, Zykloalkyl, R^{10a}SO₂-, R¹⁰A¹-, R¹⁰aOC(=O)-, R¹⁰aC(=O)O-, (R¹⁰)(H)NC(=O)-, (R¹⁰)(alkyl)NC(=O)-, R¹⁰aC(=O)N(H)-, (R¹⁰)(H)N- (R¹⁰)(alkyl)N-, (R¹⁰)(H)NC(=A¹)N(H)-, (R¹⁰)(alkyl)NC(=A¹)N(H)-;
"optional substituiertes Heteroalkyl" bedeutet eine Heteroalkyl-Gruppe, optional substituiert durch 1 bis 6 Substituenten, unabhängig ausgewählt aus der Gruppe, bestehend aus Oxo, Halogen, Nitro, Cyano, Aryl, Heteroaryl, Zykloalkyl.
"optional substituiertes Zykloalkyl" bedeutet eine Zykloalkyl-Gruppe, optional substituiert durch 1 bis 6 Substituenten, unabhängig ausgewählt aus der Gruppe, bestehend aus Oxo, Halogen, Nitro, Cyano, Aryl, Hereroaryl, Alkyl, R^{10a}C(=O)-, R^{10a}SO₂-, R¹⁰A¹-, R¹⁰aOC(=O)-, R^{10a}C(=O)O-, (R¹⁰)(H)NC(=O)-, (R¹⁰)(alkyl)NC(=O)-, R^{10a}C(=O)N(H-, (R¹⁰)(H)N-, (R¹⁰)(alkyl)N-, (R¹⁰)(H)NC(=A¹)N(H)-, (R¹⁰)(alkyl)NC(=A¹)N(H)-;
"optional substituiertes Aryl" bedeutet (i) eine Aryl-Gruppe, optional substituiert durch 1 bis 3 Substituenten, unabhängig ausgewählt aus der Gruppe, bestehend aus Halogen, Nitro, Cyano, Hydroxy, C₁ bis C₆-Alkyl, C₃ bis C₆-Zykloalkyl, C₁ bis C₆-Perhaloalkyl, Alkyl-O-, Perhaloalkyl-O-, Alkyl-N(alkyl)-, Alkyl-N(H)-, H₂N-, Alkyl-SO₂-, Perhaloalkyl-SO₂-, Alkyl-C(=O)N(alkyl)-, Alkyl-C(=O)N(H)-, Alkyl-N(alkyl)C(=O)-, Alkyl-N(H)C(=O)-, H₂NC(=O)-, Alkyl-N(alkyl)SO₂-, Alkyl-N(H)SO₂- H₂NSO₂-, einem 3- bis 6-gliedrigen Heterozyklus, der 1 bis 2 Heteroatome enthält, ausgewählt aus N, O und S, optional substituiert durch Alkyl oder Alkyl-C(=O)-, (ii) einen Arylring, optional mit Zykloalkan oder einem Heterozyklus über eine Bindung fusioniert, optional substituiert durch Oxo, Alkyl oder Alkyl-C(=O)-;
"optional substituiertes Heterocyclyl" bedeutet (i) eine Heterocyclyl-Gruppe, optional substituiert auf Ringkohlenstoffen mit 1 bis 6 Substituenten, unabhängig ausgewählt aus der Gruppe, bestehend aus Oxo, Halogen, Nitro, Cyano, Aryl, Hereroaryl, Alkyl, R¹⁰A¹-, R^{10a}OC(=O)-, R^{10a}C(=O)O-, (R¹⁰)(H)NC(=O)-, (R¹⁰)(alkyl)NC(O)-, R^{10a}C(=O)N(H)- (R¹⁰)(H)N-, (R¹⁰)(alkyl)N-, (R¹⁰)(H)NC(=A¹)N(H)-, (R¹⁰)(alkyl)NC(=A¹)N(H)-; (ii) eine Heterocyclyl-Gruppe optional substituiert auf Ring-Stickstoff(en) mit Substituenten, ausgewählt aus der Gruppe, bestehend aus Aryl, Hereroaryl, Alkyl, R^{10a}C(=O)-, R^{10a}SO₂-, R^{10a}OC(=O)-, (R¹⁰)(H)NC(=O)-, (R¹⁰)(alkyl)NC(=O)-;
"optional substituiertes Heteroaryl" bedeutet eine Heteroaryl-Gruppe optional substituiert durch 1 bis 3 Substituenten, unabhängig ausgewählt aus der Gruppe, bestehend aus Halogen, Nitro, Cyano, Hydroxy, C₁ bis C₆-Alkyl, C₃ bis C₆-Zykloalkyl, C₁ bis C₆-Perhaloalkyl, Alkyl-O-, Perhaloalkyl-O-, Alkyl-N(alkyl)-, Alkyl-N(H)-, H₂N-, Alkyl-SO₂-, Perhaloalkyl-SO₂-, Alkyl-C(=O)N(alkyl)-, Alkyl-C(=O)N(H)-, Alkyl-N(alkyl)C(=O)-, Alkyl-N(H)C(=O)-, H₂NC(=O)-, Alkyl-N(alkyl)SO₂-, Alkyl-N(H)SO₂- H₂NSO₂-, einen 3- bis 6-gliedrigen Heterozyklus, bestehend aus 1 bis 2 Heteroatomen, ausgewählt aus N, O und S, optional substituiert durch Alkyl oder Alkyl-C(=O)-;
wobei gilt R¹⁰ ist ausgewählt aus Wasserstoff, Alkyl, Aryl, Heteroaryl, Zykloalkyl oder Heterocyclyl; und A¹ ist ausgewählt aus S und O; und R^{10a} ist ausgewählt aus Alkyl, Perhaloalkyl, Aryl, Heteroaryl, Zykloalkyl oder Heterocyclyl.

11. Die Verbindung zur Verwendung bei der Behandlung einer Krankheit oder einer Störung oder eines Zustandes gemäß Anspruch 10, wobei die Verbindungen ausgewählt sind aus,
4-(5-(4,4-dimethylchroman-6-yl)-2-methyl-3-propionyl-1*H*-pyrrol-1-yl)benzenesulfonamid;
4-(5-(2,3-dihydrobenzo[b][1,4]dioxin-6-yl)-2-methyl-3-propionyl-1H-pyrrol-1-yl )benzenesulfonamid;
4-(2-(2,3-dihydrobenzo[b][1,4]dioxin-6-yl)-3,5-dimethyl-4-propionyl-1H-pyrrol-1-yl)benzenesulfonamid;
Ethyl 5-(2,3-dihydrobenzo[b][1,4]dioxin-6-yl)-2,4-dimethyl-1-(4-sulfamoylphenyl)-1H-pyrrol-3-carboxylat;
4-(5-(2,3-dihydro-1H-inden-4-yl)-2-methyl-3-propionyl-1H-pyrrol-1-yl)benzenesulfonamid;
4-(5-(2,2-dimethylchroman-6-yl)-2-methyl-3-propionyl-1H-pyrrol-1-yl)benzenesulfonamid;
4-(5-(8-fluor-4,4-dimethylchroman-6-yl)-2-methyl-3-propionyl-1H-pyrrol-1-yl)benzenesulfonamid;
4-(5-(2-acetyl-4,4-dimethyl-1,2,3,4-tetrahydroisoquinolin-7-yl)-2-methyl-3-propionyl-1 H-pyrrol-1-yl)benzenesulfonamid;
4-(5-(2-acetyl-4,4-dimethyl-1,2,3,4-tetrahydroisoquinolin-6-yl)-2-methyl-3-propionyl-1H-pyrrol-1-yl)benzenesulfonamid;
4-(5-(4,4-dimethyl-1,2,3,4-tetrahydroisoquinolin-7-yl)-2-methyl-3-propionyl-1H-pyrrol-1-yl)benzenesulfonamid;
4-(2-methyl-3-propionyl-5-(3H-spiro[benzo[b][1,4]dioxin-2,1'-cyclopropan]-7-yl)-1H-pyrrol-1-yl)benzenesulfonamid;
4-(2-methyl-3-propionyl-5-(3H-spiro[benzo[b][1,4]dioxin-2,1'-cyclopropan]-6-yl)-1H-pyrrol-1-yl)benzenesulfonamid;
4-(5-(1-acetyl-4,4-dimethyl-1,2,3,4-tetrahydroquinolin-6-yl)-2-methyl-3-propionyl-1H-pyrrol-1-yl)benzenesulfonamid;
4-(5-(1-acetyl-4,4-dimethyl-1,2,3,4-tetrahydroquinolin-7-yl)-2-methyl-3-propionyl-1H-pyrrol-1-yl)benzenesulfonamid;
4-(5-(4,4-dimethyl-1,2,3,4-tetrahydroquinolin-6-yl)-2-methyl-3-propionyl-1H-pyrrol-1-yl)benzenesulfonamid;
4-(5-(4,4-dimethyl-1,2,3,4-tetrahydroquinolin-7-yl)-2-methyl-3-propionyl-1H-pyrrol-1-yl)benzenesulfonamid;
4-(5-(4,4-dimethyl-2-oxo-1,2,3,4-tetrahydroquinolin-6-yl)-2-methyl-3-propionyl-1H-pyrrol-1-yl)benzenesulfonamid;
4-(5-(4,4-dimethyl-2-oxo-1,2,3,4-tetrahydroquinolin-7-yl)-2-methyl-3-propionyl-1H-pyrrol-1-yl)benzenesulfonamid;
4-(2-methyl-3-propionyl-5-(1,4,4-trimethyl-2oxo-1,2,3,4-tetrahydroquinolin-6-yl)-1H-pyrrol-1-yl)benzenesulfonamid;
4-(2-methyl-3-propionyl-5-(5,6,7,8-tetrahydroquinolin-2-yl)-1 H-pyrrol-1-yl)benzenesulfonamid.

12. Die Verbindung für die Verwendung bei der Behandlung einer Krankheit oder Störung oder eines Zustandes gemäß Anspruch 10, wobei die Störung oder der Zustand oder die Krankheit ausgewählt ist aus der Gruppe, bestehend aus Alzheimer, leichter kognitiver Beeinträchtigung, seniler Demenz, vaskulärer Demenz, Demenz der Parkinsonkrankheit, Aufmerksamkeitsdefizitsyndrom, Aufmerksamkeitsdefizitsyndrom mit Hyperaktivität, Demenz in Verbindung mit Lewy-Körperchen, AIDS-Demenz-Komplex, Pick-Krankheit, Demenz in Verbindung mit dem Down-Syndrom, Huntington-Krankheit, kognitive Defizite in Verbindung mit traumatischer Hirnverletzung, kognitive und sensomotorisches Gating-Defizit in Verbindung mit Schizophrenie, kognitive Defizite in Verbindung mit einer bipolaren Störung, kognitive Beeinträchtigungen in Verbindung mit Depression, akute Schmerzen, postchirurgische oder postoperative Schmerzen, chronische Schmerzen, Entzündungen, entzündliche Schmerzen, neuropathische Schmerzen, Raucherentwöhnung, Notwendigkeit neuen Blutgefäßwachstums in Verbindung mit Wundheilung, Notwendigkeit neuen Blutgefäßwachstums in Verbindung mit der Vaskularisierung bei Hauttransplantationen, und Durchblutungsstörungen, Arthritis, Gelenkrheumatismus, Psoriasis, Morbus Crohn, Colitis ulcerosa, Pouchitis, chronisch-entzündliche Darmerkrankungen, Zöliakie, Periodontitis, Sarkoidose, Bauchspeicheldrüsenentzündung, Abstoßungsreaktionen bei Organtransplantationen, akute Immunkrankheit in Verbindung mit Organtransplantationen, chronische Immunkrankheit in Verbindung mit Organtransplantationen, septischer Schock, toxisches Schocksyndrom, Sepsissyndrom, Depression und Spondylitis ankylosans, und die den Schritt der Verabreichung einer Verbindung der Formel I umfasst.

13. Die Verbindung zur Verwendung bei der Behandlung einer Krankheit oder Störung oder eines Zustandes gemäß Anspruch 10, wobei die Krankheit oder Störung oder der Zustand ausgewählt ist aus der Gruppe, die als schwere oder leichte neurokognitive Störungen oder Störungen aufgrund neurodegenerativer Erkrankungen klassifiziert oder diagnostiziert sind.

14. Die Verbindung zur Verwendung bei der Behandlung einer Krankheit oder Störung oder eines Zustandes gemäß Anspruch 10, bestehend aus der Verabreichung einer Verbindung der Formel I in Kombination mit oder als Zusatz zu Medikationen, die bei der Behandlung von Aufmerksamkeitsdefizitsyndromen mit Hyperaktivität, Schizophrenie und anderer kognitiver Beeinträchtigungen, wie Alzheimer, Parkinson-Demenz, vaskulärer Demenz oder Demenz in Verbindung mit Lewy-Körperchen und traumatischen Hirnverletzungen, eingesetzt werden.

15. Die Verbindung zur Verwendung bei der Behandlung einer Krankheit oder Störung oder eines Zustandes gemäß Anspruch 10, darüberhinaus bestehend aus der Verabreichung einer Verbindung der Formel I in Kombination mit oder als Zugabe zu Inhibitoren der Acetylcholinhydrolase, krankheitsverändernden Medikamenten oder biologischen Präparaten für neurodegenerative Störungen, dopaminergen Medikamenten, Antidepressiva, typischen oder atypischen antipsychotischen Medikamenten.

## Revendications

1. Composé de formule générale I, ses formes tautomériques, ses stéréoisomères et sels pharmaceutiquement acceptables ; 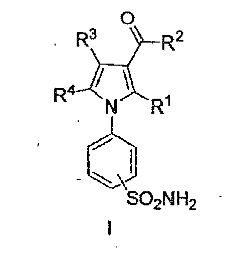 dans lequel,
R¹ est sélectionné parmi : hydrogène, halogène, alkyle substitué optionnellement, perhaloalkyle, cycloalkyle substitué optionnellement, aryle substitué optionnellement ; hétérocyclyle substitué optionnellement, hétéroaryle substitué optionnellement ;
R² est sélectionné parmi : alkyle substitué optionnellement, hétéroalkyle substitué optionnellement, aryle substitué optionnellement, hétéroaryle substitué optionnellement, cycloalkyle substitué optionnellement, hétérocyclyle substitué optionnellement, ou -NR⁵(R⁶)-A¹R⁵-N(R⁵)OR⁶ ;
R³ est sélectionné parmi : hydrogène, alkyle substitué optionnellement, halo, cycloalkyle substitué optionnellement, aryle substitué optionnellement, hétérocyclyle substitué optionnellement, hétéroaryle substitué optionnellement, cyano, nitro ou -NR⁵(R⁶)-OR⁵ ;
R⁴ est 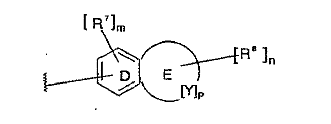
dans lequel l'anneau de phényle 'D' est fusionné avec l'anneau 'E', qui est un anneau de cinq à huit éléments non-aromatique incluant un ou des groupes 'Y' ;
Y est sélectionné indépendamment à chaque répétition parmi -O--S--NH-, 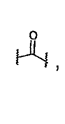 ou 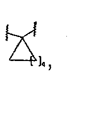 où q = 1 - 4 ; dans lequel, lorsque Y est sélectionné en tant que -NH- ou 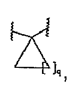 il est optionnellement substitué par [R⁸]ₙ ;
dans lequel, R⁵ et R⁶ sont sélectionnés indépendamment parmi : hydrogène, alkyle substitué optionnellement, hétéroalkyle substitué optionnellement, aryle substitué optionnellement, hétéroaryle substitué optionnellement, cycloalkyle substitué optionnellement, hétérocyclyle substitué optionnellement, R^{9a}C(=A¹)- ;
R⁷ est sélectionné indépendamment à chaque occurrence dans le groupe composé de halogène, alkyle substitué optionnellement, cycloalkyle substitué optionnellement ;
R⁸ est sélectionné indépendamment à chaque occurrence dans le groupe composé de : alkyle substitué optionnellement, R⁹A¹-, R^{9a}C(=A¹)- ;
m = 0 à 2 ;
n = 0 à 3 ;
p = 0 à 4 ;
de sorte que, lorsque p = 0, alors n ≠ 0 ;
dans lequel, R⁹ partout où il apparaît, est sélectionné parmi : hydrogène, optionnellement substitué C₁₋₆ alkyle, hétéroalkyle substitué optionnellement, aryle substitué optionnellement, hétéroaryle substitué optionnellement, cycloalkyle substitué optionnellement, et hétérocyclyle substitué optionnellement ; et A¹ est sélectionné parmi : O et S ;
R^{9a} partout où il apparaît, est sélectionné parmi : optionnellement substitué C₁₋₆ alkyle, hétéroalkyle substitué optionnellement, aryle substitué optionnellement, hétéroaryle substitué optionnellement, cycloalkyle substitué optionnellement, et hétérocyclyle substitué optionnellement ;
dans lequel,
"alkyle substitué optionnellement, ", signifie un groupe alkyle optionnellement substitué par 1 à 6 substituants sélectionnés indépendamment dans le groupe composé d'oxo, halogène, nitro, cyano, aryle, hétéroaryle, cycloalkyle, R^{10a}SO₂-, R¹⁰A¹-, R^{10a}OC(=O)-, R^{10a}C(=O)O-, (R¹⁰)(H)NC(=O)-, (R¹⁰)(alkyle)NC(=O)-, R^{10a}C(=O)N(H)-, (R¹⁰)(H)N-, (R¹⁰)(alkyle)N-, (R¹⁰)(H)NC(=A¹)N(H)-, (R¹⁰)(alkyle)NC(-A¹)N(H)- ;
"hétéroalkyle substitué optionnellement, " signifie un groupe hétéroalkyle optionnellement substitué par 1 à 6 substituants sélectionnés indépendamment dans le groupe composé d'oxo, halogène, nitro, cyano, aryle, hétéroaryle, cycloalkyle.
"cycloalkyle substitué optionnellement, " signifie un groupe cycloalkyle optionnellement substitué par 1 à 6 substituants sélectionnés indépendamment dans le groupe composé d'oxo, halogène, nitro, cyano, aryle, hétéroaryle, alkyle, R^{10a}C(=O)-, R^{10a}SO₂-, R¹⁰A¹-, R^{11a}OC(=O)-, R^{10a}C(=O)O-, (R¹⁰)(H)NC(=O)-, (R¹⁰)(alkyle)NC(=O)-, R^{10a}C(=O)N(H-, (R¹⁰)(H)N-, (R¹⁰)(alkyle)N-, (R¹⁰)(H)NC(=A¹)N(H)-, (R¹⁰)(alkyle)NC(=A¹)N(H)- ;
"aryle substitué optionnellement, " signifie (i) un aryle optionnellement substitué par 1 à 3 substituants sélectionnés indépendamment dans le groupe composé de halogène, nitro, cyano, hydroxy, C₁ à C₆ alkyle, C₃ à C₆ cycloalkyle, C₁ à C₆ perhaloalkyle, alkyle-O-, perhaloalkyle-O-, alkyle-N(alkyle)-, alkyle-N(H)-, H₂N-, alkyle-SO₂-, perhaloalkyle-SO₂-, alkyle-C(=O)N(alkyle)-, alkyle-C(=O)N(H)-, alkyle-N(alkyle)C(=O)-, alkyle-N(H)C(=O)-, H₂NC(=O)-, alkyle-N(alkyle)SO₂-, alkyle-N(H)SO₂-, H₂NSO₂-, hétérocycle de 3 à 6 éléments contenant 1 à 2 hétéroatomes sélectionnés parmi N, O et S optionnellement substitués par de l'alkyle ou de l'alkyle-C(=O)-, (ii) un anneau d'aryle optionnellement fusionné avec un cycloalkane ou hétérocycle sur une liaison optionnellement substituée par de l'oxo, alkyle ou alkyle-C(=O)- ;
"hétérocyclyle substitué optionnellement, " signifie un groupe (i) hétérocyclyle optionnellement substitué sur un anneau de carbone par 1 à 6 substituants sélectionnés indépendamment dans le groupe composé d'oxo, halogène, nitro, cyano, aryle, hétéroaryle, alkyle, R¹⁰A¹-, R^{10a}OC(=O)-, R^{10a}C(=O)O-, (R¹⁰)(H)NC(=O)-, (R¹⁰)(alkyle)NC(O)-, R^{10a}C(=O)N(H)-, (R¹⁰)(H)N-, (R¹⁰)(alkyle)N-, (R¹⁰)(H)NC(=A¹)N(H)-, (R¹⁰)(alkyle)NC(=A¹)N(H)- ; (ii) hétérocyclyle optionnellement substitué sur un anneau d'azote(s) par des substituants sélectionnés parmi the groupe composé d'aryle, hétéroaryle, alkyle, R^{10a}C(=O₎₋, R^{10a}SO₂-, R^{10a}OC(=O)-, (R¹⁰)(H)NC(=O)-, (R¹⁰)(alkyle)NC(=O)- ;
"hétéroaryle substitué optionnellement, " signifie un groupe hétéroaryle optionnellement substitué par 1 à 3 substituants sélectionnés indépendamment dans le groupe composé de halogène, nitro, cyano, hydroxy, C₁ à C₆ alkyle, C₃ à C₆ cycloalkyle, C₁ à C₆ perhaloalkyle, alkyle-O-, perhaloalkyle-O-, alkyle-N(alkyle)-, alkyle-N(H)-, H₂N-, alkyle-SO₂-, perhaloalkyle-SO₂- alkyle-C(=O)N(alkyle), alkyle-C(=O)N(H)-, alkyle-N(alkyle)C(=O)-, alkyle-N(H)C(=O)-, H₂NC(=O)-, alkyle-N(alkyle)SO₂- alkyle-N(H)SO₂-, H₂NSO₂-, hétérocycle de 3 à 6 éléments contenant 1 à 2 hétéroatomes sélectionnés parmi N, O et S optionnellement substitué par alkyle ou alkyle-C(=O)- ;
dans lequel R¹⁰ est sélectionné parmi : hydrogène, alkyle, aryle, hétéroaryle, cycloalkyle ou hétérocyclyle ; et A1 est sélectionné parmi : S et O ; et R^{10a} est sélectionné parmi : alkyle, perhaloalkyle, aryle, hétéroaryle, cycloalkyle ou hétérocyclyle, .

2. Composé de formule I selon la revendication 1, dans lequel R¹ est sélectionné en tant que méthyle, .

3. Composé de formule I selon la revendication 1, dans lequel R² est sélectionné parmi : éthyle et éthoxy.

4. Composé de formule I selon la revendication 1, dans lequel R³ est sélectionné parmi : hydrogène et méthyle.

5. Composé de formule I selon la revendication 1, dans lequel R⁴ est sélectionné parmi :

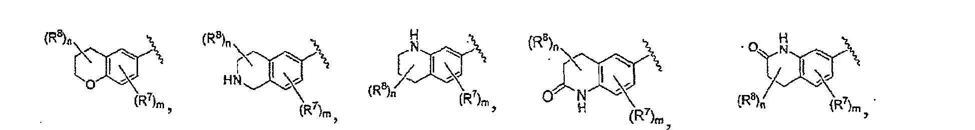

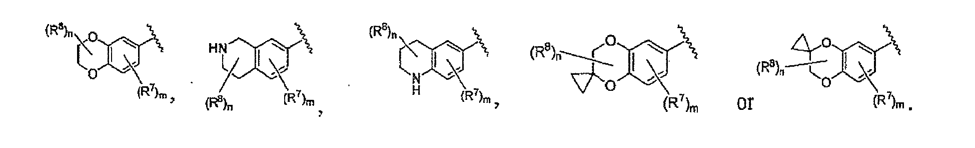

6. Composé de formule I selon la revendication 1, dans lequel R¹ est sélectionné parmi : méthyle, R² est sélectionné parmi : éthyle et éthoxy, R³ est sélectionné parmi : hydrogène et méthyle, et R⁴ est sélectionné parmi :

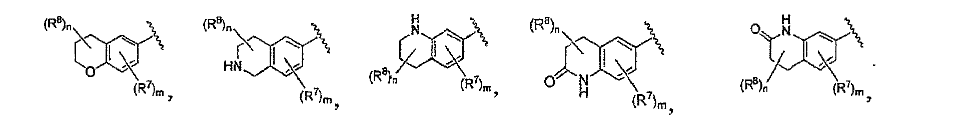

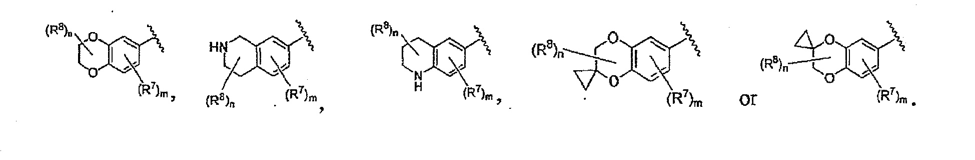

7. Composé de formule I selon la revendication 1, dans lequel Composé est sélectionné parmi :-
4-(5-(4,4-diméthyle, chromane-6-yle)-2-méthyle-3-propionyle-1*H*-pyrrole-1-yle)benzènesulfonamide ;
4-(5-(2,3-dihydrobenzo[b][1,4]dioxin-6-yle)-2-méthyle-3-propionyle-1H-pyrrole-1-yle)benzènesulfonamide ;
4-(2-(2,3-dihydrobenzo[b][1,4]dioxin-6-yle)-3,5-diméthyle-4-propionyle-1H-pyrrole-1-yle)benzènesulfonamide ;
éthyle 5-(2,3-dihydrobenzo[b][1,4]dioxin-6-yle)-2,4-diméthyle-1-(4-sulfamoyle, phényle)-1H-pyrrolee-3-carboxylate ;
4-(5-(2,2-diméthyle, chromane-6-yle)-2-méthyle-3-propionyle-1H-pyrrole-1-yle)benzènesulfonamide ;
4-(5-(8-fluoro-4,4-diméthyle, chromane-6-yle)-2-méthyle-3-propionyle-1H-pyrrole-1-yle)benzènesulfonamide ;
4-(5-(2-acétyle-4,4-diméthyle-1,2,3,4-tétrahydroisoquinoline-7-yle)-2-méthyle-3-propionyle-1H-pyrrole-1-yle)benzènesulfonamide ;
4-(5-(2-acétyle-4,4-diméthyle-1,2,3,4-tétrahydroisoquinoline-6-yle)-2-méthyle-3-propionyle-1H-pyrrole-1-yle)benzènesulfonamide ;
4-(5-(4,4-diméthyle-1,2,3,4-tétrahydroisoquinoline-7-yle)-2-méthyle-3-propionyle-1 H-pyrrole-1-yle)benzènesulfonamide ;
4-(2-méthyle-3-propionyle-5-(3H-spiro[benzo[b][1,4]dioxine-2,1'-cyclopropane]-7-yle)-1H-pyrrole-1-yle)benzènesulfonamide ;
4-(2-méthyle-3-propionyle-5-(3H-spiro[benzo[b][1,4]dioxine-2,1'-cyclopropane]-6-yle)-1H-pyrrole-1-yle)benzènesulfonamide ;
4-(5-(1-acétyle-4,4-diméthyle-1,2,3,4-tétrahydroquinoline-6-yle)-2-méthyle-3-propionyle-1H-pyrrole-1-yle)benzènesulfonamide ;
4-(5-(1-acétyle-4,4-diméthyle-1,2,3,4-tétrahydroquinoline-7-yle)-2-méthyle-3-propionyle-1H-pyrrole-1-yle)benzènesulfonamide ;
4-(5-(4,4-diméthyle-1,2,3,4-tétrahydroquinoline-6-yle)-2-méthyle-3-propionyle-1 H-pyrrole-1-yle)benzènesulfonamide ;
4-(5-(4,4-diméthyle-1,2,3,4-tétrahydroquinoline-7-yle)-2-méthyle-3-propionyle-1 H-pyrrole-1-yle)benzènesulfonamide ;
4-(5-(4,4-diméthyle-2-oxo-1,2,3,4-tétrahydroquinoline-6-yle)-2-méthyle-3-propionyle-1H-pyrrole-1-yle)benzènesulfonamide ;
4-(5-(4,4-diméthyle-2-oxo-1,2,3,4-tétrahydroquinoline-7-yle)-2-méthyle-3-propionyle-1H-pyrrole-1-yle)benzènesulfonamide ;
4-(2-méthyle-3-propionyle-5-(1,4,4-triméthyle-2-oxo-1,2,3,4-tétrahydroquinoline-6-yle)-1 H-pyrrole-1-yle)benzènesulfonamide.

8. Composition pharmaceutique contenant le composé selon la revendication 1 et un support pharmaceutiquement acceptable.

9. Composé selon la revendication 1 destiné à un usage dans la prévention et le traitement d'une maladie ou de ses symptômes ou d'un trouble induit partiellement ou entièrement par des récepteurs d'acétylcholine nicotinique.

10. Composé de formule I destiné à un usage dans le traitement d'une maladie d'un trouble ou d'un état, 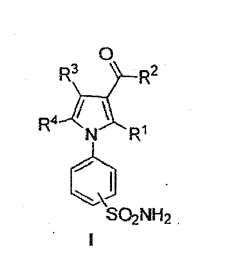 dans lequel,
R¹ est sélectionné parmi : hydrogène, halogène, alkyle substitué optionnellement, perhaloalkyle, cycloalkyle substitué optionnellement, aryle substitué optionnellement ; hétérocyclyle substitué optionnellement, hétéroaryle substitué optionnellement ;
R² est sélectionné parmi : alkyle substitué optionnellement, hétéroalkyle substitué optionnellement, aryle substitué optionnellement, hétéroaryle substitué optionnellement, cycloalkyle substitué optionnellement, hétérocyclyle substitué optionnellement, ou -NR⁵(R⁶)-A¹R¹-N(R⁵)OR⁶ ;
R³ est sélectionné parmi : hydrogène, alkyle substitué optionnellement, halo, cycloalkyle substitué optionnellement, aryle substitué optionnellement, hétérocyclyle substitué optionnellement, hétéroaryle substitué optionnellement, cyano, nitro ou -NR⁵(R⁶)-OR⁵ ;
R⁴ est 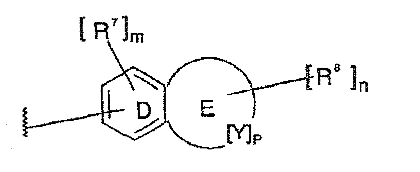
dans lequel l'anneau de phényle 'D' est fusionné avec l'anneau 'E', qui est un anneau non aromatique de cinq à huit élément incluant un ou des groupes 'Y';
Y est sélectionné indépendamment à chaque répétition parmi -O--S--NH-, 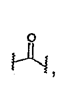 ou 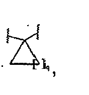
où q = 1-4 ; dans lequel, lorsque Y est sélectionné en tant que -NH- ou 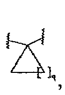 il est optionnellement substitué par [R⁸]ₙ ;
dans lequel, R⁵ et R⁶ sont sélectionnés indépendamment parmi : hydrogène, alkyle substitué optionnellement, hétéroalkyle substitué optionnellement, aryle substitué optionnellement, hétéroaryle substitué optionnellement, cycloalkyle substitué optionnellement, hétérocyclyle substitué optionnellement, R^{9a}C(=A¹)- ;
R⁷ est sélectionné indépendamment à chaque occurrence dans le groupe composé de halogène, alkyle substitué optionnellement, cycloalkyle substitué optionnellement ;
R⁸ est sélectionné indépendamment à chaque occurrence dans le groupe composé de alkyle substitué optionnellement, R⁹A¹-, R^{9a}C(=A¹)- ;
m = 0 à 2 ;
n = 0 à 3 ;
p = 0 à 4 ;
dans lequel, R⁹ partout où il apparaît, est sélectionné parmi : hydrogène, optionnellement substitué C₁₋₆ alkyle, hétéroalkyle substitué optionnellement, aryle substitué optionnellement, hétéroaryle substitué optionnellement, cycloalkyle substitué optionnellement, et hétérocyclyle substitué optionnellement ; et A¹ est sélectionné parmi : O et S ;
R^{9a} partout où il apparaît, est sélectionné parmi : C₁₋₆ alkyle optionnellement substitué, hétéroalkyle substitué optionnellement, aryle substitué optionnellement, hétéroaryle substitué optionnellement, cycloalkyle substitué optionnellement, et hétérocyclyle substitué optionnellement ;
dans lequel,
"alkyle substitué optionnellement, " signifie un groupe alkyle optionnellement substitué par 1 à 6 substituants sélectionnés indépendamment dans le groupe composé d'oxo, halogène, nitro, cyano, aryle, hétéroaryle, cycloalkyle, R^{10a}SO₂-, R¹⁰A¹-, R^{10a}(=O)-, R^{10a}(=O)O-, (R¹⁰)(H)NC(=O)-, (R¹⁰)(alkyle)NC(=O)-, R^{10a}C(=O)N(H)-, (R¹⁰)(H)N-, (R¹⁰)(alkyle)N-, (R¹⁰)(H)NC(=A¹)N(H)-, (R¹⁰)(alkyle)NC(=A¹)N(H)- ;
"hétéroalkyle substitué optionnellement, " signifie un groupe hétéroalkyle optionnellement substitué par 1 à 6 substituants sélectionnés indépendamment dans le groupe composé d'oxo, halogène, nitro, cyano, aryle, hétéroaryle, cycloalkyle,
"cycloalkyle substitué optionnellement, " signifie un groupe cycloalkyle optionnellement substitué par 1 à 6 substituants sélectionnés indépendamment dans le groupe composé d'oxo, halogène, nitro, cyano, aryle, hétéroaryle, alkyle, R^{10a}C(=O)-, R^{10a}SO₂-, R¹⁰A¹-, R^{10a}OC(=O)-, R^{10a}C(=O)O-, (R¹⁰)(H)NC(=O), (R¹⁰)(alkyle)NC(=O)-, R^{10a}C(=O)N(H)-, (R¹⁰)(H)N-, (R¹⁰)(alkyle)N-, (R¹⁰)(H)NC(=A¹)N(H)-, (R¹⁰)(alkyle)NC(=A¹)N(H)- ;
"aryle substitué optionnellement, " signifie (i) un aryle optionnellement substitué par 1 à 3 substituants sélectionnés indépendamment dans le groupe composé de halogène, nitro, cyano, hydroxy, C₁ à C₆ alkyle, C₃ à C₆ cycloalkyle, C₁ à C₆ perhaloalkyle, alkyle-O-, perhaloalkyle-O-, alkyle-N(alkyle)-, alkyle-N(H)-, H₂N-, alkyle-SO₂-, perhaloalkyle-SO₂-, alkyle-C(=O)N(alkyle)-, alkyle-C(=O)N(H)-, alkyle-N(alkyle)C(=O)-, alkyle-N(H)C(=O)-, H₂NC(=O)-, alkyle-N(alkyle)SO₂- alkyle-N(H)SO₂-, H₂NSO₂-, hétérocycle de 3 à 6 éléments contenant 1 à 2 hétéroatomes sélectionnés parmi N, O et S optionnellement substitués par alkyle ou alkyle-C(=O)-, (ii) un anneau d'aryle optionnellement fusionné avec un cycloalkane ou hétérocycle dans une liaison optionnellement substituée par oxo, alkyle ou alkyle-C(=O)- ;
"hétérocyclyle substitué optionnellement, " signifie un groupe (i) hétérocyclyle optionnellement substitué sur des anneaux de carbone par 1 à 6 substituants sélectionnés indépendamment dans le groupe composé d'oxo, halogène, nitro, cyano, aryle, hétéroaryle, alkyle, R¹⁰A¹-, R^{10a}OC(=O)-, R^{10a}C(=O)O-, (R¹⁰)(H)NC(=O)-, (R¹⁰)(alkyle)NC(O)-, R^{10a}C(=O)N(H)-, (R¹⁰)(H)N-, (R¹⁰)(alkyle)N-, (R¹⁰)(H)NC(=A¹)N(H), (R¹⁰)(alkyle)NC(=A¹)N(H)-; (ii) hétérocyclyle optionnellement substitué sur un ou des anneaux d'azote par des substituants sélectionnés parmi le groupe composé de : aryle, hétéroaryle, alkyle, R^{10a}C(=O)-, R¹⁰SO₂-, R^{10a}OC(=O)-, (R¹⁰)(H)NC(=O)-, (R¹⁰)(alkyle)NC(=O)- ;
"hétéroaryle substitué optionnellement, " signifie un groupe hétéroaryle optionnellement substitué par 1 à 3 substituants sélectionnés indépendamment dans le groupe composé de halogène, nitro, cyano, hydroxy, C₁ à C₆ ; alkyle, C₃ à C₆ cycloalkyle, C₁ à C₆ perhaloalkyle, alkyle-O-, perhaloalkyle-O-, alkyle-N(alkyle)-, alkyle-N(H)-, H₂N-, alkyle-SO₂-,
perhaloalkyle-SO₂-, alkyle-C(=O)N(alkyle)-, alkyle-C(=O)N(H)-, alkyle-N(alkyle)C(=O)-, alkyle-N(H)C(=O)-, H₂NC(=O)-, alkyle-N(alkyle)SO₂-, alkyle-N(H)SO₂-, H₂NSO₂-, hétérocycle de 3 à 6 éléments contenant 1 à 2 hétéroatomes sélectionnés parmi N, O et S optionnellement substitué par alkyle ou alkyle-C(=O)- ;
dans lequel R¹⁰ est sélectionné parmi : hydrogène, alkyle, aryle, hétéroaryle, cycloalkyle ou hétérocyclyle ; et A¹ est sélectionné parmi : S et O ; et R^{10a} est sélectionné parmi : alkyle, perhaloalkyle, aryle, hétéroaryle, cycloalkyle ou hétérocyclyle.

11. Composé destiné à un usage dans le traitement d'une maladie ou d'un trouble ou d'un état selon la revendication10, dans lequel les composés sont sélectionnés parmi :
4-(5-(4,4-diméthyle, chromane-6-yle)-2-méthyle-3-propionyle-1*H*-pyrrole-1-yle)benzènesulfonamide ;
4-(5-(2,3-dihydrobenzo[b][1,4]dioxin-6-yl)-2-methyl-3-propionyle-1H-pyrrole-1-yle)benzènesulfonamide ;
4-(2-(2,3-dihydrobenzo[b][1,4]dioxin-6-yle)-3,5-diméthyle-4-propionyle-1H-pyrrole-1-yle)benzènesulfonamide ;
éthyle 5-(2,3-dihydrobenzo[b][1,4]dioxin-6-yle)-2,4-diméthyle-1-(4-sulfamoyle, phenyl)-1H-pyrrolee-3-carboxylate ;
4-(5-(2,3-dihydro-1H-inden-4-yle)-2-méthyle-3-propionyle-1H-pyrrole-1-yle)benzènesulfonamide ;
4-(5-(2,2-diméthyle, chromane-6-yle)-2-méthyle-3-propionyle-1H-pyrrole-1-yle)benzènesuifonamide ;
4-(5-(8-fluoro-4,4-diméthyle, chromane-6-yle)-2-méthyle-3-propionyle-1H-pyrrole-1-yle)benzènesulfonamide ;
4-(5-(2-acétyle-4,4-diméthyle-1,2,3,4-tétrahydroisoquinoline-7-yle)-2-méthyle-3-propionyle-1 H-pyrrole-1-yle)benzènesulfonamide ;
4-(5-(2-acétyle-4,4-diméthyle-1,2,3,4-tétrahydroisoquinoline-6-yle)-2-méthyle-3-propionyle-1H-pyrrole-1-yle)benzènesulfonamide ;
4-(5-(4,4-diméthyle-1,2,3,4-tétrahydroisoquinoline-7-yle)-2-méthyle-3-propionyle-1H-pyrrole-1-yle)benzènesulfonamide ;
4-(2-méthyle-3-propionyle-5-(3H-spiro[benzo[b][1,4]dioxine-2,1'-cyclopropane]-7-yle)-1 H-pyrrole-1-yle)benzènesulfonamide ;
4-(2-méthyle-3-propionyle-5-(3H-spiro[benzo[b][1,4]dioxine-2,1'-cyclopropane]-6-yle)-1 H-pyrrole-1-yle)benzènesulfonamide ;
4-(5-(1-acétyle-4,4-diméthyle-1,2,3,4-tétrahydroquinoline-6-yle)-2-méthyle-3-propionyle-1 H-pyrrole-1-yle)benzènesulfonamide ;
4-(5-(1-acétyle-4,4-diméthyle-1,2,3,4-tétrahydroquinoline-7-yle)-2-méthyle-3-propionyle-1 H-pyrrole-1-yle)benzènesulfonamide ;
4-(5-(4,4-diméthyle-1,2,3,4-tétrahydroquinoline-6-yle)-2-méthyle-3-propionyle-1 H-pyrrole-1-yle)benzènesulfonamide ;
4-(5-(4,4-diméthyle-1,2,3,4-tétrahydroquinoline-7-yle)-2-méthyle-3-propionyle-1H-pyrrole-1-yle)benzènesulfonamide ;
4-(5-(4,4-diméthyle-2-oxo-1,2,3,4-tétrahydroquinoline-6-yle)-2-méthyle-3-propionyle-1 H-pyrrole-1-yle)benzènesulfonamide ;
4-(5-(4,4-diméthyle-2-oxo-1,2,3,4-tétrahydroquinoline-7-yle)-2-méthyle-3-propionyle-1H-pyrrole-1-yle)benzènesulfonamide ;
4-(2-méthyle-3-propionyle-5-(1,4,4-triméthyle-2oxo-1,2,3,4-tétrahydroquinoline-6-yle)-1H-pyrrole-1-yle)benzènesulfonamide ;
4-(2-méthyle-3-propionyle-5-(5,6,7,8-tétrahydroquinoline-2-yle)-1H-pyrrole-1-yle)benzènesulfonamide.

12. Composé destiné à un usage dans le traitement d'une maladie ou d'un trouble ou d'un état selon la revendication 10, dans lequel le trouble ou l'état ou la maladie est sélectionné parmi : le groupe composé de : la maladie d'Alzheimer, le trouble cognitif léger, la démence sénile, la démence vasculaire, la démence de la maladie de Parkinson, le trouble de déficit de l'attention, le trouble de déficit de l'attention avec hyperactivité, la démence à corps de Lewy, le complexe de démence du SIDA, la maladie de Pick, la démence associée au syndrome de Down, la maladie de Huntington, les déficits cognitifs associés à des traumatismes crâniens, les déficits de déclenchement cognitif et sensomoteur associés à la schizophrénie, les déficits cognitifs associés aux troubles bipolaires, les troubles cognitifs associés à la dépression, la douleur aiguë, la douleur post-chirurgicale ou post-opératoire, la douleur chronique, l'inflammation, la douleur inflammatoire, la douleur névropathique, le sevrage tabagique, le besoin de développement de nouveaux vaisseaux sanguins suite à une cicatrisation de plaies, le besoin de développement de nouveaux vaisseaux sanguins suite à la vascularisation de greffes cutanées et les problèmes de circulation, l'arthrite, l'arthrite rhumatoïde, le psoriasis, la maladie de Crohn, la colite ulcéreuse, la pochite, la maladie intestinale inflammatoire, la maladie coeliaque, la périodontite, la sarcoïdose, la pancréatite, le rejet de greffes organiques, la maladie auto-immune aiguë associée à la greffe d'organes, la maladie auto-immune chronique associée à la greffe d'organes, le choc septique, le syndrome de choc toxique, le syndrome septique, la dépression et la spondylite rhumatoïde, comprenant l'étape d'administration d'un composé de formule I.

13. Composé destiné à un usage dans le traitement d'une maladie ou d'un trouble ou d'un état selon la revendication 10, dans lequel la maladie ou le trouble ou l'état est sélectionné dans le groupe classifié ou diagnostiqué en tant que troubles neurocognitifs majeurs ou mineurs ou troubles dus à une neurodégénérescence.

14. Composé destiné à un usage dans le traitement d'une maladie ou d'un trouble ou d'un état selon la revendication 10, comprenant l'administration d'un composé de formule I combiné avec ou en complément de médicaments utilisés pour le traitement des troubles du déficit de l'attention avec hyperactivité, de la schizophrénie, et d'autres troubles cognitifs comme la maladie d'Alzheimer, la démence de la maladie de Parkinson, la démence vasculaire ou la démence associée à des corps de Lewy, à des traumatismes crâniens.

15. Composé destiné à un usage dans le traitement d'une maladie ou d'un trouble ou d'un état selon la revendication 10, comprenant en outre l'administration d'un composé de formule I combiné avec ou en complément d'inhibiteurs d'acétylcholinestérase, de médicaments ou produits biologiques modifiant la pathologie destinés aux troubles neurodégénératifs, de médicaments dopaminergiques, d'antidépresseurs, d'antipsychotiques typiques ou atypiques.
